# EUROPEAN PATENT SPECIFICATION

(11) **EP 3 687 515 B1**
(45) Date of publication and mention of the grant of the patent: **06.07.2022**
(21) Application number: 18782135.0
(22) Date of filing: 25.09.2018
(51) Int. Cl.: A61K 31/135, A61K 31/343, A61K 9/20, A61K 9/48, A61P 25/24, A61P 25/00

(54) **SOLID ORAL DOSAGE FORMS OF KETAMINE DERIVATIVES**
FESTE ORALE DARREICHUNGSFORMEN VON KETAMINDERIVATEN
FORMES GALÉNIQUES SOLIDES ORALES DE DÉRIVÉS DE KÉTAMINE

(30) Priority: 25.09.2017 GB 201715500; 18.05.2018 GB 201808150
(43) Date of publication of application: 05.08.2020
(73) Proprietor: Small Pharma Ltd, 6-8 Bonhill Street London EC2A 4BX (GB)
(72) Inventor: ROBBINS, Trevor, Cambridge Cambridgeshire CB2 3EB (GB); PHILLIPS, Benjamin, Cambridge Cambridgeshire CB2 3EB (GB); PEARSON, David, Penicuik Midlothian EH26 0BE (GB); SHARP, Lorraine, Penicuik Midlothian EH26 0BE (GB); MYERSON, Richard, High Peak Derbyshire SK23 0PG (GB); RANDS, Peter, London Greater London E2 7PR (GB); LAYZELL, Marie, London Greater London E2 7PR (GB); JOEL, Zelah, London Greater London E2 7PR (GB); BENWAY, Tiffanie, London Greater London E2 7PR (GB); JAMES, Ellen, London Greater London E2 7PR (GB)
(74) Representative: Patent Boutique LLP
(86) International application number: PCT/GB2018/052726
(87) International publication number: WO 2019/058145

(56) References cited:
- WO-A1-2014/020155
- WO-A1-2017/208031
- PANOS ZANOS ET AL: "NMDAR inhibition-independent antidepressant actions of ketamine metabolites", NATURE, vol. 533, no. 7604, 4 May 2016 (2016-05-04), pages 481-486, XP55368059, London ISSN: 0028-0836, DOI: 10.1038/nature17998 cited in the application

## Description

### FIELD OF THE INVENTION

This invention relates to high concentration solid oral dosage forms of ketamine metabolites. This invention further relates to crystalline forms of ketamine metabolites and to high concentration solid oral dosage forms thereof.

### BACKGROUND TO THE INVENTION

Ketamine derivatives, and in particular compounds derived from the ketamine metabolite 2R,6R-hydroxynorketamine and 2S,6S-hydroxynorketamine, show promise in the treatment of depression.

WO 2014/020155 A1 discloses methods and compositions for the treatment of pain, in particular the oral transmucosal administration of S-Ketamine, its salts or derivatives Parenteral formulations of 2R,6R-hydroxynorketamine and 2S,6S-hydroxynorketamine are known from Zanos et al, Nature, (2016), 533, 481-486. However, parenteral formulations suffer drawbacks in the treatment of most depression sufferers, for whom treatment in an outpatient setting without the need of a medical professional would be preferable. The provision of solid oral dosage forms of ketamine metabolites is therefore advantageous over parenteral dosage forms. There are, however, challenges in the development of solid oral dosage forms of ketamine metabolites such as 2R,6R-hydroxynorketamine, 2S,6S-hydroxynorketamine, R-5,6-dehydronorketamine and S-5,6-dehydronorketamine. For example, in the free base form they readily form a viscous oil or gum under ambient conditions, are chemically unstable with a tendency to dimerise, and are particularly difficult to process into a pharmaceutical formulation unless in the liquid state.

Moreover, medication compliance in psychiatric and neurological disorders is a significant hurdle to effective therapy. The solid oral dosage forms of the present invention address such problems.

### SUMMARY OF THE INVENTION

The invention is as defined in the claims.

Provided herein, as defined in the claims, is a solid oral dosage form comprising a ketamine metabolite selected from 2R,6R-hydroxynorketamine and 2S,6S-hydroxynorketamine as a solid low molecular weight salt thereof, wherein the dosage form comprises at least 20% by weight of the ketamine metabolite, wherein the solid oral dosage form delivers the active ingredient with a human oral bioavailability of 60% or more; and wherein the low molecular weight salt of the ketamine metabolite is obtainable by reaction of the ketamine metabolite with an organic acid having Formula I or II 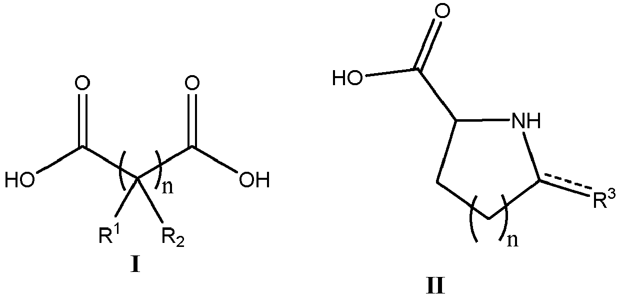 wherein n = 0-3,
R¹ and R² are each independently selected from -H, -OH, and -COOH, and wherein when n = 2, both R² may together represent a C=C bond, and wherein R³ is -H, -OH, =O, or -COOH.

It has now been found that ketamine metabolites of the present invention have high oral bioavailability once a dosing threshold has been overcome. Poor oral bioavailability observed at low doses can be explained by glucuronidation in the gut. Conversely, high oral bioavailability observed at high doses (eg. >30mg/kg as measured in mice) can be explained by saturation of UGT enzymes in the gut by substrate ketamine metabolites.

Moreover, high doses of ketamine metabolites of the present invention are effective in enhancing cognition in domains such as executive function, perceptual function, learning ability, memory, and attention. Surprisingly, this cognitive enhancing effect is observed at doses higher than the reported dose necessary to produce an antidepressant effect. Doses of 10mg/kg and 30mg/kg have no effect on cognition in mice, despite their showing a robust effect in the forced swim test and other assays predictive of antidepressant effects (reported in Zanos (2016)).

Development of a suitable solid oral dosage form to deliver a sufficiently high dose of a ketamine metabolite of the present invention to obtain high oral bioavailability and/or to produce cognitive enhancement presents multiple challenges. The solid state of the drug substance must be stable, processable, and preferably is readily soluble in aqueous solvents. Moreover, the solid oral dosage form must be able to deliver a sufficient brain concentration without being so large as to cause difficulties swallowing. The solid oral dosage forms of the present invention overcome these inherent obstacles with respect to the subject ketamine metabolites.

In embodiments of the invention the solid oral dosage form comprises from 10mg to 400mg of the ketamine metabolite. In embodiments of the invention the solid oral dosage form comprises from 10mg to 200mg of the ketamine metabolite. In embodiments of the invention the solid oral dosage form comprises from 10mg to 150mg of the ketamine metabolite. In embodiments of the invention the solid oral dosage form comprises from 10mg to 100mg of the ketamine metabolite. In embodiments of the invention the solid oral dosage form comprises from 10mg to 50mg of the ketamine metabolite. In embodiments of the invention the solid oral dosage form comprises from 10mg to 20mg of the ketamine metabolite.

In embodiments of the invention, the solid oral dosage form comprises 50mg or more of the ketamine metabolite. In embodiments of the invention, the solid oral dosage form comprises 100mg or more of the ketamine metabolite. In embodiments of the invention the solid oral dosage form comprises 150mg or more of the ketamine metabolite. In embodiments of the invention the solid oral dosage form comprises 200mg or more of the ketamine metabolite. In embodiments of the invention the solid oral dosage form comprises 250mg or more of the ketamine metabolite. In embodiments of the invention the solid oral dosage form comprises 300mg or more of the ketamine metabolite. In embodiments of the invention the solid oral dosage form comprises 350mg or more of the ketamine metabolite. In embodiments of the invention the solid oral dosage form comprises 400mg or more of the ketamine metabolite. In embodiments of the invention the solid oral dosage form comprises 450mg or more of the ketamine metabolite. In embodiments of the invention the solid oral dosage form comprises 500mg or more of the ketamine metabolite.

In embodiments of the invention the solid oral dosage form has a length no greater than 16mm. In embodiments of the invention the solid oral dosage form has a length no greater than 14.5mm. In embodiments the solid oral dosage form has a length between 6mm and 12mm.

The solid oral dosage form of the invention delivers the active ingredient with a human oral bioavailability of 60% or more. In embodiments of the invention the solid oral dosage form delivers the active ingredient with a human oral bioavailability of 70% or more. In embodiments of the invention the solid oral dosage form delivers the active ingredient with a human oral bioavailability of 80% or more.

Example anionic counterions, including those falling within the scope of the claims, are provided herein.

In embodiments of the invention the solid oral dosage form comprises a low molecular weight salt of the ketamine metabolite which is obtainable by reaction of the ketamine metabolite with an organic acid having Formula I or II wherein the organic acid of Formula I or II is chiral.

In embodiments of the invention the low molecular weight salt of the ketamine metabolite comprises up to two stoichiometric equivalents of an anionic counterion having a molecular weight of 240 daltons or less.

Example anionic counterion include those selected from acetate, angelate, aspartate, benzoate, besylate, bromide, carbonate, chloride, citrate, decanoate, edisylate, esylate, fumarate, gentisate, glutarate, glutamate, gluconate, glucoronate, glycolate, hexanoate, hippurate, iodide, isethionate, lactate, malate, maleate, mandelate, mesylate, methylbromide, methylsufate, mucate, napthoate, napsylate, nitrate, octanoate, oxalate, phosphate, pyroglulamate, succinate, sulfate, tartrate, tiglate, tosylate, and trifluoroacetate.

In embodiments of the invention the low molecular weight salt of the ketamine metabolite comprises up to two stoichiometric equivalents of an anionic counterion having a molecular weight of 160 daltons or less.

Example anionic counterion include those selected from acetate, angelate, aspartate, benzoate, besylate, bromide, carbonate, chloride, esylate, fumarate, gentisate, glutarate, glutamate, glycolate, hexanoate, iodide, isethionate, lactate, malate, maleate, mandelate, mesylate, methylbromide, methylsufate, nitrate, octanoate, oxalate, phosphate, pyroglulamate, succinate, sulfate, tartrate, tiglate, and trifluoroacetate.

In embodiments of the invention the low molecular weight salt of the ketamine metabolite comprises up to two stoichiometric equivalents of an anionic counterion having a molecular weight of 120 daltons or less.

Example anionic counterion include those selected from acetate, angelate, bromide, carbonate, chloride, esylate, fumarate, glycolate, hexanoate, lactate, maleate, mesylate, ethylbromide, methylsufate, nitrate, oxalate, phosphate, succinate, sulfate, tiglate, and trifluoroacetate.

In embodiments of the invention the low molecular weight salt of the ketamine metabolite comprises up to two stoichiometric equivalents of an anionic counterion having a molecular weight of 80 daltons or less.

Example anionic counterion include those selected from acetate, bicarbonate, bromide, carbonate, chloride, glycolate, and nitrate.

In embodiments of the invention the low molecular weight salt of the ketamine metabolite comprises up to two stoichiometric equivalents of an anionic counterion having a molecular weight of 40 daltons or less.

Example organic acid may be selected from aspartic acid, citric acid, fumaric acid, glutaric acid, glutamic acid, hippuric acid, malic acid, maleic acid, mucic acid, oxalic acid, pyroglutamic acid, succinic acid, and tartaric acid, and most preferably pyroglutamic acid.

In embodiments of the invention the low molecular weight salt of the ketamine metabolite comprises about one stoichiometric equivalent of the anionic counterion.

In embodiments of the invention the solid oral dosage form comprises at least 25% by weight of the ketamine metabolite.

In embodiments of the invention the solid oral dosage form comprises at least 40% by weight of the ketamine metabolite.

In embodiments of the invention the solid oral dosage form comprises at least 50% by weight of the ketamine metabolite.

In embodiments of the invention the solid oral dosage form comprises at least 60% by weight of the ketamine metabolite.

In embodiments of the invention the solid oral dosage form comprises at least 70% by weight of the ketamine metabolite.

In embodiments of the invention the solid oral dosage form comprises at least 80% by weight of the ketamine metabolite.

In embodiments of the invention the solid oral dosage form comprises at least 90% by weight of the ketamine metabolite.

In embodiments of the invention the form of the ketamine metabolite low molecular weight salt thereof is a crystalline form.

In embodiments of the invention the solid oral dosage form is a capsule.

In embodiments of the invention the solid oral dosage form is a tablet.

In embodiments of the invention the solid oral dosage form is non-sedative.

In embodiments of the invention the solid oral dosage form is non-neurotoxic.

In embodiments of the invention the solid oral dosage form comprises a diluent blend comprising one or more diluent. In embodiments of the invention the diluent blend comprises microcrystalline cellulose. In embodiments of the invention the diluent blend comprises dicalcium phosphate.

In embodiments of the invention the solid oral dosage form is a capsule comprising a capsule shell comprising a constituent selected from gelatin and hydroxypropyl methylcellulose.

In embodiments of the invention the low molecular weight salt of the ketamine metabolite is selected from 2R,6R-hydroxynorketamine L-tartrate and 2S,6S-hydroxynorketamine D-tartrate having an X-ray powder diffraction pattern comprising characteristic peaks expressed in degrees 2-theta at positions 22.7, 25.7 and 28.7.

In embodiments of the invention the crystalline form of the ketamine metabolite selected from 2R,6R-hydroxynorketamine L-tartrate and 2S,6S-hydroxynorketamine D-tartrate has an X-ray powder diffraction pattern further comprising characteristic peaks expressed in degrees 2-theta at positions 6.5 and 12.8.

In embodiments of the invention the crystalline form of the ketamine metabolite selected from 2R,6R-hydroxynorketamine L-tartrate and 2S,6S-hydroxynorketamine D-tartrate has an X-ray powder diffraction pattern further comprising characteristic peaks expressed in degrees 2-theta at positions 10.2, 17.2, 20.3, 21.2, 21.9 and 30.5, and wherein the crystalline form is anhydrous.

In embodiments of the invention the crystalline form of the ketamine metabolite selected from anhydrous 2R,6R-hydroxynorketamine L-tartrate or 2S,6S-hydroxynorketamine D-tartrate has an X-ray powder diffraction pattern further comprising characteristic peaks expressed in degrees 2-theta at positions 11.4, 13.8, 16.2, 26.5, 26.7, 29.5, 31.3 and 32.6.

In embodiments of the invention the crystalline form of the ketamine metabolite having an X-ray powder diffraction pattern further comprising characteristic peaks expressed in degrees 2-theta at positions 8.8, 17.6, 23.7, 25.5, 25.6, 28.4 and 30.9, and wherein the low molecular weight salt is hydrated.

In embodiments of the invention the crystalline form of the ketamine metabolite having an X-ray powder diffraction pattern further comprising characteristic peaks expressed in degrees 2-theta at positions 13.7, 15.0, 18.6, 19.8, 21.4, 23.2, 29.2, 32.2 and 34.5, wherein the crystalline form is anhydrous.

In embodiments of the invention the crystalline form is selected from 2R,6R-hydroxynorketamine difumarate and 2S,6S-hydroxynorketamine difumarate having an X-ray powder diffraction pattern comprising characteristic peaks expressed in degrees 2-theta at positions 11.7, 12.4, 22.5 and 22.8.

In embodiments of the invention the crystalline form selected from 2R,6R-hydroxynorketamine difumarate and 2S,6S-hydroxynorketamine difumarate having an X-ray powder diffraction pattern further comprising characteristic peaks expressed in degrees 2-theta at positions 14.7, 16.5, 18.8, 26.8 and 29.4.

In embodiments of the invention the crystalline form selected from 2R,6R-hydroxynorketamine difumarate and 2S,6S-hydroxynorketamine difumarate having an X-ray powder diffraction pattern further comprising characteristic peaks expressed in degrees 2-theta at positions 10.2, 21.4, 23.2, 25.7, and 29.7.

In embodiments of the invention the crystalline form selected from 2R,6R-hydroxynorketamine difumarate and 2S,6S-hydroxynorketamine difumarate having an X-ray powder diffraction pattern further comprising characteristic peaks expressed in degrees 2-theta at positions 18.3, 20.4, 31.0 and 33.0.

In embodiments of the invention the crystalline form is selected from 2R,6R-hydroxynorketamine L-malate and 2S,6S-hydroxynorketamine D-malate having an X-ray powder diffraction pattern comprising a characteristic peak expressed in degrees 2-theta at position 23.1.

In embodiments of the invention the crystalline form is selected from 2R,6R-hydroxynorketamine L-pyroglutamate and 2S,6S-hydroxynorketamine D-pyroglutamate having an X-ray powder diffraction pattern comprising characteristic peaks expressed in degrees 2-theta at position 14.3.

In embodiments of the invention the crystalline form is selected from 2R,6R-hydroxynorketamine L-pyroglutamate and 2S,6S-hydroxynorketamine D-pyroglutamate having an X-ray powder diffraction pattern further comprising characteristic peaks expressed in degrees 2-theta at positions 19.9 and 23.8.

In embodiments of the invention the crystalline form is selected from 2R,6R-hydroxynorketamine L-pyroglutamate and 2S,6S-hydroxynorketamine D-pyroglutamate having an X-ray powder diffraction pattern further comprising characteristic peaks expressed in degrees 2-theta at positions 12.0, 13.6, 15.2, 20.8, 26.2, and 28.9.

In embodiments of the invention the crystalline form is selected from 2R,6R-hydroxynorketamine L-pyroglutamate and 2S,6S-hydroxynorketamine D-pyroglutamate having an X-ray powder diffraction pattern further comprising characteristic peaks expressed in degrees 2-theta at positions 12.8, 17.6, 18.1, and 25.5.

In embodiments of the invention the crystalline form is selected from 2R,6R-hydroxynorketamine L-pyroglutamate and 2S,6S-hydroxynorketamine D-pyroglutamate having an X-ray powder diffraction pattern further comprising characteristic peaks expressed in degrees 2-theta at positions 13.8, 14.1, 17.8, 20.3, 21.0, 21.8, 22.7, 24.6, 25.0, 25.3, 27.3, 28.5, 28.7, 30.6, 32.3, 32.7, 33.3, 33.9, and 34.1.

In embodiments of the invention the crystalline form is selected from 2R,6R-hydroxynorketamine L-pyroglutamate and 2S,6S-hydroxynorketamine D-pyroglutamate having an X-ray powder diffraction pattern further comprising characteristic peaks expressed in degrees 2-theta at positions 9.0, 16.6, 21.4, 22.4, 23.1, 25.7, 26.5, 27.8, 28.2, 29.3, 30.1, 31.1, 31.5, 33.1, 33.7, and 34.6.

In embodiments of the invention the crystalline form is obtained by precipitation or crystallisation from an organic solvent, for example acetonitrile, isopropyl acetate, t-butyl methyl ether, ethyl acetate, or diisopropyl ether.

In embodiments of the invention the solid oral dosage form defined herein comprises a crystalline form as defined herein. In embodiments of the invention the solid oral dosage form defined herein consists essentially of a crystalline form as defined herein. In embodiments of the invention the solid oral dosage form defined herein consists of a crystalline form as defined herein.

In embodiments of the invention, the solid oral dosage form is for administration in combination with a serotonin modulator.

In embodiments of the invention the serotonin modulator is a 5HT2A agonist or partial agonist, for example ergolines (eg. D-lysergic acid amide, ergobasine, methergine, methysergide, D-lysergic acid diethylamide, and D-lysergic acid α-hydroxyethylamide), tryptamines (eg. psilocybin and dimethyltryptamine), and phenethylamines (eg. amphetamine, mescaline).

In embodiments of the invention the serotonin modulator is a selective serotonin reuptake inhibitor (SSRI).

In embodiments of the invention the solid oral dosage form and the serotonin modulator are administered simultaneously, sequentially or separately.

In embodiments of the invention the serotonin modulator is selected from Citalopram, Escitalopram, Paroxetine, Fluoxetine, Fluvoxamine, Sertraline, Desvenlafaxine, Duloxetine, Levomilnacipran, Milnacipran, Tofenacin, Venlafaxine, Vilazodone, Vortioxetine, Etoperidone, Nefazodone, and Trazodone, or a pharmaceutically acceptable salt thereof.

In embodiments of the invention the serotonin modulator is selected from Citalopram, Escitalopram, Paroxetine, Sertraline, Duloxetine, and Venlafaxine, Vortioxetine, or a pharmaceutically acceptable salt thereof.

In embodiments of the invention the solid oral dosage form comprising the ketamine metabolite is for use in treating a disorder in a human patient wherein said disorder is selected from a psychiatric disorder and a neurological disorder.

In embodiments of the invention the solid oral dosage form comprising the ketamine metabolite is for use in treating a disorder in a human patient wherein said disorder is selected from a depressive disorder and a pain disorder.

In embodiments of the invention the solid oral dosage form comprising the ketamine metabolite is for use in treating a patient suffering from one or more of a neurocognitive disorder, a neurodevelopmental disorder, and a psychocognitive disorder.

In preferred embodiments, the neurocognitive disorder is selected from (i) delirium, (ii) Alzheimer's disease, (iii) pseudodementia, (iv) frontotemporal neurocognitive disorder, (v) dementia with Lewy Bodies (vi) vascular neurocognitive disorder, (vii) multi-infarct dementia, (viii) a tauopathy, (ix) Parkinson's Disease, (x) Huntingdon's disease, (xi) transmissible spongiform encephalopathy, (xii) amyotrophic lateral sclerosis, (xiii) traumatic brain injury, (xiv) post-concussion syndrome, (xv) amnesia, (xvi) substance-induced neurocognitive disorder, (xvii) alcohol-induced neurocognitive disorder, (xviii) stroke disorder, (xix) hypersomnia, and (xx) clonic perseveration.

In preferred embodiments, the neurodevelopmental disorder is selected from (i) intellectual disability, (ii) learning disability, (iii) dyslexia, (iv) dyscalculia, (v) dyspraxia, (vi) dysgraphia, (vii) autism-spectrum disorder, (viii) stereotypic movement disorder, (ix) tic disorder, (x) cerebal palsy (xi) fragile-X syndrome, (xii) Down syndrome, (xiii) attention-deficit disorder, (xiv) hypogonadotropic hypogonadal syndrome (xv) neurotoxicant poisoning, (xvi) foetal alcohol spectrum disorder, (xvii) Minamata disease, and (xviii) Rett syndrome.

In preferred embodiments, the psychocognitive disorder is selected from (i) an obsessive compulsive disorder, (ii) a depressive disorder, (iii) a schizophrenia disorder, (iv) a schizotypal disorder, (v) an anxiety disorder, (vi) substance abuse, and (vii) an avolition disorder.

In preferred embodiments, the solid oral dosage form comprising the ketamine metabolite is for use in treating a disorder of diminished motivation in a patient. In preferred embodiments, the solid oral dosage form comprising the ketamine metabolite is for use in treating a disorder of diminished motivation in a patient suffering from a comorbid disorder selected from a neurocognitive disorder, a neurodevelopmental disorder, and a psychocognitive disorder.

In embodiments of the invention the human patient has experienced one or more manic episode or hypomanic episode, is suffering a manic episode or hypomanic episode, or is at risk of suffering one or more manic episode or hypomanic episode.

In embodiments of the invention the human patient is suffering from obsessive-compulsive disorder, bipolar depression type 1, bipolar depression type 2, bipolar depression, or obsessive-compulsive disorder comorbid with depression.

In embodiments of the invention the solid oral dosage form is for use as a first-line therapy.

In embodiments of the invention the solid oral dosage form is for use as an adjunct to behavioural therapy.

Also provided is a bottle comprising a solid oral dosage form as described herein wherein the bottle is fitted with a safety cap.

Also provided is a blister pack comprising a solid oral dosage form as described herein. The blister pack may comprise a metal foil lidding seal. The metal foil lidding seal may comprise an aluminium foil. The blister pack may comprise 7, or 14, or 21, or 28 unit doses.

Also provided is a kit comprising a bottle or a blister pack as described herein alongside instructions for therapeutic administration.

The bottle, blister pack or kit described herein is for use alongside cognitive and/or behavioural therapy.

### BRIEF DESCRIPTION OF THE FIGURES

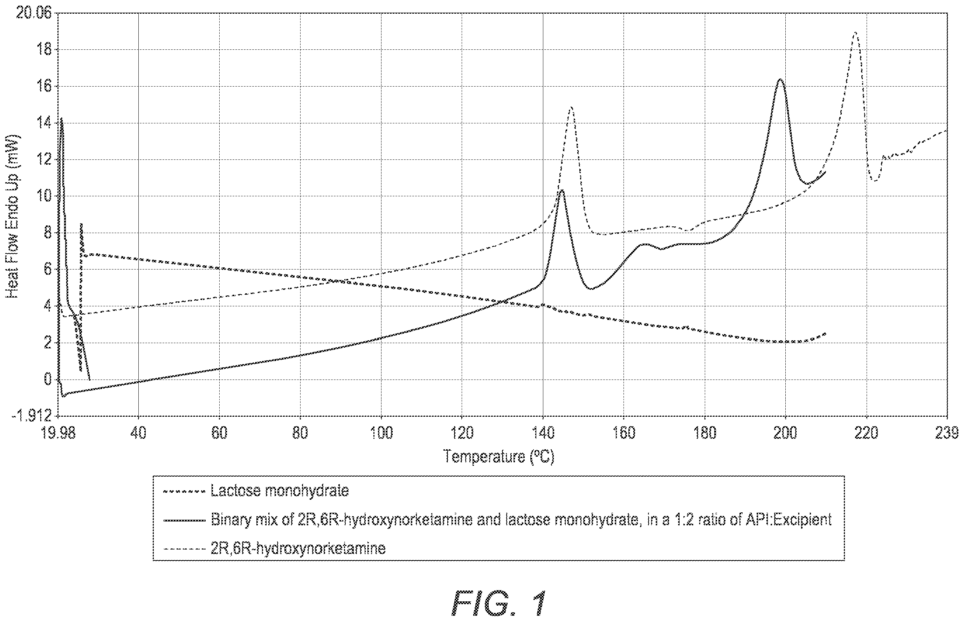

**Figure 1****:** Shows differential scanning calorimetry (DSC) analysis of a binary mix of 2R,6R-hydroxynorketamine and lactose monohydrate in a 1:2 ratio of API:Excipient, compared against 2R,6R-hydroxynorketamine, and against lactose monohydrate.

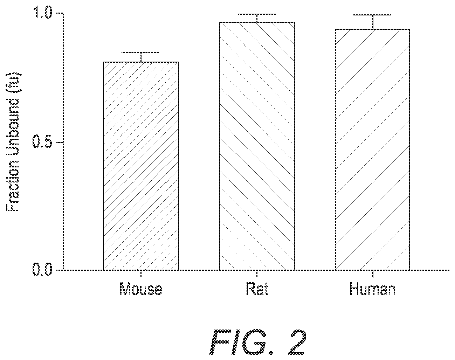

**Figure 2****:** Mean unbound fraction of 2R,6R-hydroxynorketamine in mouse, rat and human plasma. Test compound is prepared in 100 % species-specific plasma. Plasma solution was added to one side of the membrane in an equilibrium dialysis system while buffer (pH 7.4) was added to the other side. The system was allowed to equilibrate at 37 °C. Compound concentration on both sides of the membrane was measured by LC-MS/MS and the unbound fraction (fu) was calculated. Error bars ± SD. PPB in dog was seen to be comparable to other species, however, a mean fu could not be calculated due to interference from endogenous binding.

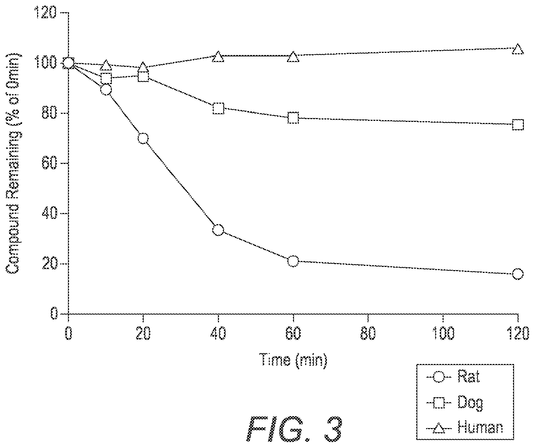

**Figure 3****:** Shows the rate of hepatic clearance in human, rat and dog hepatocyte assays. 2R,6R-HNK is not cleared by human hepatocytes. Test compound (3 µM) was incubated with cryopreserved hepatocytes in suspension. Samples are removed at 6 time points over the course of 120 minutes and analysed by LC-MS/MS.

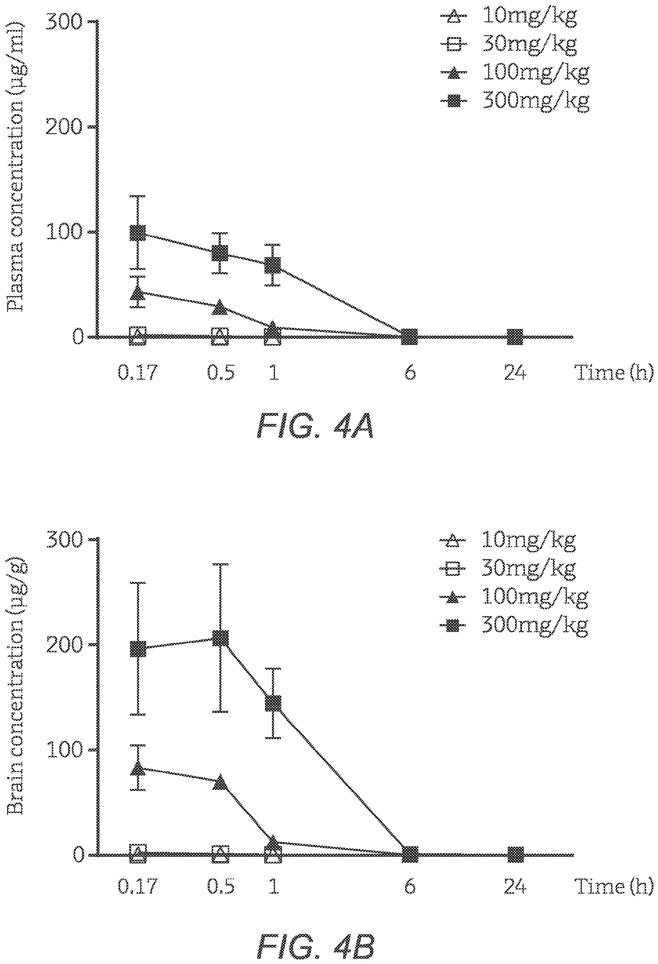

**Figures 4A and 4B****:** PK profile of 2R,6R-hydroxynorketamine hydrochloride in both plasma (A) and brain tissue (B) of male C57B1/6J mice, following administration of 10, 30, 100 or 300mg/kg by oral gavage.

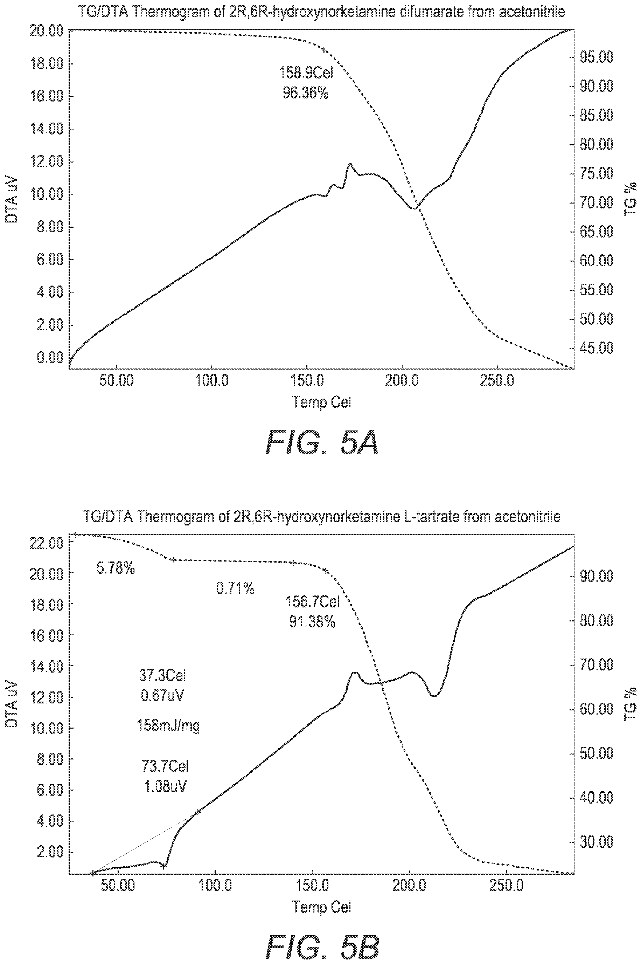

**Figures 5A to 5F****:** shows TG/DTA thermogram of 2R,6R-hydroxynorketamine crystalline salt forms according to the present invention.

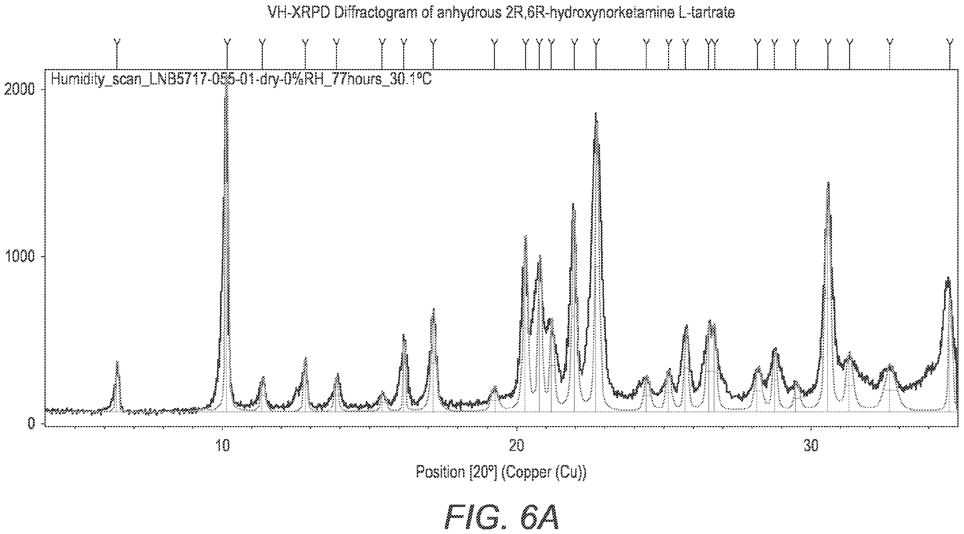

**Figure 6A to 6D****:** shows VH-XRPD diffractogram of 2R,6R-hydroxynorketamine crystalline salt forms according to the present invention.

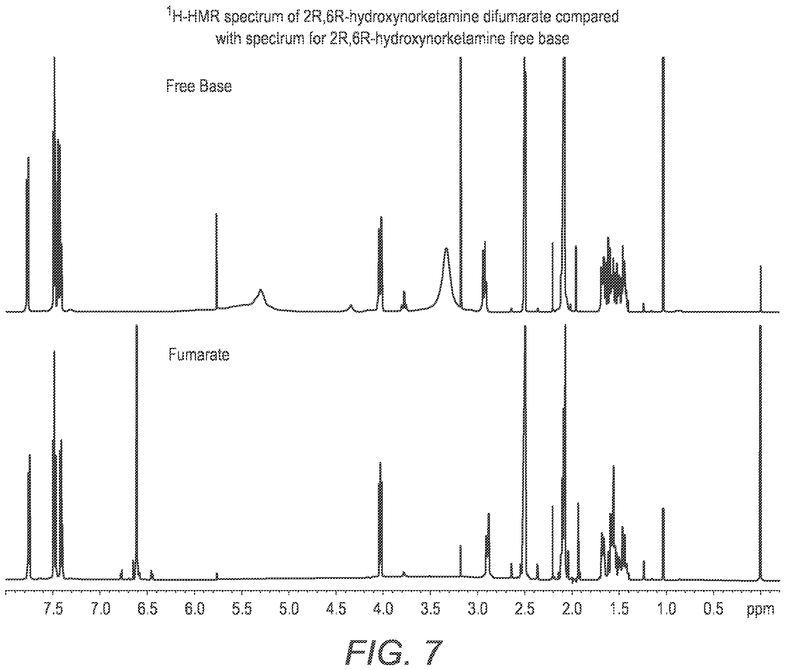

**Figure 7****:** ¹H-NMR spectrum of 2R,6R-hydroxynorketamine difumarate compared with spectrum for 2R,6R-hydroxynorketamine free base.

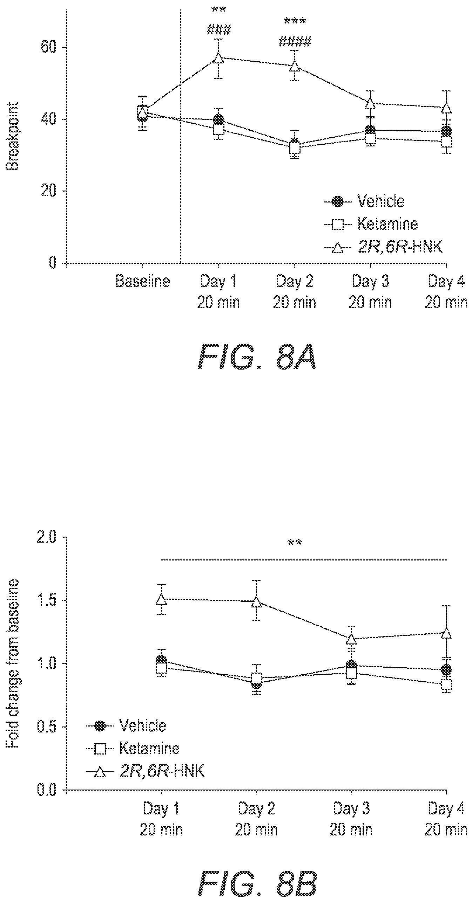

**Figure 8A to 8C****:** Daily administration of 300mg/kg 2R,6R-hydroxynorketamine as an L-pyroglutamate salt (2R,6R-HNK) resulted in a significant increase in motivation as tested using the progressive ratio task. Baseline testing was completed on day 1. Following this, 2R,6R-HNK (300mg/kg), ketamine (10mg/kg) or saline were administered (IP) daily for 4 consecutive days. Mice were tested 20 minutes after each injection. 2R,6R-HNK resulted in a significant increase in breakpoint compared to ketamine and vehicle groups (A (interaction p<0.0001), B). Analysis of the running rate coefficients revealed a trend toward a main effect of drug in predicted peak responding (*C,p*= 0.12). 2R,6R-HNK n=16; ketamine n=16, vehicle n=13. Error bars indicate SEM. Two-way ANOVA with Tukey's *post hoc* comparison, significance of 2R,6R-HNK from vehicle indicated by asterisks, significance of 2R,6R-HNK from ketamine indicated by hashes; p<0.01^{∗∗}/##, p<0.001 ^{∗∗∗}/###, p<0.0001^{∗∗∗∗}/####.

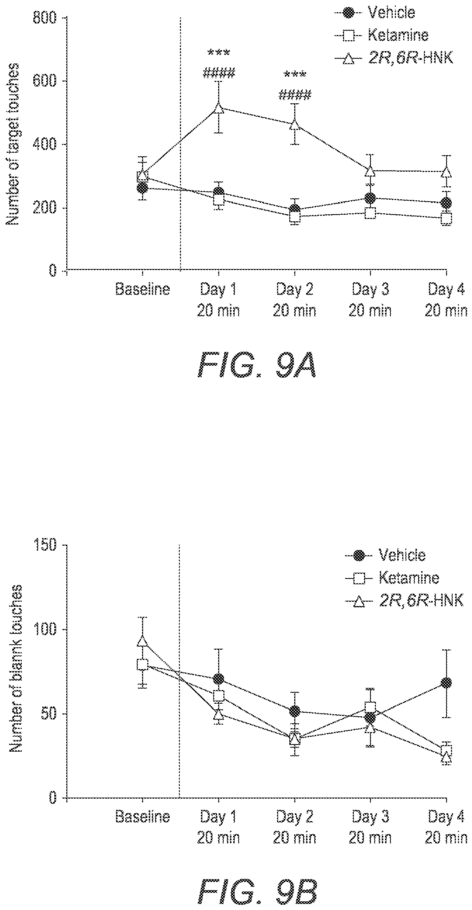

**Figure 9A to 9D****:** Daily administration of 300mg/kg 2R,6R-HNK resulted in a significant increase in accuracy as tested using the progressive ratio task. Baseline testing was completed on day 1. Following this, 2R,6R-HNK (300mg/kg), ketamine (10mg/kg) or saline were administered (IP) daily for 4 consecutive days. Mice were tested 20 minutes after each injection. 2R,6R-HNK resulted in a significant increase in the number of target touches (A, interaction p<0.0001), the ratio of target touches to blank touches (C, interaction p=0.01) and the % of correct touches (D, interaction p=0.0023) compared to ketamine and vehicle groups. 2R,6R-HNK n=16; ketamine n=16, vehicle n=13. Error bars indicate SEM. Two-way ANOVA with Tukey's *post hoc* comparison, significance of 2R,6R-HNK from vehicle indicated by asterisks, significance of 2R,6R-HNK from ketamine indicated by hashes; p<0.05^{∗}/#, p<0.01^{∗∗}/##, p<0.001 ^{∗∗∗}/###, p<0.0001 ^{∗∗∗∗}/####.

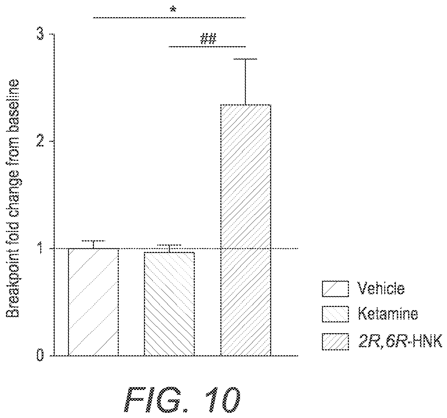

**Figure 10****:** The increase in motivation following administration of 300mg/kg 2R,6R-HNK could be replicated. 2R,6R-HNK resulted in a significant increase in breakpoint compared to ketamine and vehicle groups. 2R,6R-HNK n=16; ketamine n=16, vehicle n=13. Error bars indicate SEM. One-way ANOVA with Tukey's *post hoc* comparison, significance of 2R,6R-HNK from vehicle indicated by asterisks, significance of 2R,6R-HNK from ketamine indicated by hashes; p<0.05^{∗}/#, p<0.01^{∗∗}/##.

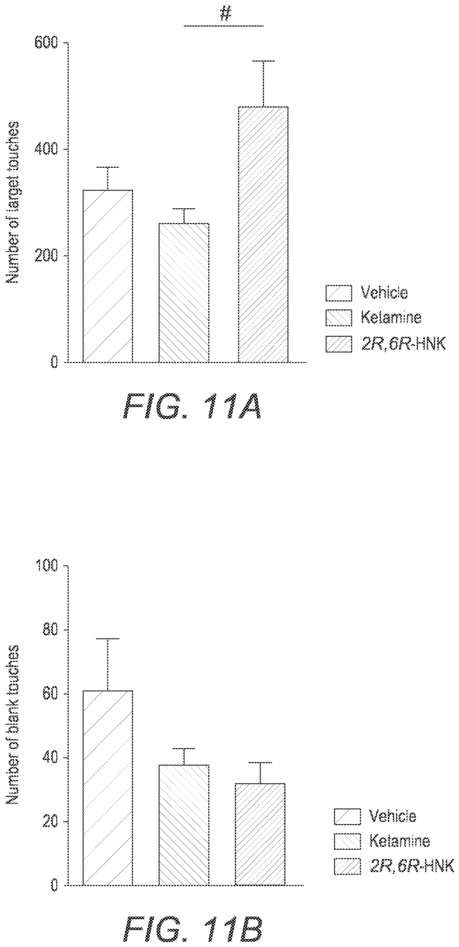

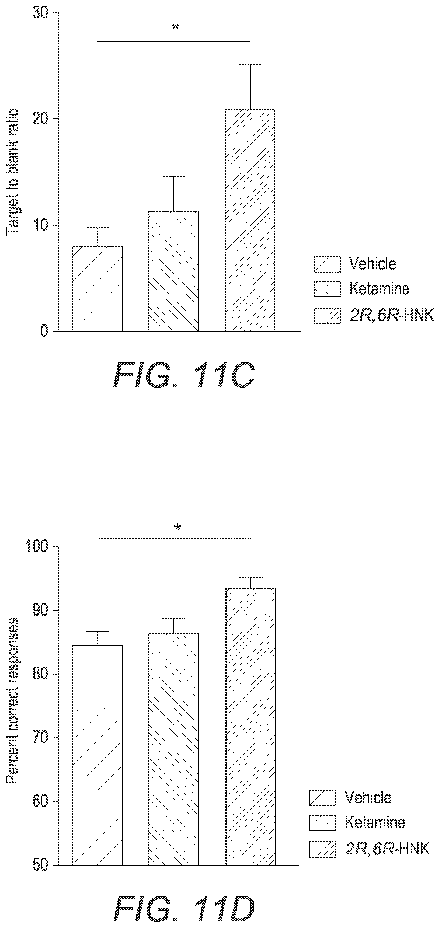

**Figure 11A to 11D****:** The increase in accuracy following administration of 300mg/kg 2R,6R-HNK could be replicated. 2R,6R-HNK resulted in a significant increase in the number of target touches (A), the ratio of target touches to blank touches (C) and the % of correct touches (D) compared to ketamine and vehicle groups. 2R,6R-HNK n=16; ketamine n=16, vehicle n=13. Error bars indicate SEM. One-way ANOVA with Tukey's *post hoc* comparison, significance of 2R,6R-HNK from vehicle indicated by asterisks, significance of 2R,6R-HNK from ketamine indicated by hashes; p<0.05^{∗}/#.

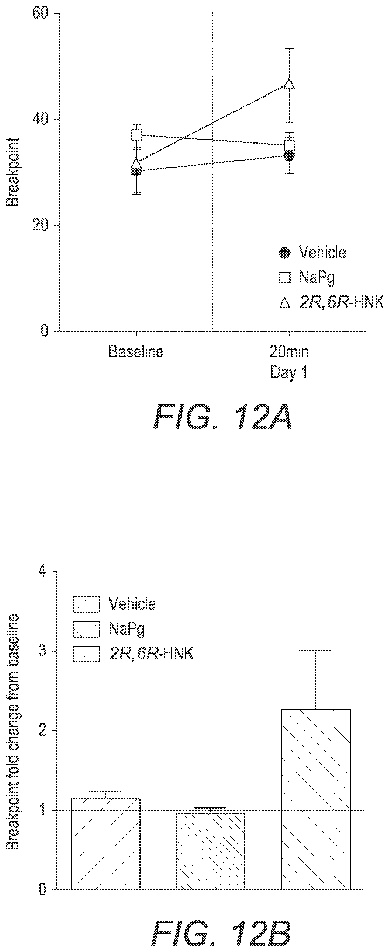

**Figure 12A to 12B****:** The cognitive effects seen following administration of 2R,6R-HNK are not mediated by the pyroglutamate counterion. Sodium L-pyroglutamate (NaPg) did not increase breakpoint compared to vehicle controls (A, B). 2R,6R-HNK n=16; NaPg n=15, vehicle n=14. Error bars indicate SEM.

### DETAILED DESCRIPTION OF THE INVENTION

Throughout this specification, one or more aspects of the invention may be combined with one or more features described in the specification to define distinct embodiments of the invention.

References herein to a singular of a noun encompass the plural of the noun, and vice-versa, unless the context implies otherwise.

As used herein the terms 'active ingredient' and 'ketamine metabolite' are used interchangeably and refer to a compound selected from 2R,6R-hydroxynorketamine, and 2S,6S-hydroxynorketamine. As used herein, the terms '2R,6R-hydroxynorketamine' and '2S,6S-hydroxynorketamine' refer to 2R,6R-2-(2-Chlorophenyl)-2-(amino)-6-hydroxycyclohexanone and 2S,6S-2-(2-Chlorophenyl)-2-(amino)-6-hydroxycyclohexanone respectively. As used herein the terms 'R-5,6-dehydronorketamine' and 'S-5,6-dehydronorketamine' refer to 2R-2-(2-Chlorophenyl)-2-(amino)-5,6-cyclohexenone and 2R-2-(2-Chlorophenyl)-2-(amino)-5,6-cyclohexenone respectively. Ketamine metabolites according to the present invention can be made according to methods known in the art, for example following synthetic schemes disclosed in WO2013/056229.

As used herein, the term '2R,6R-hydroxynorketamine L-pyroglutamate' refers to the acid addition salt formed by the reaction of 2R,6R-2-(2-Chlorophenyl)-2-(amino)-6-hydroxycyclohexanone with L-pyroglutamic acid. Among the advantages of 2R,6R-hydroxynorketamine L-pyroglutamate are high crystallinity, low hygroscopicity, good flow characteristics, and high aqueous solubility (2R,6R-hydroxynorketamine L-pyroglutamate has aqueous solubility of 64mg/ml at 25°C, compared with 14 to 28mg/ml for 2R,6R-hydroxynorketamine hydrochloride (Tocris, cat. no. 6094, Certificate of Analysis, 26 Jan 2017).

As used herein -H means a covalently bonded hydrogen.

As used herein -OH means a covalently bonded hydroxyl.

As used herein =O taken with the carbon to which it is bonded means a carbonyl group.

As used herein -COOH means a carboxylic acid group.

As used herein C=C means an olefin, in other words a carbon-carbon double bond.

As used herein the term 'chiral' means a structure which is not superimposable on its mirror image.

As used herein the term 'homochiral' refers to a composition that comprises substantially one enantiomer of a chiral material.

Quantities of weight provided herein refer to the free base equivalent of a compound of the present invention. For example a unit dose of 50mg 2R,6R-hydroxynorketamine hydrochloride, contains the mass equivalent of 50mg freebase 2R,6R-hydroxynorketamine, and has an actual mass of 57.6 mg.

As used herein the term 'psychiatric disorder' is a clinically significant behavioural or psychological syndrome or pattern that occurs in an individual and that is associated with present distress (e.g., a painful symptom) or disability (i.e., impairment in one or more important areas of functioning) or with a significantly increased risk of suffering death, pain, disability, or an important loss of freedom.

As used herein the term 'neurological disorder' means any disorder of the nervous system, including diseases, conditions, or symptoms resulting from structural, biochemical or electrical abnormalities in the brain, spinal cord or other nerves.

As used herein the term 'attention' describes the behavioural and cognitive process of selectively concentrating on a discrete aspect of information, whether deemed subjective or objective, while ignoring other perceivable information. Attention may be regarded as a set of processes of cognitive resource allocation, and include basic cognitive processes such as sustained attention, divided attention, selective attention and processing speed.

As used herein the term 'executive function' refers to a set of cognitive processes that are necessary for the cognitive control of behaviour. Executive functions include basic cognitive processes such as planning, decision making, response to feedback, cognitive inhibition, inhibitory control, working memory, and cognitive flexibility. As used herein the term 'cognitive control' means the ability of an individual to select and successfully monitor behaviours that facilitate the attainment of chosen goals. An individual's elicited behaviour results from an interaction between cognitive control and stimulus control.

As used herein the term 'learning and memory' is the process of acquiring and storing for future recall of new knowledge, behaviours, skills, values, or preferences, or the modification of those existing. Subdomains involved in learning and memory include free recall, cued recall, recognition memory, semantic and autobiographic long-term memory, and implicit learning.

As used herein the term 'perceptual-motor function', or simply 'perceptual function', is a mode of comprehension by organisation, identification, and interpretation of sensory information in order to represent and understand the presented information, or the environment which generated the information. Perceptual-motor function involves subdomains including visual perception, visuocontructional reasoning and perceptual-motor coordination.

The subject ketamine metabolites of the present invention have been found to be effective in increasing one or more cognitive domain selected from executive function, perceptual function, learning ability, memory, and attention in a human. Specific subdomains of perceptual function which may be improved by solid oral dosage forms of the present invention include visuoconstructional reasoning and sensory memory. Specific subdomains of learning and memory which may be improved by solid oral dosage forms of the present invention include cued recall, recognition memory, implicit learning, and associative learning. Specific subdomains of attention which may be improved by solid oral dosage forms of the present invention include sustained attention, selective attention, and processing speed. Specific subdomains of executive function which may be improved by solid oral dosage forms of the present invention include working memory, responding to feedback, and sensory memory.

The ability of solid oral dosage forms of the present invention to enhance these particular neurocognitive domains and subdomains render them effective in treating either symptoms or underlying pathology in a patient suffering from one or more neurocognitive, neurodevelopmental, or psychocognitive disorders, in particular the specific disorders described herein.

As used herein the term 'non-neurotoxic' describes a method or composition which does not cause injury or death to nervous tissue.

As used herein the term 'non-sedating' describes a method or composition which does not assist the induction of sleep.

As used herein the term 'behavioural therapy' means psychotherapy and/or behaviour analysis falling within a discipline selected from applied behaviour analysis (ABA), the teaching-family model (TFM), positive behaviour support (PBS) and cognitive behaviour therapy (CBT).

Diagnostic criteria for neurocognitive disorders, neurodevelopmental disorders, and psychocognitive disorders referred to herein are provided in the Diagnostic and Statistical Manual of Mental Disorders, Fifth Edition, (DSM-5).

In embodiments of the invention the solid oral dosage form comprising the ketamine metabolite is for use in treating a disorder in a human patient wherein said disorder is selected from a depressive disorder and a pain disorder.

In embodiments of the invention the solid oral dosage form comprising the ketamine metabolite is for use in treating a patient suffering from one or more of a neurocognitive disorder, a neurodevelopmental disorder, and a psychocognitive disorder.

In preferred embodiments of the present invention, the neurocognitive disorder is selected from (i) delirium, (ii) Alzheimer's disease, (iii) pseudodementia, (iv) frontotemporal neurocognitive disorder, (v) dementia with Lewy Bodies (vi) vascular neurocognitive disorder, (vii) multi-infarct dementia, (viii) a tauopathy, (ix) Parkinson's Disease, (x) Huntingdon's disease, (xi) transmissible spongiform encephalopathy, (xii) amyotrophic lateral sclerosis, (xiii) traumatic brain injury, (xiv) post-concussion syndrome, (xv) amnesia, (xvi) substance-induced neurocognitive disorder, (xvii) alcohol-induced neurocognitive disorder, and (xviii) stroke disorder, (xix) hypersomnia, and (xx) clonic perseveration.

As used herein the term 'delirium' means an acute confusional state, caused by decline from a baseline level of mental function. It often varies in severity over a short period of time, and includes attentional deficits, and disorganisation of behaviour.

As used herein the term 'Alzheimer's disease' describes a neurodegenerative disease characterised by the build-up of proteins in the brain to form structures called plaques or tangles. Alzheimer's disease is the most common cause of dementia. Symptoms can include memory loss and difficulties with thinking, problem-solving or language.

As used herein the term 'pseudodementia' refers to a disorder which results in a dementia-like phenotype having predominantly cognitive symptoms such as loss of memory, and vagueness, as well as prominent slowing of movement and reduced or slowed speech. Disorders which can manifest a pseudodementia phenotype include major depressive disorder, mania, bipolar disorder, schizophrenia, dissociative disorders, Ganser syndrome, conversion reaction, and psychoactive drug abuse. A major subcategory of pseudodementia to which the present invention is directed is depressive pseudodementia, also referred to as depressive dementia or major depression with depressive dementia, which is a syndrome in which the patient suffering major depression exhibits symptoms consistent with dementia.

As used herein the term 'frontotemporal neurocognitive disorder', also referred to as frontotemporal dementia (FTD), refers to a neurocognitive disorder involving the frontal or temporal lobes. Types of frontotemporal neurocognitive disorder include behavioural variant of FTD, primary progressive aphasia (semantic variant or nonfluent agrammatic), corticobasal syndrome, progressive supranuclear palsy, and FTD associated with motor neuron disease.

As used herein the term 'dementia with Lewy Bodies' refers to a type of dementia which involves widespread deposits of abnormal clumps of alpha-synuclein protein in neurons, known as Lewy bodies. A feature of dementia with Lewy Bodies is REM sleep behaviour disorder (RBD), in which individuals lose normal muscle paralysis during REM sleep. Other frequent symptoms include visual hallucinations; marked fluctuations in attention or alertness; and slowness of movement, trouble walking, or rigidity.

As used herein the term 'vascular neurocognitive disorder' refers to cognitive impairment caused by restriction of supply of blood to the brain.

As used herein the term 'multi-infarct dementia' also known as vascular dementia, or vascular cognitive impairment, is dementia caused by problems in the supply of blood to the brain leading to worsening cognitive decline.

As used herein, a 'tauopathy' is a disease involving a dysfunction of tau. Examples of tauopathies include Pick disease, argyrophilic grain disease and corticobasal degeneration. As used herein the term 'Pick disease' refers to a cause of frontotemporal lobar degeneration characterised by build-up of tau proteins in neurons, accumulating into silver-staining, spherical aggregations known as Pick bodies.

As used herein the term 'Parkinson's Disease' is a long-term degenerative disorder of the central nervous system that mainly affects the motor system. The motor symptoms of the disease result from the death of cells in the substantia nigra, a region of the midbrain. The reason for this cell death appears to involve the build-up of proteins into Lewy bodies in the neurons. Diagnosis of typical cases is mainly based on symptoms, with tests such as neuroimaging being used to rule out other diseases.

As used herein the term 'Huntingdon's disease' refers to an inherited disorder that results in death of brain cells, caused by an autosomal dominant mutation in either of an individual's two copies of a gene called Huntingtin.

As used herein the term 'transmissible spongiform encephalopathy' refers to a group of progressive, invariably fatal, conditions that affect the brain and nervous system caused by prions, a form of infectious protein. Transmissible spongiform encephalopathies include kuru, Creutzfeldt-Jakob disease (CJD), variant Creutzfeldt-Jakob disease (vCJD or nvCJD), Gerstmann-Sträussler-Scheinker syndrome (GSS), and fatal familial insomnia.

As used herein the term 'amyotrophic lateral sclerosis' or ALS, also known as motor neurone disease (MND), and Lou Gehrig's disease, is a specific disease which causes the death of neurons controlling voluntary muscles. ALS is characterised by stiff muscles, muscle twitching, and weakness, resulting in difficulty speaking, swallowing, and eventually breathing.

As used herein the term 'traumatic brain injury' refers to damage to the brain resulting from external mechanical force.

As used herein the term 'post-concussion syndrome' is a set of symptoms that may continue for a period of time following a mild traumatic brain injury. The syndrome is charactierised by physical symptoms, such as headache, cognitive symptoms, such as difficulty concentrating, and emotional and behavioural symptoms, such as irritability.

As used herein the term 'amnesia' refers to a deficit in memory caused by brain damage, disease, or psychological trauma.

As used herein the term 'substance-induced neurocognitive disorder' refers to a neurocognitive disorder which is caused or exacerbated by substance abuse.

As used herein the term 'alcohol-induced neurocognitive disorder' refers to a neurocognitive disorder which is caused or exacerbated by alcohol abuse.

As used herein the term 'stroke disorder' refers to a condition in which poor blood flow to the brain results in cell death.

As used herein the term 'hypersomnia' refers to a disorder of excessive time spent sleeping or excessive sleepiness.

As used herein the term 'clonic perseveration' refers to a form of motor perseveration in which inappropriate repetition of an action, once initiated, occurs in the absence of an ongoing cue.

In preferred embodiments of the present invention, the neurodevelopmental disorder is selected from (i) intellectual disability, (ii) learning disability, (iii) dyslexia, (iv) dyscalculia, (v) dyspraxia, (vi) dysgraphia, (vii) autism-spectrum disorder, (viii) stereotypic movement disorder, (ix) tic disorder, (x) cerebral palsy (xi) fragile-X syndrome, (xii) Down Syndrome, (xiii) attention-deficit disorder, (xiv) hypogonadotropic hypogonadal syndrome (xv) neurotoxicant poisoning, (xvi) foetal alcohol spectrum disorder, (xvii) Minamata disease, and (xviii) Rett Syndrome.

As used herein the term 'intellectual disability' refers to a neurodevelopmental disorder characterised by significantly impaired intellectual and adaptive functioning. An intellectual disability is typically defined in a person having an IQ under 70 alongside deficits in two or more adaptive behaviours that affect everyday living.

As used herein the term 'learning disability' refers to a significant general impairment in intellectual functioning acquired during childhood.

As used herein the term 'dyslexia' refers to difficulty in reading despite normal intelligence.

As used herein the term 'dyscalculia' refers to difficulty in learning or comprehending arithmetic.

As used herein the term 'dyspraxia' refers to is a chronic neurological disorder beginning in childhood, which affects the planning of movements and motor co-ordination. It may also affect speech. Dyspraxia is thought to occur as a result of brain messages not being accurately transmitted to the body.

As used herein the term 'dysgraphia' refers to a deficiency in the ability to write.

As used herein the term 'autism spectrum disorder' describes a range of developmental conditions, including Asperger syndrome, that affect a person's social interaction, communication, interests and behaviour as defined by the diagnostic criteria set out in DSM-5. An individual with an autism spectrum disorder often presents with social problems that include difficulty communicating and interacting with others, repetitive behaviours, as well as limited interests or activities.

As used herein the term 'stereotypic movement disorder' refers to a motor disorder with onset in childhood involving repetitive, nonfunctional motor behaviour that interferes with normal activities, or results in bodily injury.

As used herein the term 'tic disorder' refers to a disorder characterised by sudden, rapid, nonrhythmic movements. Tic disorders include Tourette's syndrome, which is characterised by motor tics alongside at least one vocal tic.

As used herein the term 'cerebral palsy' refers to a group of permanent disorders of the development of movement and posture, causing activity limitation, that are attributed to non-progressive disturbances that occurred in the developing foetal or infant brain.

As used herein the term 'fragile X syndrome' describes a condition typically caused by an expansion of the CGG triplet repeat within the Fragile X mental retardation 1 gene on the X chromosome. This genetic mutation results in a range of developmental problems including learning disabilities and cognitive impairment. Typically males are more severely affected by this disorder than females.

Affected individuals usually have delayed development of speech and language, with mild to moderate intellectual disability observed with development. The majority of males and about half of females with fragile X syndrome have characteristic physical features that become more apparent with age, including a long and narrow face, large ears, a prominent jaw and forehead, unusually flexible fingers, flat feet, and in males, enlarged testicles.

As used herein the term 'Down Syndrome', also known as trisomy 21, is a genetic disorder caused by the presence of all or part of a third copy of chromosome 21.

As used herein the term 'attention-deficit disorder' refers to a neurodevelopmental disorder characterised by problems paying attention, excessive activity, or difficulty controlling behaviour which is not appropriate for a person's age. The term encompasses attention-deficit hyperactivity disorder (ADHD), in which symptoms must be present for six months or more to a degree that is much greater than others of the same age. Symptoms are evident prior to the age of 12, and must cause significant problems functioning in at least two settings (e.g., social, school/work, or home). ADHD is divided into three subtypes: predominantly inattentive (ADHD-PI or ADHD-I), predominantly hyperactive-impulsive (ADHD-PH or ADHD-HI), and combined type (ADHD-C).

As used herein the term 'hypogonadotropic hypogonada syndrome' refers to a condition characterised by hypogonadism due to an impaired secretion of gonadotropins, including follicle-stimulating hormone (FSH) and luteinizing hormone (LH), by the pituitary gland in the brain, and in turn decreased gonadotropin levels and a resultant lack of sex steroid production.

As used herein the term 'neurotoxicant poisoning' refers to an adverse effect on the structure or function of the central and/or peripheral nervous system caused by a biological or chemical agent.

As used herein the term 'foetal alcohol spectrum disorder' refers to a group of conditions that can occur in a person whose mother drank alcohol during pregnancy. The most severe form of the condition is known as foetal alcohol syndrome (FAS). Other types include partial foetal alcohol syndrome (pFAS), alcohol-related neurodevelopmental disorder (ARND) and alcohol-related birth defects (ARBD).

As used herein the term 'Minamata disease' refers to a neurological syndrome caused by severe mercury poisoning.

As used herein the term 'Rett syndrome' refers to a rare (1 in 12,000), severe neurological disorder that affects mostly girls, owing to the fact it is caused by mutations in the MECP2 gene located on the X chromosome. It is usually discovered within the first two years of life. The clinical features of Rett syndrome include small hands and feet and a deceleration of the rate of head growth (including microcephaly in some). Repetitive stereotyped hand movements, such as wringing and/or repeatedly putting hands into the mouth, are also noted. People with Rett syndrome are prone to gastrointestinal disorders and a majority of individuals experience seizures. They typically have no verbal skills, in addition to experiencing problems with muscles and motor coordination meaning they cannot walk.

In preferred embodiments of the present invention, the psychocognitive disorder is selected from an (i) obsessive compulsive disorder, (ii) a depressive disorder, (iii) a schizophrenia disorder, (iv) a schizotypal disorder, (v) an anxiety disorder, (vi) substance abuse, and (vii) an avolition disorder.

As used herein the term 'obsessive-compulsive disorder' is defined by the presence of either obsessions or compulsions, but commonly both. The symptoms can cause significant functional impairment and/or distress. An obsession is defined as an unwanted intrusive thought, image or urge that repeatedly enters the person's mind. Compulsions are repetitive behaviours or mental acts that the person feels driven to perform. Typically obsessive-compulsive disorder (OCD) manifests as one or more obsession which drives adoption of a compulsion. For example, an obsession with germs may drive a compulsion to clean. A compulsion can either be overt and observable by others, such as checking that a door is locked, or a covert mental act that cannot be observed, such as repeating a certain phrase in one's mind.

As used herein the term 'depressive disorder' includes major depressive disorder, persistent depressive disorder, bipolar disorder, and bipolar depression.

As used herein the term 'major depressive disorder' (MDD, also referred to as major depression or clinical depression) is defined as the presence of five or more of the following symptoms over a period of two-weeks or more (also referred to herein as a 'major depressive episode'), most of the day, nearly every day.
- depressed mood, such as feeling sad, empty or tearful (in children and teens, depressed mood can appear as constant irritability);
- significantly reduced interest or feeling no pleasure in all or most activities;
- significant weight loss when not dieting, weight gain, or decrease or increase in appetite (in children, failure to gain weight as expected);
- insomnia or increased desire to sleep;
- either restlessness or slowed behaviour that can be observed by others;
- fatigue or loss of energy;
- feelings of worthlessness, or excessive or inappropriate guilt;
- trouble making decisions, or trouble thinking or concentrating;
- recurrent thoughts of death or suicide, or a suicide attempt.

At least one of the symptoms must be either a depressed mood or a loss of interest or pleasure.

Persistent depressive disorder, also known as dysthymia, is defined as a patient exhibiting the following two features:
A. has depressed mood for most the time almost every day for at least two years. Children and adolescents may have irritable mood, and the time frame is at least one year.
B. While depressed, a person experiences at least two of the following symptoms:
   - Either overeating or lack of appetite.
   - Sleeping too much or having difficulty sleeping.
   - Fatigue, lack of energy.
   - Poor self-esteem.
   - Difficulty with concentration or decision making.

As used herein 'bipolar disorder' also known as manic-depressive illness, is a disorder that causes unusual shifts in mood, energy, activity levels, and the ability to carry out day-to-day tasks.

There are three defined sub-categories of bipolar disorder; all of them involve clear changes in mood, energy, and activity levels. These moods range from periods of extremely "up," elated, and energised behaviour (known as manic episodes, and defined further below) to very sad, "down," or hopeless periods (known as depressive episodes). Less severe manic periods are known as hypomanic episodes.

Bipolar I Disorder- defined by manic episodes that last at least 7 days, or by manic symptoms that are so severe that the person needs immediate hospital care. Usually, depressive episodes occur as well, typically lasting at least 2 weeks. Episodes of depression with mixed features (having depression and manic symptoms at the same time) are also possible.

Bipolar II Disorder- defined by a pattern of depressive episodes and hypomanic episodes, but not the full-blown manic episodes described above.

As used herein 'bipolar depression' is defined as an individual who is experiencing depressive symptoms with a previous or coexisting episode of manic symptoms, but does not fit the clinical criteria for bipolar disorder.

As used herein the term 'schizophrenia disorder' means is a mental disorder characterised by abnormal social behaviour and failure to understand what is real. According to the DSM-5, to meet the criteria for diagnosis of schizophrenia, the patient must have experienced at least 2 of the following symptoms: delusions, hallucinations, disorganised speech, disorganised or catatonic behaviour, negative symptoms. At least one of the symptoms must be the presence of delusions, hallucinations, or disorganised speech.

As used herein the term 'a schizotypal disorder' refers to a disorder characterised by severe social anxiety, thought disorder, paranoid ideation, derealisation, transient psychosis, and often unconventional beliefs.

As used herein the term 'anxiety disorder' includes generalised anxiety disorder, phobia, panic disorder, social anxiety disorder, and post-traumatic stress disorder.

'Generalised anxiety disorder' (GAD) as used herein means a chronic disorder characterised by long-lasting anxiety that is not focused on any one object or situation. Those suffering from GAD experience non-specific persistent fear and worry, and become overly concerned with everyday matters. GAD is characterised by chronic excessive worry accompanied by three or more of the following symptoms: restlessness, fatigue, concentration problems, irritability, muscle tension, and sleep disturbance.

'Phobia' is defined as a persistent fear of an object or situation the affected person will go to great lengths to avoid, typically disproportional to the actual danger posed. If the feared object or situation cannot be avoided entirely, the affected person will endure it with marked distress and significant interference in social or occupational activities.

A patient suffering a from a 'panic disorder' is defined as one who experiences one or more brief attack (also referred to as a panic attack) of intense terror and apprehension, often marked by trembling, shaking, confusion, dizziness, nausea, and/or difficulty breathing. A panic attack is defined as a fear or discomfort that abruptly arises and peaks in less than ten minutes.

'Social anxiety disorder' is defined as an intense fear and avoidance of negative public scrutiny, public embarrassment, humiliation, or social interaction. Social anxiety often manifests specific physical symptoms, including blushing, sweating, and difficulty speaking.

'Post-traumatic stress disorder' (PTSD) is an anxiety disorder that results from a traumatic experience. Post-traumatic stress can result from an extreme situation, such as combat, natural disaster, rape, hostage situations, child abuse, bullying, or even a serious accident. Common symptoms include hypervigilance, flashbacks, avoidant behaviours, anxiety, anger and depression.

As used herein the term 'substance abuse' means a patterned use of a drug in which the user consumes the substance in amounts or with methods which are harmful to themselves or others.

As used herein the term 'an avolition disorder' refers to a disorder which includes as a symptom the decrease in motivation to initiate and perform self-directed purposeful activities.

In preferred embodiments of the present invention, the composition is for use in treating a disorder of diminished motivation.

As used herein the term 'a disorder of diminished motivation' refers to a disorder which manifests in a deficit in a person's ability to direct behaviour, or in a subject's ability or desire to repeat a behaviour.

In preferred embodiments, the disorder of diminished motivation is selected from (i) apathy, (ii) aboulia, and (iii) akinetic mutism.

As used herein the term 'apathy' refers to a state of indifference, or the suppression of emotions such as concern, excitement, motivation, or passion. Apathy levels in a subject may be measured using the Apathy Evaluation Scale (AES), which measures apathy as related to brain-related pathology, as set out in RS Marin, RC Biedrzycki, S Firinciogullari: "Reliability and Validity of the Apathy Evaluation Scale," Psychiatry Research, 38:143-162, 1991.

As used herein the term 'aboulia' refers to a lack of will or initiative.

As used herein the term 'akinetic mutism' refers to a disorder in which a subject lacks most motor functions such as speech, facial expressions, and gestures, but demonstrate apparent alertness.

In particularly preferred embodiments of the present invention, the disorder of diminished motivation is apathy and the neurocognitive disorder, neurodevelopmental disorder, or psychocognitive disorder is selected from (i) an obsessive-compulsive disorder, (ii) a depressive disorder, (iii) an anxiety disorder, (iv) a schizophrenia disorder, (v) substance abuse, (vi) attention-deficit hyperactivity disorder, (viii) Alzheimer's disease, (ix) Parkinson's disease, (x) Huntington's disease, (xi) amyotrophic lateral sclerosis, (xii) a stroke disorder, and (xiii) a brain injury disorder. Preferably, the neurocognitive disorder, neurodevelopmental disorder, or psychocognitive disorder is selected from (i) an obsessive-compulsive disorder, (ii) a depressive disorder, (iii) an anxiety disorder, and (iv) a schizophrenia disorder.

The methods and compositions of the present invention are particularly useful in enhancing a cognitive subdomain selected from (i) visuocontructional reasoning, (ii) cued recall, (iii) recognition memory, (iv) implicit learning, (v) associative learning, (vi) sustained attention, (vii) selective attention, (viii) processing speed, (ix) working memory, and (x) sensory memory.

As used herein the term 'Cambridge neuropsychological test automated battery', or 'CANTAB', refers to a computer-based cognitive assessment system consisting of a battery of 25 neuropsychological tests, administered to subjects using a touch screen computer.

As used herein the term 'low dose' of the ketamine metabolite refers to a dose of <30mg/kg in mice, or may alternatively refer to an equivalent dose in any other species (eg. human) as calculated by an appropriate pharmacokinetic model. As used herein the term 'high dose' of the ketamine metabolite refers to a dose of >30mg/kg in mice, or may alternatively refer to an equivalent dose in any other species (eg. human) as calculated by an appropriate pharmacokinetic model.

As used herein, the term 'patient' preferably refers to a human patient, but may also refer to a domestic mammal. The term does not encompass laboratory mammals.

As used herein, the term 'first-line therapy' is defined as the first course of pharmaceutical treatment administered in response to an episode of a disorder. The term 'episode' refers to a single noteworthy happening in the course of a longer series of events, such as one critical period of several during a prolonged disorder.

As used herein, the term 'ketamine' refers to 2-(2-Chlorophenyl)-2-(methylamino)-cyclohexanone. As used herein, the term '2R,6R-hydroxynorketamine' refers to 2R,6R-2-(2-Chlorophenyl)-2-(amino)-6-hydroxycyclohexanone.

The present invention provides a solid oral dosage form comprising an active ingredient selected from 2R,6R-hydroxynorketamine, and 2S,6S-hydroxynorketamine, as a low molecular weight salt thereof as defined in the claims, wherein the dosage form comprises at least 20% by weight of the active ingredient.

The data disclosed herein suggests neurocognitive enhancement by 2R,6R-hydroxynorketamine is observed at doses approximately an order of magnitude higher than doses which have been reported to elicit an antidepressant response. Such high doses present challenges when formulated as unit solid doses as large solid dosage forms can be difficult to administer to a patient, with concomitant reductions in patient compliance.

An unexpected advantage of the solid oral dosage forms of the present invention is high human oral bioavailability. The property of high bioavailability and their compatibility with high drug load solid oral dosage forms as described herein renders it possible to formulate ketamine metabolites of the present invention as solid oral dosage forms such as capsules or tablets having spatial dimensions which can be comfortably taken by mouth by a patient. In particular, the solid oral dosage forms of the present invention are able to deliver a sufficiently high dose of the ketamine metabolite to enhance cognitive domains without causing discomfort to the patient.

In preferred embodiments the active ingredient is 2R,6R-hydroxynorketamine.

In preferred embodiments the solid oral dosage form of the invention comprises from 10mg to 400mg of the active ingredient. In preferred embodiments the solid oral dosage form comprises from 10mg to 200mg of the active ingredient. In preferred embodiments the solid oral dosage form comprises from 10mg to 150mg of the active ingredient. In preferred embodiments the solid oral dosage form comprises from 10mg to 100mg of the active ingredient. In preferred embodiments the solid oral dosage form comprises from 10 mg to 50mg of the active ingredient. In preferred embodiments the solid oral dosage form comprises from 10 mg to 20mg of the active ingredient.

In embodiments of the invention, the solid oral dosage form comprises 100mg or more of the active ingredient. In embodiments of the invention the solid oral dosage form comprises 150mg or more of the active ingredient. In embodiments of the invention the solid oral dosage form comprises 200mg or more of the active ingredient. In embodiments of the invention the solid oral dosage form comprises 250mg or more of the active ingredient. In embodiments of the invention the solid oral dosage form comprises 300mg or more of the active ingredient. In embodiments of the invention the solid oral dosage form comprises 350mg or more of the active ingredient. In embodiments of the invention the solid oral dosage form comprises 400mg or more of the active ingredient. In embodiments of the invention the solid oral dosage form comprises 450mg or more of the active ingredient. In embodiments of the invention the solid oral dosage form comprises 500mg or more of the active ingredient.

In preferred embodiments the solid oral dosage form of the present invention has a length between 6mm and 16mm. In preferred embodiments the solid oral dosage form of the present invention has a length no greater than 16mm. In preferred embodiments the solid oral dosage form of the present invention has a length no greater than 14.5mm. In preferred embodiments the solid oral dosage form of the present invention has a length no greater than 12mm. In preferred embodiments the solid oral dosage form of the present invention has a length no greater than 11mm. In preferred embodiments the solid oral dosage form of the present invention has a length no greater than 10mm. In preferred embodiments the solid oral dosage form of the present invention has a length no greater than 9mm. In preferred embodiments the solid oral dosage form of the present invention has a length no greater than 8mm. In preferred embodiments the solid oral dosage form of the present invention has a length no greater than 7mm. In preferred embodiments the solid oral dosage form of the present invention has a length no greater than 6mm.

As used herein, the term 'length' as applied to a solid oral dosage form refers to the longest distance which passes in a straight line through the centre from one edge of the dosage form to the opposite edge. For spherical dosage forms, 'length' has the same meaning as 'diameter' .

The solid oral dosage form of the invention has human oral bioavailability of 60% or more. Preferably the solid oral dosage of the ketamine metabolite has human oral bioavailability of 70% or more. Preferably the solid oral dosage form of the ketamine metabolite has an oral bioavailability of 80% or more.

Example anionic counterions, including those falling within the scope of the claims, are provided herein.

In preferred embodiments of the solid oral dosage form of the present invention, the low molecular weight salt of 2R,6R-hydroxynorketamine or 2S,6S-hydroxynorketamine comprises up to two stoichiometric equivalents of an anionic counterion having a molecular weight of 240 daltons or less. In preferred embodiments the anionic counterion is selected from acetate, angelate, aspartate, benzoate, besylate, bromide, carbonate, chloride, citrate, decanoate, edisylate, esylate, fumarate, gentisate, glutarate, glutamate, gluconate, glucoronate, glycolate, hexanoate, hippurate, iodide, isethionate, lactate, malate, maleate, mandelate, mesylate, methylbromide, methylsufate, mucate, napthoate, napsylate, nitrate, octanoate, oxalate, phosphate, pyroglulamate, succinate, sulfate, tartrate, tiglate, tosylate, and trifluoroacetate.

In preferred embodiments of the solid oral dosage form of the present invention, the low molecular weight salt of 2R,6R-hydroxynorketamine or 2S,6S-hydroxynorketamine comprises up to two stoichiometric equivalents of an anionic counterion having a molecular weight of 160 daltons or less. In preferred embodiments the anionic counterion is selected from acetate, angelate, aspartate, benzoate, besylate, bromide, carbonate, chloride, esylate, fumarate, gentisate, glutarate, glutamate, glycolate, hexanoate, iodide, isethionate, lactate, malate, maleate, mandelate, mesylate, methylbromide, methylsufate, nitrate, octanoate, oxalate, phosphate, pyroglulamate, succinate, sulfate, tartrate, tiglate, and trifluoroacetate.

In preferred embodiments of the solid oral dosage form of the present invention, the low molecular weight salt of 2R,6R-hydroxynorketamine or 2S,6S-hydroxynorketamine comprises up to two stoichiometric equivalents of an anionic counterion having a molecular weight of 120 daltons or less. In preferred embodiments the anionic counterion is selected from acetate, angelate, bromide, carbonate, chloride, esylate, fumarate, glycolate, hexanoate, lactate, maleate, mesylate, ethylbromide, methylsufate, nitrate, oxalate, phosphate, succinate, sulfate, tiglate, and trifluoroacetate.

In preferred embodiments of the solid oral dosage form of the present invention, the low molecular weight salt of 2R,6R-hydroxynorketamine or 2S,6S-hydroxynorketamine comprises up to two stoichiometric equivalents of an anionic counterion having a molecular weight of 80 daltons or less. In preferred embodiments the anionic counterion is selected from acetate, bicarbonate, bromide, carbonate, chloride, glycolate, and nitrate.

In preferred embodiments of the solid oral dosage form of the present invention, the low molecular weight salt of 2R,6R-hydroxynorketamine or 2S,6S-hydroxynorketamine comprises up to two stoichiometric equivalents of an anionic counterion having a molecular weight of 40 daltons or less. In preferred embodiments the anionic counterion is chloride.

In preferred embodiments of the present invention the low molecular weight salt comprises about one stoichiometric equivalent of the anionic counterion.

In preferred embodiments the solid oral dosage form comprises at least 25% by weight of the active ingredient. In preferred embodiments the solid oral dosage form comprises at least 40% by weight of the active ingredient. In preferred embodiments the solid oral dosage form comprises at least 50% by weight of the active ingredient. In preferred embodiments the solid oral dosage form comprises at least 60% by weight of the active ingredient. In preferred embodiments the solid oral dosage form comprises at least 70% by weight of the active ingredient. In preferred embodiments the solid oral dosage form comprises at least 80% by weight of the active ingredient. In preferred embodiments the solid oral dosage form comprises at least 90% by weight of the active ingredient.

In preferred embodiments, the solid oral dosage form is a tablet comprising up to 500mg of the active ingredient, at least 40% by weight of the active ingredient, and a length no greater than 12mm. Preferably the tablet comprises a blend of one or more diluent wherein the first diluent is selected from starch and microcrystalline cellulose or a combination thereof, and one or more optional further diluent is selected from anhydrous lactose, D-mannitol, dicalcium phosphate, calcium carbonate, magnesium oxide, magnesium carbonate, glucose, sorbitol, sucrose, calcium sulphate, starch, dextrates, kaolinite, maltodextrin and lactitol.

In preferred embodiments of the invention, the form of low molecular weight salt thereof is a crystalline form.

In preferred embodiments of the invention the solid oral dosage is a capsule. In alternative preferred embodiments the solid oral dosage form is a tablet. In preferred embodiments the solid oral dosage form is a capsule comprising a capsule shell comprising a constituent selected from gelatin and hydroxypropyl methylcellulose.

In some embodiments, the solid oral dosage form of the invention comprises a crystalline form of a low molecular weight salt of 2R,6R-hydroxynorketamine or 2S,6S-hydroxynorketamine having an X-ray powder diffraction pattern comprising characteristic peaks expressed in degrees 2-theta at positions 19.2, 26.4 and 31.5.

In preferred embodiments, the crystalline low molecular weight salt of 2R,6R-hydroxynorketamine or 2S,6S-hydroxynorketamine has an X-ray powder diffraction pattern further comprising characteristic peaks expressed in degrees 2-theta at positions 14.1, 16.9, 23.5, 24.0 and 29.9.

In embodiments the low molecular weight salt is selected from 2R,6R-hydroxynorketamine L-tartrate and 2S,6S-hydroxynorketamine D-tartrate and displays an X-ray powder diffraction pattern comprising characteristic peaks expressed in degrees 2-theta at positions 22.7, 25.7 and 28.7.

In embodiments the low molecular weight salt is selected from 2R,6R-hydroxynorketamine L-tartrate and 2S,6S-hydroxynorketamine D-tartrate and displays an X-ray powder diffraction pattern further comprising characteristic peaks expressed in degrees 2-theta at positions 6.5 and 12.8.

In embodiments the low molecular weight salt is selected from 2R,6R-hydroxynorketamine L-tartrate and 2S,6S-hydroxynorketamine D-tartrate and displays an X-ray powder diffraction pattern further comprising characteristic peaks expressed in degrees 2-theta at positions 10.2, 17.2, 20.3, 21.2, 21.9 and 30.5, and wherein the low molecular weight salt is anhydrous. In embodiments the anhydrous 2R,6R-hydroxynorketamine L-tartrate or 2S,6S-hydroxynorketamine D-tartrate has an X-ray powder diffraction pattern further comprising characteristic peaks expressed in degrees 2-theta at positions 11.4, 13.8, 16.2, 26.5, 26.7, 29.5, 31.3 and 32.6.

In embodiments the low molecular weight salt is selected from 2R,6R-hydroxynorketamine L-tartrate and 2S,6S-hydroxynorketamine D-tartrate and displays an X-ray powder diffraction pattern further comprising characteristic peaks expressed in degrees 2-theta at positions 8.8, 17.6, 23.7, 25.5, 25.6, 28.4 and 30.9, and wherein the low molecular weight salt is hydrated. In embodiments the anhydrous 2R,6R-hydroxynorketamine L-tartrate or 2S,6S-hydroxynorketamine D-tartrate has an X-ray powder diffraction pattern further comprising characteristic peaks expressed in degrees 2-theta at positions 13.7, 15.0, 18.6, 19.8, 21.4, 23.2, 29.2, 32.2 and 34.5.

In embodiments the low molecular weight salt is selected from 2R,6R-hydroxynorketamine difumarate and 2S,6S-hydroxynorketamine difumarate and displays an X-ray powder diffraction pattern comprising characteristic peaks expressed in degrees 2-theta at positions 11.7, 12.4, 22.5 and 22.8.

In embodiments the low molecular weight salt is selected from 2R,6R-hydroxynorketamine difumarate and 2S,6S-hydroxynorketamine difumarate and displays an X-ray powder diffraction pattern further comprising characteristic peaks expressed in degrees 2-theta at positions 14.7, 16.5, 18.8, 26.8 and 29.4.

In embodiments the low molecular weight salt is selected from 2R,6R-hydroxynorketamine difumarate and 2S,6S-hydroxynorketamine difumarate and displays an X-ray powder diffraction pattern further comprising characteristic peaks expressed in degrees 2-theta at positions 10.2, 21.4, 23.2, 25.7, and 29.7.

In embodiments the low molecular weight salt is selected from 2R,6R-hydroxynorketamine difumarate and 2S,6S-hydroxynorketamine difumarate and displays an X-ray powder diffraction pattern further comprising characteristic peaks expressed in degrees 2-theta at positions 18.3, 20.4, 31.0 and 33.0.

In embodiments the low molecular weight salt is selected from 2R,6R-hydroxynorketamine L-malate and 2S,6S-hydroxynorketamine D-malate and displays an X-ray powder diffraction pattern comprising a characteristic peak expressed in degrees 2-theta at position 23.1.

In embodiments the low molecular weight salt is selected from 2R,6R-hydroxynorketamine L-pyroglutamate and 2S,6S-hydroxynorketamine D-pyroglutamate and displays an X-ray powder diffraction pattern comprising characteristic peaks expressed in degrees 2-theta at position 14.3.

In embodiments the low molecular weight salt is selected from 2R,6R-hydroxynorketamine L-pyroglutamate and 2S,6S-hydroxynorketamine D-pyroglutamate and displays an X-ray powder diffraction pattern further comprising characteristic peaks expressed in degrees 2-theta at positions 19.9 and 23.8.

In embodiments the low molecular weight salt is selected from 2R,6R-hydroxynorketamine L-pyroglutamate and 2S,6S-hydroxynorketamine D-pyroglutamate and displays an X-ray powder diffraction pattern further comprising characteristic peaks expressed in degrees 2-theta at positions 12.0, 13.6, 15.2, 20.8, 26.2, and 28.9.

In embodiments the low molecular weight salt is selected from 2R,6R-hydroxynorketamine L-pyroglutamate and 2S,6S-hydroxynorketamine D-pyroglutamate and displays an X-ray powder diffraction pattern further comprising characteristic peaks expressed in degrees 2-theta at positions 12.8, 17.6, 18.1, and 25.5.

In embodiments the low molecular weight salt is selected from 2R,6R-hydroxynorketamine L-pyroglutamate and 2S,6S-hydroxynorketamine D-pyroglutamate and displays an X-ray powder diffraction pattern further comprising characteristic peaks expressed in degrees 2-theta at positions 13.8, 14.1, 17.8, 20.3, 21.0, 21.8, 22.7, 24.6, 25.0, 25.3, 27.3, 28.5, 28.7, 30.6, 32.3, 32.7, 33.3, 33.9, and 34.1.

In embodiments the low molecular weight salt is selected from 2R,6R-hydroxynorketamine L-pyroglutamate and 2S,6S-hydroxynorketamine D-pyroglutamate and displays an X-ray powder diffraction pattern further comprising characteristic peaks expressed in degrees 2-theta at positions 9.0, 16.6, 21.4, 22.4, 23.1, 25.7, 26.5, 27.8, 28.2, 29.3, 30.1, 31.1, 31.5, 33.1, 33.7, and 34.6.

For reference, the crystalline form of 2R,6R-hydroxynorketamine or 2S,6S-hydroxynorketamine in the free base form is obtainable by crystallisation or precipitation from acetonitrile.

For reference, the crystalline free base form displays a characteristic XRPD peak, expressed in degrees 2-theta, at 26.6.

For reference, the crystalline free base form displays one or more characteristic XRPD peaks, expressed in degrees 2-theta, selected from 22.9, 26.6 and 28.4.

For reference, the crystalline free base form displays two or more characteristic XRPD peaks, expressed in degrees 2-theta, selected from 22.9, 26.6, 28.4 and 32.6.

For reference, the crystalline free base form displays three or more characteristic XRPD peaks, expressed in degrees 2-theta, selected from 22.9, 26.6, 27.3, 28.4 and 32.6.

For reference, the crystalline free base form displays four or more characteristic XRPD peaks, expressed in degrees 2-theta, selected from 22.9, 26.6, 27.3, 28.4 and 32.6.

For reference, the crystalline free base form displays five characteristic XRPD peaks, expressed in degrees 2-theta, at 22.9, 26.6, 27.3, 28.4 and 32.6.

In embodiments the acid addition salt is selected from anhydrous 2R,6R-hydroxynorketamine L-tartrate and anhydrous 2S,6S-hydroxynorketamine D-tartrate and displays an X-ray powder diffraction pattern comprising characteristic peaks expressed in degrees 2-theta at the positions defined in Table 1:

**Table 1**

| Peak | Degrees 2-theta | Peak | Degrees 2-theta | Peak | Degrees 2-theta |
|---|---|---|---|---|---|
| 1 | 6.5 | 11 | 20.8 | 21 | 28.7 |
| 2 | 10.2 | 12 | 21.2 | 22 | 29.5 |
| 3 | 11.4 | 13 | 21.9 | 23 | 30.5 |
| 4 | 12.8 | 14 | 22.7 | 24 | 31.3 |
| 5 | 13.8 | 15 | 24.4 | 25 | 32.6 |
| 6 | 15.5 | 16 | 25.1 | 26 | 34.7 |
| 7 | 16.2 | 17 | 25.7 | | |
| 8 | 17.2 | 18 | 26.5 | | |
| 9 | 19.2 | 19 | 26.7 | | |
| 10 | 20.3 | 20 | 28.2 | | |

In embodiments the acid addition salt is selected from hydrated 2R,6R-hydroxynorketamine L-tartrate and hydrated 2S,6S-hydroxynorketamine D-tartrate and displays an X-ray powder diffraction pattern comprising characteristic peaks expressed in degrees 2-theta at the positions defined in Table 2:

**Table 2**

| Peak | Degrees 2-theta | Peak | Degrees 2-theta | Peak | Degrees 2-theta | Peak | Degrees 2-theta |
|---|---|---|---|---|---|---|---|
| 1 | 6.5 | 11 | 19.6 | 21 | 25.7 | 31 | 33.4 |
| 2 | 8.8 | 12 | 19.8 | 22 | 26.4 | 32 | 34.1 |
| 3 | 12.8 | 13 | 21.4 | 23 | 28.4 | 33 | 34.5 |
| 4 | 13.7 | 14 | 22.7 | 24 | 28.7 | | |
| 5 | 14.1 | 15 | 23.2 | 25 | 29.2 | | |
| 6 | 15.0 | 16 | 23.5 | 26 | 29.8 | | |
| 7 | 16.8 | 17 | 23.6 | 27 | 30.2 | | |
| 8 | 17.6 | 18 | 23.7 | 28 | 30.9 | | |
| 9 | 18.6 | 19 | 25.5 | 29 | 31.6 | | |
| 10 | 19.2 | 20 | 25.6 | 30 | 32.2 | | |

In embodiments the acid addition salt is selected from 2R,6R-hydroxynorketamine difumarate and 2S,6S-hydroxynorketamine difumarate and displays an X-ray powder diffraction pattern comprising characteristic peaks expressed in degrees 2-theta at the positions defined in Table 3:

**Table 3**

| Peak | Degrees 2-theta | Peak | Degrees 2-theta | Peak | Degrees 2-theta | Peak | Degrees 2-theta | Peak | Degrees 2-theta | Peak | Degrees 2-theta |
|---|---|---|---|---|---|---|---|---|---|---|---|
| 1 | 10.2 | 11 | 16.9 | 21 | 22.5 | 31 | 25.2 | 41 | 29.0 | 51 | 33.5 |
| 2 | 11.7 | 12 | 18.0 | 22 | 22.8 | 32 | 25.7 | 42 | 29.4 | 52 | 34.7 |
| 3 | 12.1 | 13 | 18.3 | 23 | 23.2 | 33 | 26.4 | 43 | 29.7 | | |
| 4 | 12.4 | 14 | 18.8 | 24 | 23.5 | 34 | 26.5 | 44 | 29.9 | | |
| 5 | 13.7 | 15 | 19.2 | 25 | 24.0 | 35 | 26.8 | 45 | 30.3 | | |
| 6 | 14.1 | 16 | 19.5 | 26 | 24.3 | 36 | 27.0 | 46 | | | 31.0 |
| 7 | 14.7 | 17 | 20.4 | 27 | 24.5 | 37 | 27.4 | 47 | | | 31.5 |
| 8 | 15.2 | 18 | 20.8 | 28 | 24.6 | 38 | 27.7 | 48 | | | 31.9 |
| 9 | 15.6 | 19 | 21.4 | 29 | 24.7 | 39 | 28.1 | 49 | | | 32.5 |
| 10 | 16.5 | 20 | 22.0 | 30 | 24.9 | 40 | 28.8 | 50 | | | 33.0 |

In embodiments the acid addition salt is selected from 2R,6R-hydroxynorketamine L-pyroglutamate and 2S,6S-hydroxynorketamine D-pyroglutamate and displays an X-ray powder diffraction pattern comprising characteristic peaks expressed in degrees 2-theta at the positions defined in Table 4:

**Table 4**

| Peak | Degrees 2-theta | Peak | Degrees 2-theta | Peak | Degrees 2-theta | Peak | Degrees 2-theta | Peak | Degrees 2-theta |
|---|---|---|---|---|---|---|---|---|---|
| 1 | 9.0 | 11 | 17.8 | 21 | 23.1 | 31 | 27.8 | 41 | 32.3 |
| 2 | 12.0 | 12 | 18.1 | 22 | 23.8 | 32 | 28.2 | 42 | 32.7 |
| 3 | 12.8 | 13 | 19.9 | 23 | 24.6 | 33 | 28.5 | 43 | 33.1 |
| 4 | 13.6 | 14 | 20.3 | 24 | 25.0 | 34 | 28.7 | 44 | 33.3 |
| 5 | 13.8 | 15 | 20.8 | 25 | 25.3 | 35 | 28.9 | 45 | 33.7 |
| 6 | 14.1 | 16 | 21.0 | 26 | 25.5 | 36 | 29.3 | 46 | 33.9 |
| 7 | 14.3 | 17 | 21.4 | 27 | 25.7 | 37 | 30.1 | 47 | 34.1 |
| 8 | 15.2 | 18 | 21.8 | 28 | 26.2 | 38 | 30.6 | 48 | 34.6 |
| 9 | 16.6 | 19 | 22.4 | 29 | 26.5 | 39 | 31.1 | | |
| 10 | 17.6 | 20 | 22.7 | 30 | 27.3 | 40 | 31.5 | | |

In preferred embodiments, the crystalline form is obtained by precipitation or crystallisation from an organic solvent selected from acetonitrile, isopropyl acetate, t-butyl methyl ether, ethyl acetate, and diisopropyl ether.

Preferred embodiments of the solid oral dosage form comprise a crystalline form as defined above. Embodiments of the solid oral dosage form of the present invention consist essentially of a crystalline form as defined above. Embodiments of the solid oral dosage form of the present invention consist of a crystalline form as defined above.

In some embodiments, the solid oral dosage form of the invention comprises a metabolite of ketamine selected from 2R,6R-hydroxynorketamine and 2S,6S-hydroxynorketamine as a solid low molecular weight salt thereof as defined in the claims, for co-administration with a serotonin modulator. As used herein, the term 'serotonin modulator' is defined as any compound which affects the serotonin neurotransmitter (serotonergic system). Serotonin modulators include serotonin stimulators (e.g. vortioxetine), , serotonin agonists, in particular 5HT2A agonists, serotonin antagonist and reuptake inhibitors (SARIs; e.g. etoperidone, lorpiprazole, lubazodone, mepiprazole, nefazodone, and trazodone), selective serotonin reuptake inhibitors (SSRIs; e.g. citalopram, escitalopram, fluoxetine, fluvoxamine, paroxetine, sertraline, dapoxetine), serotonin-norepinephrine reuptake inhibitors (SNRIs; e.g. venlafaxine, milnacipran, duloxetine, levomilnacipran, desvenlafaxine, sibutramine), and noradrenergic and specific serotonergic antidepressants (NaSSAs; e.g. aptazapine, esmirtazapine, mianserin, mirtazapine, setiptiline).

As used herein, the term 'coadministration' is defined as either the administration of a formulation which contains both the dosage form of the present invention and the serotonin modulator, or the simultaneous, essentially simultaneous, sequential or separate administration within a given dosing period of separate formulations containing the solid oral dosage form of the present invention and the serotonin modulator, respectively.

A problem common to all combination therapies is the risk of drug-drug interactions (DDIs) which can lead to unwanted side effects which are not present in one or other members of the combination. Specifically, the potential for adverse DDIs increases if one member of the combination affects the rate of metabolism of another member of the combination. Furthermore, when combined with a serotonin modulator, a drug which interferes with the serotonergic system may result in adverse DDIs. It has been discovered that ketamine metabolites according to the present invention do not adversely affect the rate of metabolism of serotonin modulators, nor do they interfere with the serotonergic system, and combinations of compounds of the present invention with serotonin modulators exhibit limited adverse DDIs.

Also provided are solid oral dosage forms according to the present invention, wherein the solid oral dosage form is for use in combination with a serotonin modulator. The serotonin modulator is a selective serotonin reuptake inhibitor. Preferred serotonin modulators are selected from vortioxetine, etoperidone, lorpiprazole, lubazodone, mepiprazole, nefazodone, trazodone, citalopram, escitalopram, fluoxetine, fluvoxamine, paroxetine, sertraline, dapoxetine, venlafaxine, milnacipran, duloxetine, levomilnacipran, desvenlafaxine, sibutramine, aptazapine, esmirtazapine, mianserin, mirtazapine, and setiptiline. Particularly preferred serotonin modulators are selected from citalopram, escitalopram, fluoxetine, fluvoxamine, paroxetine, sertraline, vortioxetine and dapoxetine.

A preferred class of serotonin modulators are SSRIs. Preferred SSRIs are citalopram, escitalopram, fluoxetine, fluvoxamine, paroxetine, sertraline and dapoxetine. Most preferred SSRIs are citalopram or escitalopram.

Also provided is a combination of an SSRI and the solid oral dosage form as described herein for use in the treatment of a depressive disorder in a patient.

Suitable unit doses of the serotonin modulators for use in the solid oral dosage form of the present invention include the unit doses for these compounds as described in Martindale: The Complete Drug Reference (London, the Pharmaceutical Press, 38^{th} Edition) and US Pharmacopoeia and National Formulary 2016 Edition.

Suitable unit doses for citalopram include 10mg, 20mg and 40mg. Suitable unit doses for escitalopram include 5mg, 10mg and 20mg. Suitable unit doses for fluoxetine include 20mg and 60mg. Suitable unit doses for fluvoxamine include 50mg and 100mg. Suitable unit doses for paroxetine include 10mg, 20mg and 30mg. Suitable unit doses for sertraline include 50mg and 100mg. Suitable unit doses for dapoxetine include 30mg and 60mg.

Also provided are solid oral dosage forms according to the invention, for use in treating a patient has experienced one or more manic episode or hypomanic episode, and/or is suffering or is at risk of suffering one or more manic episode or hypomanic episode.

In preferred embodiments, the solid oral dosage form of the present invention is for use in treating a disorder in a patient wherein said disorder is selected from a psychiatric disorder and a pain disorder. In preferred embodiments, the solid oral dosage form of the present invention is for use in treating a disorder in a patient wherein said disorder is selected from a depressive disorder and a pain disorder. In preferred embodiments, said patient is suffering from a condition selected from major depression, bipolar disorder Type I, bipolar disorder Type II, bipolar depression, postpartum depression, dementia-related depression, obsessive-compulsive disorder, obsessive-compulsive disorder (OCD) with co-morbid depression, and post-traumatic stress disorder (PTSD) with co-morbid depression, tics with comorbid depression, and social anxiety with comorbid depression.

In embodiments of the invention the solid oral dosage form of the present invention is for use in treating a disorder in a human patient wherein said disorder is selected from a psychiatric disorder and a neurological disorder.

In embodiments of the invention the solid oral dosage form of the present invention is for use in treating a disorder in a human patient wherein said disorder is selected from a depressive disorder and a pain disorder.

In embodiments of the invention the solid oral dosage form of the present invention is for use in treating a patient suffering from one or more of a neurocognitive disorder, a neurodevelopmental disorder, and a psychocognitive disorder.

In preferred embodiments, the neurocognitive disorder is selected from (i) delirium, (ii) Alzheimer's disease, (iii) pseudodementia, (iv) frontotemporal neurocognitive disorder, (v) dementia with Lewy Bodies (vi) vascular neurocognitive disorder, (vii) multi-infarct dementia, (viii) a tauopathy, (ix) Parkinson's Disease, (x) Huntingdon's disease, (xi) transmissible spongiform encephalopathy, (xii) amyotrophic lateral sclerosis, (xiii) traumatic brain injury, (xiv) post-concussion syndrome, (xv) amnesia, (xvi) substance-induced neurocognitive disorder, (xvii) alcohol-induced neurocognitive disorder, (xviii) stroke disorder, (xix) hypersomnia, and (xx) clonic perseveration.

In preferred embodiments, the neurodevelopmental disorder is selected from (i) intellectual disability, (ii) learning disability, (iii) dyslexia, (iv) dyscalculia, (v) dyspraxia, (vi) dysgraphia, (vii) autism-spectrum disorder, (viii) stereotypic movement disorder, (ix) tic disorder, (x) cerebal palsy (xi) fragile-X syndrome, (xii) Down syndrome, (xiii) attention-deficit disorder, (xiv) hypogonadotropic hypogonadal syndrome (xv) neurotoxicant poisoning, (xvi) foetal alcohol spectrum disorder, (xvii) Minamata disease, and (xviii) Rett syndrome.

In preferred embodiments, the psychocognitive disorder is selected from (i) an obsessive compulsive disorder, (ii) a depressive disorder, (iii) a schizophrenia disorder, (iv) a schizotypal disorder, (v) an anxiety disorder, (vi) substance abuse, and (vii) an avolition disorder.

In preferred embodiments, the solid oral dosage form of the present invention is for use in treating a disorder of diminished motivation in a patient. In preferred embodiments, the solid oral dosage form of the present invention is for use in treating a disorder of diminished motivation in a patient suffering from a comorbid disorder selected from a neurocognitive disorder, a neurodevelopmental disorder, and a psychocognitive disorder.

In preferred embodiments, the solid oral dosage form of the present invention is for use in increasing cognitive flexibility or cognitive inhibition in a patient suffering from a disorder selected from a psychiatric disorder and a neurological disorder.

In preferred embodiments the psychiatric disorder or neurological disorder is selected from (i) an obsessive-compulsive disorder, (ii) an autism spectrum disorder, (iii) fragile X syndrome, (iv) Rett syndrome, (v) a body-focused disorder, (vi) a body dysmorphic disorder, (vii) a hoarding disorder, (viii) a tic disorder, (ix) a stammering disorder, (x) an eating disorder, (xi) a depressive disorder, (xii) an anxiety disorder, and (xiii) a schizophrenia disorder, (xiv) substance abuse, (xv) attention-deficit hyperactivity disorder, and (xvi) a pain disorder.

In preferred embodiments of the present invention increased cognitive flexibility or cognitive inhibition comprises, or manifests in whole or in part, in reduction of perseveration.

The present invention further provides solid oral dosage forms of the invention for use as a first-line therapy.

In a preferred embodiment, the solid oral dosage form is for use in a patient who has failed to achieve adequate control of depression symptoms using psychotherapy alone.

In another preferred embodiment, the solid oral dosage form is for use in a patient who has not previously been treated with an antidepressant agent.

The present invention provides a solid oral dosage form comprising a metabolite of ketamine selected from 2R,6R-hydroxynorketamine or 2S,6S-hydroxynorketamine as a solid low molecular weight salt thereof, wherein the dosage form comprises at least 20% by weight of the active ingredient, wherein the solid oral dosage form delivers the active ingredient with a human oral bioavailability of 60% or more, and wherein the low molecular weight salt is obtainable by reaction of the metabolite of ketamine with an organic acid having Formula I or II 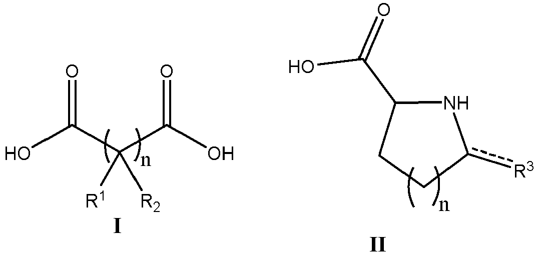 wherein n = 0-3,
R¹ and R² are each independently selected from -H, -OH, and -COOH, and wherein when n = 2 or 3, two adjacent R² groups may together represent a C=C bond, and wherein
R³ is -H, -OH, =O, or -COOH.

It has been discovered that 2R,6R-hydroxynorketamine and 2S,6S-hydroxynorketamine form crystalline salts readily with organic acids as described herein. Moreover, organic acids as used in the present invention may be chiral, enabling the formation of a pharmaceutically acceptable salt to be performed simultaneously with chiral resolution of the active ingredient.

In embodiments of the present invention, the low molecular weight salt is obtainable with a chiral organic acid having Formula III, IV or V: 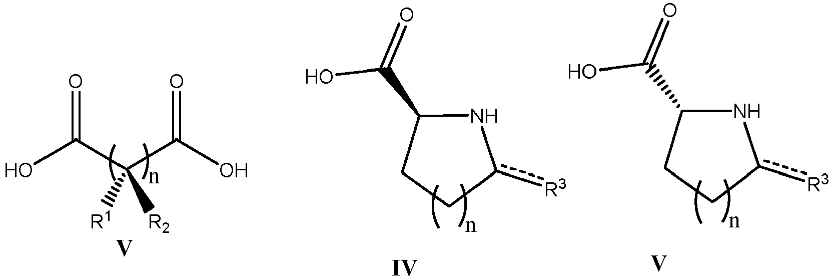 wherein n = 0-3,
R¹ and R² are each independently selected from -H, -OH, and -COOH, wherein at least one pair of R¹ and R² are different, and wherein when n = 2 or 3, two adjacent R² groups may together represent a C=C bond, and wherein
R³ is -H, -OH, =O, or -COOH.

In embodiments of the invention the low molecular weight salt is obtainable with an organic acid of Formula I, wherein n=1 or 2, and wherein each R¹ is H, one or both R² is -OH and any remaining R² is -H.

In embodiments of the invention the low molecular weight salt is obtainable with an organic acid of Formula I, wherein n=2 or 3, and wherein each R¹ is H, two adjacent R² groups are taken together to represent a C=C bond, and any remaining R² is -H.

In embodiments of the invention the low molecular weight salt is obtainable with an organic acid of Formula I, wherein n=2, and wherein each R¹ is -H and both R² groups are taken together to represent a C=C bond.

In embodiments of the invention the low molecular weight salt is obtainable by reaction with an organic acid of Formula II, wherein n=1 or 2, and wherein R³ is =O.

In preferred embodiments of the present invention, the low molecular weight salt is selected from aspartic acid, citric acid, fumaric acid, glutaric acid, glutamic acid, hippuric acid, malic acid, maleic acid, mucic acid, oxalic acid, pyroglutamic acid, succinic acid, and tartaric acid.

In preferred embodiments of the present invention, the low molecular weight salt is selected from citric acid, L-tartaric acid, D-tartaric acid, L-malic acid, D-malic acid, fumaric acid, D-pyroglutamic acid, and L-pyroglutamic acid.

In preferred embodiments, the organic acid is homochiral.

In preferred embodiments of the present invention, the low molecular weight salt is selected from 2R,6R-hydroxynorketamine L-tartrate, 2S,6S-hydroxynorketamine D-tartrate, 2R,6R-hydroxynorketamine difumarate, 2S,6S-hydroxynorketamine difumarate, 2R,6R-hydroxynorketamine D-pyroglutamate, 2S,6S-hydroxynorketamine D-pyroglutamate, 2R,6R-hydroxynorketamine L-pyroglutamate, 2S,6S-hydroxynorketamine L-pyroglutamate, 2R,6R-hydroxynorketamine L-malate, and 2S,6S-hydroxynorketamine D-malate.

In preferred embodiments of the present invention, the low molecular weight salt is in substantially crystalline form.

In preferred embodiments of the present invention, the solid oral dosage form is selected from a tablet and a capsule.

In embodiments of the present invention, the solid oral dosage form comprises a blend of one or more diluent. Preferably the diluent blend comprises one or more, and preferably two or more, diluents selected from anhydrous lactose, D-mannitol, dicalcium phosphate, calcium carbonate, magnesium oxide, magnesium carbonate, glucose, sorbitol, sucrose, calcium sulphate, starch, dextrates, kaolinite, maltodextrin and lactitol. More preferred diluents are selected from microcrystalline cellulose, dicalcium phosphate, kaolinite, starch and calcium carbonate.

In embodiments of the invention the solid oral dosage form is a tablet and comprises a diluent blend comprising microcrystalline cellulose. In further embodiments, the diluent blend comprises two or more diluents wherein one diluent is selected from microcrystalline cellulose and starch, and wherein a further diluent is an inorganic diluent.

In embodiments of the invention, the diluent blend comprises dicalcium phosphate and microcrystalline cellulose.

Preferred embodiments of the invention are wherein the solid oral dosage form is a capsule wherein the capsule shell comprises a constituent selected from gelatin and hydroxypropyl methylcellulose.

In preferred embodiments the dosage form is a tablet and the blend of one or more diluent comprises microcrystalline cellulose. In preferred embodiments the dosage form is a capsule and the capsule shell comprises a constituent selected from gelatin and hydroxypropyl methylcellulose.

In alternative embodiments the solid oral dosage form of the present invention does not comprise a diluent.

In embodiments of the invention the solid oral dosage form of the present invention comprises at least one disintegrant, preferably selected from cross-linked polyvinylpyrrolidinone, alginic acid, sodium alginate, and guar gum.

In embodiments of the invention the solid oral dosage form of the present invention comprises at least one glidant, preferably selected from colloidal silicon dioxide, silica, colloidal silica, e.g. colloidal silica anhydrous, magnesium trisilicate, powdered cellulose, starch and talc.

In embodiments of the invention the solid oral dosage form of the present invention comprises at least one lubricant, preferably selected from magnesium stearate, calcium stearate, aluminium stearate, and PEG 4000 - 8000.

According to the present invention, the amount of diluent may vary within a range of from about 1 to 40%, preferably 1 to 30%, preferably 1 to 25% by weight based on the total weight of the solid oral dosage form.

The amount of disintegrant may vary within a range of from to 5 to 40%, preferably 10 to 35% by weight based on the total weight of the solid oral dosage form.

The amount of glidant may vary within ranges of from 0.1 to 10%, preferably 0.1 to 5%, preferably 0.5 to 3%, preferably 2 to 4 % by weight based on the total weight of the solid oral dosage form.

The amount of lubricant may vary within a range of from 0.1 to 5%, e.g. 0.5 to 2% by weight based on the total weight of the solid oral dosage form.

In the present invention one or more excipient may serve more than one function e.g. as disintegrant, binder, glidant, and/or lubricant.

In an embodiment of the invention, the solid oral dosage form comprises the following excipients, one or more diluent in a total amount of about 1% to 25% by weight based on the total weight of the tablet, one or more disintegrants in a total amount of about 10% to 35% by weight based on the total weight of the tablet, one or more glidants in a total amount of about 0.5% to 3% by weight based on the total weight of the tablet, and/or one or more lubricants in a total amount of about 0.5% to 2% by weight based on the total weight of the tablet.

In embodiments of the invention in which the solid oral dosage form is a tablet, the process for the preparation of the tablets may comprise the steps of forming an inner phase, mixing it together with an outer phase, compressing the obtained mixture and optionally coating the tablet.

The inner phase comprises an active ingredient selected from 2R,6R-hydroxynorketamine and 2S,6S-hydroxynorketamine. Preferably, the inner phase comprises the active ingredient and one or more excipients, more preferably one or more diluent. Preferably the amount of one or more diluent in the inner phase is ranging from about 1 to 30%, preferably 1 to 20%, preferably 1 to 15%. The diluent of the inner phase according to the invention is preferably selected from starch, microcrystalline cellulose, and dicalcium phosphate, and preferably microcrystalline cellulose. The amount of microcrystalline cellulose in the inner phase may vary from about 10 to 29%, preferably 12 to 14% by weight based on the total weight of the tablet. The amount of hydroxypropylmethyl cellulose in the inner phase may vary from 1 to 5%, preferably 1 to 2% by weight based on the total weight of the tablet. The inner phase are mixed together with water and the mixture is processed for granulation, e.g. using a wet high-shear granulator to form the wet-granulates. The wet-granulates may be then, dried, e.g. using a fluid bed dryer.

The outer phase consists in a mixture of the inner phase with one or more excipients. The inner phase and one or more excipients of the outer phase are mixed together. Preferably, one or more binders are added. Most preferably microcrystalline cellulose is added. Even more preferably, microcrystalline cellulose is added in the range of 1 to 10% by weight based on the total weight of the solid oral dosage form. In a preferred embodiment of the invention, in the outer phase, the amount of microcrystalline cellulose is around 5% by weight based on the total weight of the solid oral dosage form. The outer phase according to the invention may also contain one or more disintegrant, preferably cross-linked polyvinylpyrrolidone. In an embodiment, the amount of disintegrant in the outer phase is ranging from about 10 to 30%, preferably 12 to 25%, most preferably about 15%.

In embodiments of the invention, one or more glidants are incorporated into the outer phase.

In embodiments of the invention, one or more lubricants are incorporated into the outer phase.

In embodiments of the invention, the solid oral dosage form is a tablet formed by compression of the mixture of the inner and the outer phases with a tablet press.

In specific embodiments of the invention the solid oral dosage form comprising 20% or more by weight of an active ingredient selected from 2R,6R-hydroxynorketamine, and 2S,6S-hydroxynorketamine is obtainable by a process comprising the steps of:
(i) mixing an active ingredient with microcrystalline cellulose in a high shear mixer;
(ii) adding water, subjecting the mixture to wetting and kneading in the high shear mixer, screening using a screening mill with a rotating impeller, and drying, e.g. in a fluidized bed dryer;
(iii) optionally adding one or more excipient selected from one or more diluent, one or more disintegrant, and one or more glidant, and mixing, in a diffusion mixer;
(iv) optionally adding one or more lubricant, sieving, and mixing in a diffusion mixer.

Also provided is a package comprising a solid oral dosage form of the invention. The package may comprise a bottle comprising the solid oral dosage form of the invention, wherein the bottle is fitted with a safety cap.

The package may comprise a blister pack comprising the solid oral dosage form of the invention.

The blister pack of comprises a metal foil lidding seal. Preferably the metal foil lidding seal comprises an aluminium foil. Preferably the blister pack comprises 7 or 14, or 21, or 28 unit doses.

The bottle or one or more blister pack may comprise instructions for therapeutic administration.

The present invention further provides the solid oral dosage form of the invention for use in a method of treating a patient suffering from a disorder selected from a neurocognitive disorder, a neurodevelopmental disorder, and a psychocognitive disorder, wherein said method comprises the steps of
a. identifying a deficit in a cognitive domain of said patient, wherein the cognitive domain is selected from executive function, perceptual function, learning ability, memory, and attention, and
b. administering to said patient a solid oral dosage form of the present invention.

In some embodiments of the present invention the patient is preferably suffering from a disorder selected from a neurocognitive disorder selected from (i) delirium, (ii) Alzheimer's disease, (iii) pseudodementia, (iv) frontotemporal neurocognitive disorder, (v) dementia with Lewy Bodies (vi) vascular neurocognitive disorder, (vii) multi-infarct dementia, (viii) a tauopathy, (ix) Parkinson's Disease, (x) Huntingdon's disease, (xi) transmissible spongiform encephalopathy, (xii) amyotrophic lateral sclerosis, (xiii) traumatic brain injury, (xiv) post-concussion syndrome, (xv) amnesia, (xvi) substance-induced neurocognitive disorder, (xvii) alcohol-induced neurocognitive disorder, and (xviii) stroke disorder, (xix) hypersomnia, and (xx) clonic perseveration; a neurodevelopmental disorder selected from (i) intellectual disability, (ii) learning disability, (iii) dyslexia, (iv) dyscalculia, (v) dyspraxia, (vi) dysgraphia, (vii) autism-spectrum disorder, (viii) stereotypic movement disorder, (ix) tic disorder, (x) cerebral palsy (xi) fragile-X syndrome, (xii) Down Syndrome, (xiii) attention-deficit disorder, (xiv) hypogonadotropic hypogonadal syndrome (xv) neurotoxicant poisoning, (xvi) foetal alcohol spectrum disorder, (xvii) Minamata disease, and (xviii) Rett Syndrome; or a psychocognitive disorder selected from (i) an obsessive compulsive disorder, (ii) a depressive disorder, (iii) a schizophrenia disorder, (iv) a schizotypal disorder, (v) an anxiety disorder, (vi) substance abuse, and (vii) an avolition disorder.

In particularly preferred embodiments of the present invention, the deficit in said cognitive domain is identified using a computer-based cognitive assessment. Examples of computer-based cognitive assessments which may be used to identify and monitor such cognitive domains, and to measure improvement or deterioration in cognitive domains includes the Cambridge neuropsychological test automated battery
(http://www.cambridgecognition.com/cantab).

In preferred embodiments of the present invention, the method is effective in increasing motivation. In preferred embodiments of the invention, the method is effective in treating diminished motivation, in particular apathy.

### EXAMPLES

### Example 1: Synthesis of 2R,6R-hydroxynorketamine

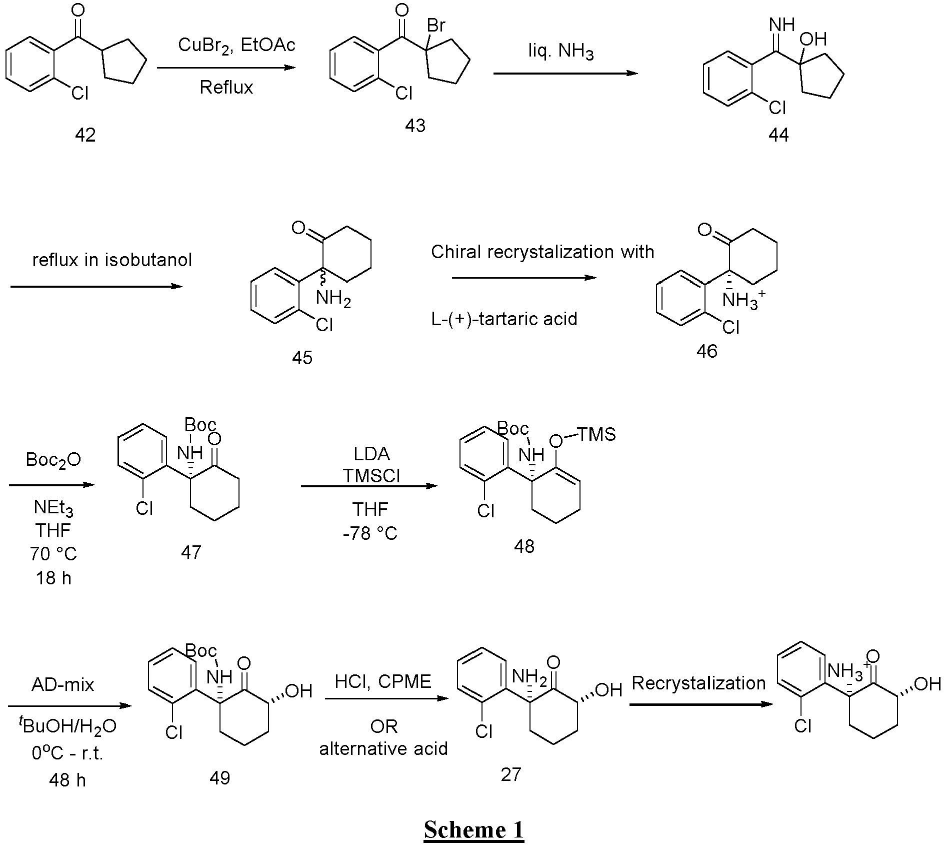

### Step 1

2-Chlorophenyl cyclopentyl ketone (200 g, 0.958 mol, Alfa Aesar, L06448) as a solution in ethyl acetate (2 L) was treated with copper (II) bromide (470 g, 2.104 mol, 2.2 Eq.) and the suspension heated to reflux over 4 hours. Gases were scrubbed with a water scrubber. The reaction mixture was allowed to cool overnight. The reaction mixture was filtered through a pad of silica (1.2 Kg) and washed with ethyl acetate (2 × 1.3 L). The solvent was removed to leave the product **37** as a dark oil (280 g, quantitative yield). The product contained some residual ethyl acetate.

UPLC-LCMS (2-98% MeCN : 10mM ammonium bicarbonate : C₁₈ XBridge column) 97%, RT 1.12 min.

### Step 2

Compound **37** (280 g contains approx. 2% w/w ethyl acetate, 0.958 mol) was stirred whilst liquid ammonia (800 mL, large excess) was added over 5 minutes. The mixture was stirred over 4 h and the ammonia was allowed to evaporate slowly. A cardice/acetone bath was used periodically to slow the rate of evaporation. The residue (a solid mass) was dissolved in THF (1.2 L) and stirred at 40°C for 30 min. The suspension was allowed to cool to room temperature and filtered to remove the inorganics. The solid was washed with THF (200 mL) and the filtrates were evaporated to leave the product **38** as a pale brown solid (236 g, quantitative yield). The product contained some residual THF.

UPLC-LCMS (2-98% MeCN : 10mM ammonium bicarbonate : C₁₈ XBridge column) 94%, RT 0.70 min.

### Step 3

Compound **38** (236 g, contains approx. 8% w/w THF, 0.958 mol) was dissolved in isobutanol (1.5 L) and heated to reflux for 18 hours. UPLC (short acidic method - 2-95% MeCN : 0.1% formic acid : H₂O; C₁₈ CSH column) showed complete conversion to product - RT 0.60 min. The solvent was removed to leave norketamine as a dark oil (223 g, contains residual isobutanol, quantitative yield).

UPLC-LCMS (2-98% MeCN : 10mM ammonium bicarbonate : C₁₈ XBridge column) 80-84%, RT 0.69 min.

### Step 4

A solution of norketamine (10 g, 40.2 mmol, approx. 90% purity) in methanol (30 mL) was treated with a solution of L-(+)-tartaric acid (6.0 g, 40.2 mmol, 1 Eq.) in methanol (70 mL) and the solution stirred at room temperature overnight. The solvent was removed and the solid residue was triturated with acetone (80 mL). The solid was filtered and then recrystallised from acetone or isopropanol (TBC) three times to give the R-Norketamine salt, approximately 10% yield from acetone.

UPLC-LCMS (2-98% MeCN : 10mM ammonium bicarbonate : C₁₈ XBridge column) 99%, RT 0.69 min.

### Step 5

(R)-Norketamine tartrate (120 mg, 0.32 mmol) was suspended in dry THF (3.2 mL, 0.1 M) with stirring under an argon atmosphere; to the stirring solution NEt₃ (179 µL, 1.28 mmol, 4 Eq.) was added as a single portion over 30 seconds. Di-*tert-*butyl-dicarbonate (91 mg, 0.417 mmol, 1.3 Eq.) was added to the reaction vessel as a single portion under an argon stream and following the complete addition the reaction was heated to 70 °C for 18 h. The reaction was followed *via* TLC (SiO₂; 25% EtOAc in heptane; visualized with KMnO₄ stain) as well as reverse phase UPLC-LCMS (2-95% MeCN : 0.1% formic acid : H₂O; C₁₈ CSH column) until complete consumption of Norketamine was observed (complete after overnight reaction ~18 h). The reaction was cooled to room temperature then diluted with EtOAc (10 mL) then washed with NH₄Cl (Sat. Aq. 2 × 10 mL) then the separated organic phase was washed with brine (10 mL), dried over Na₂SO₄, filtered and then concentrated *in vacuo* to afford crude compound **26.**

Crude material was purified *via* flash column chromatography (15-30% EtOAc in heptane) to afford **26** (102 mg, 98% yield).

### Step 6

Performed in glassware dried at ~300-400 °C under an argon atmosphere for 5 minutes.

Diisopropylamine (107 µL, 0.76 mmol) was dissolved in dry THF (1.5 mL) with stirring under an argon atmosphere then the mixture was cooled to -78 °C. *n*-BuLi (1.03 M in hexanes, freshly titrated; 708 µL; 2 Eq.) was added dropwise over 2 minutes and the reaction mixture was stirred for 15 minutes; after which a solution of **26** (117 mg, 0.36 mmol) in dry THF (2 mL) was added dropwise over 2 minutes and the reaction stirred for 25 minutes. Chlorotrimethylsilane (62 µL, 0.79 mmol, freshly distilled from CaH₂) was added to the reaction dropwise over 1 minute and the reaction stirred for 15 minutes at -78 °C then warmed to 0 °C and stirred for 1 h. After this time the reaction was diluted with heptane (30 mL), washed with NaHCO₃ (Sat. Aq. 30 mL) and the organic phase was separated and dried over Na₂SO₄, filtered and then concentrated *in vacuo* to afford crude **42.**

The crude material was purified *via* column chromatography (2-20% EtOAc in heptane) to afford **42** as a clear colourless oil (143 mg, 100% yield).

### Step 7

Compound **42** (20 mg, 0.05 mmol) was dissolved in *tert*-butanol (250 µL) with rapid stirring, then water (250 µL) was added as a single portion and the reaction was cooled to 0 °C. AD-mix β (71 mg, 0.05 mmol, 1 Eq.) was added as a single portion. The reaction was monitored *via* TLC (25% EtOAc in heptane; visualized with KMnO₄ stain) and UPLC-LCMS (2-95% MeCN : 0.1% formic acid : H₂O; C₁₈ CSH); after 18 h a further addition of AD-mix β (35 mg, 0.5 Eq.) was added as a single portion and the reaction stirred at ambient temperature for a further 24 h at which point only trace **42** was observed *via* TLC (not detectable *via* LCMS) and the reaction was diluted with EtOAc (5 mL). Na₂S₂O₃ (Sat. Aq., 4 mL) was added dropwise over 1 minute and the biphasic mixture stirred rapidly for 25 minutes; after which the organic phase was separated and the aqueous phase extracted with EtOAc (3 × 5 mL). The organic phases were combined, dried over Na₂SO₄, filtered and then concentrated *in vacuo* to afford crude **43.**

Purification was achieved *via* column chromatography (20 - 50% EtOAc in heptane) to afford **43** (19 mg, 95% yield).

### Step 8

Compound **43** (12 mg, 0.037 mmol) was transferred into a round bottom flask under an argon atmosphere with a stirrer bar and HCl (3M in CPME, 2 mL; 162 Eq (large excess)) was added with stirring; and the reaction was stirred under an argon atmosphere for 18 hours. After this time the reaction was filtered to collect the formed precipitate, which was then washed with pentane (3 × 3 mL) and dried under an argon stream to afford 2R,6R-hydroxynorketamine as a white crystalline solid (8.4 mg, 82% yield).

### Example 2: Formation of crystalline forms of 2R,6R-hydroxynorketamine salts

### 2.1 Solvent Solubility

90 mg of 2R,6R-hydroxynorketamine free base was dissolved in 18 mL of dichloromethane. 1 mL aliquots of the solution were allowed to evaporate in a fume hood. PLM images of the white solid that remained in the vial in which the material had been dissolved were recorded.

A known volume aliquot (typically 5 volumes) of solvent was added to approximately 5 mg 2R,6R-hydroxynorketamine. Between each addition, the mixture was checked for dissolution and where no dissolution was apparent, the mixture was heated to ca. 40°C and checked again. This procedure was continued until dissolution was observed or until 1 mL of solvent had been added. Any remaining solids were analysed by XRPD. Where the material had fully dissolved, the solution was left to evaporate and any resulting solids were analysed by XRPD.

### 2.2 pKₐ analysis

The sample pKa was determined using the potentiometric (pH-metric) technique following attempts to determine pKₐ via UV spectroscopic techniques.

UV-metric: The sample was initially titrated in a fast-UV triple titration between pH 2.0 - 12.0 at concentrations of 31 - 19 µM, under aqueous conditions. No evidence of any sample ionisation within the investigated pH range was inferred from the spectroscopic data obtained, meaning that any ionisable groups were remote from chromophores. Therefore, the sample was analysed using the pH-metric method.

pH-metric: The sample was subsequently titrated using the potentiometric technique to determine the non-UV active pKₐs. A triple titration was carried out under methanol-water co-solvent conditions from pH 2.0 - 12.0 at concentrations of 0.9 - 0.6 mM (the methanol mixing ratio varied from 53.0 to 33.3 % w/w). No precipitation of the sample from solution was observed so the pKₐ was determined from the potentiometric data collected, by Yasuda-Shedlovsky extrapolation of the individual results obtained. pKₐ of 2R,6R-hydroxynorketamine was calculated as 6.51+/- 0.02.

Candidate counterions were selected based on pKₐ compatibility.

### 2.3 Crystallisation of 2R,6R-hydroxynorketamine free base

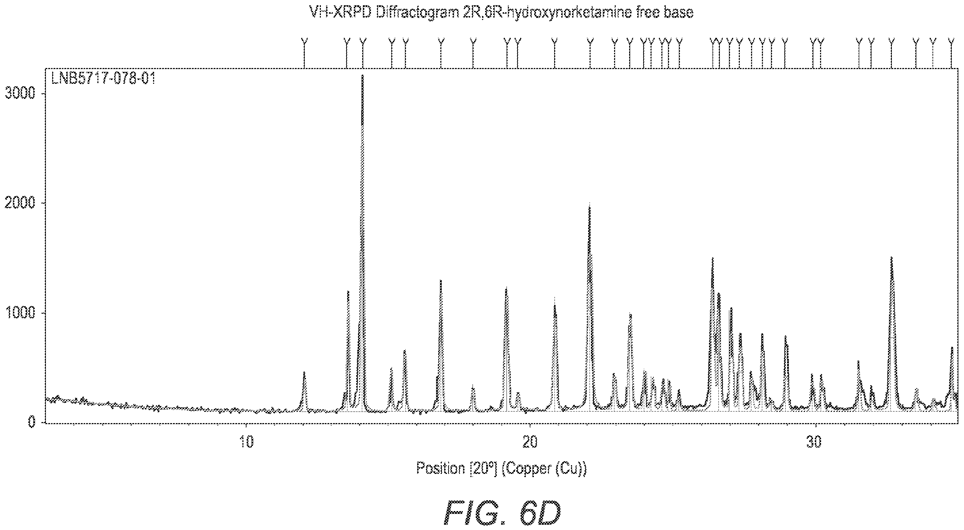

On addition of the 0.5 mL of acetonitrile to the 10mg 2R,6R-hydroxynorketamine free base, the yellowish gum dissolved and white solids immediately crashed out, leaving a pale yellow clear solution. The solids were analysed by XRPD. The diffractogram is presented in Figure 6D.

### 2.4 Crystallisation of 2R,6R-hydroxynorketamine hydrochloride

20 mg of 2R,6R-hydroxynorketamine was suspended in 100 µL each of acetone, acetonitrile, ethanol and tetrahydrofuran (THF). 87.6 µL of 1M hydrochloric acid stock solution prepared in water (1.05 equivalents) was added and the mixtures were then thermally cycled whilst being stirred for 48 hours according to the following program:
25° C to 5 ° C at 0.1° C/min; Hold at 5° C for 1 hour; 5° C to 25 ° C at 0.1° C/min; Hold at 5° C for 1 hour.

No solids were recovered post-thermal cycling so the solutions were uncapped and allowed to evaporate at ambient temperature and pressure.

No solids were recovered post-evaporation anti-solvent addition was carried out using t-butyl methyl ether (tBME) and the mixtures were matured for 16 hours. Further anti-solvent addition was then carried out and the mixtures were matured for 72 hours.

Clear solids of 2R,6R-hydroxynorketamine hydrochloride were recovered from acetone after treatment with anti-solvent. XRPD analysis was carried out on the solids. Significant preferred orientation was found, likely due to formation of needle-like crystals. The material was removed from the XRPD plate, ground and then re-analysed. Preferred orientation was again observed although in different peak positions.

### 2.5 Crystallisation of 2R,6R-hydroxynorketamine L-tartrate

20 mg of 2R,6R-hydroxynorketamine free base was suspended in 100 µL of organic solvent. 87.6 |iL of 1M L-Tartaric acid stock solution prepared in water (1.05 equivalents) was added and the mixtures were then thermally cycled whilst being stirred for 48 hours according to the following program: 25° C to 5 ° C at 0.1° C/min; hold at 5° C for 1 hour; 5° C to 25° C at 0.1° C/min; hold at 5° C for 1 hour.

Solids were recovered from all solvent systems investigated. Post-thermal cycling, white solids were identified in acetone, acetonitrile and THF. Clear Solids were recovered from ethanol post-evaporation.

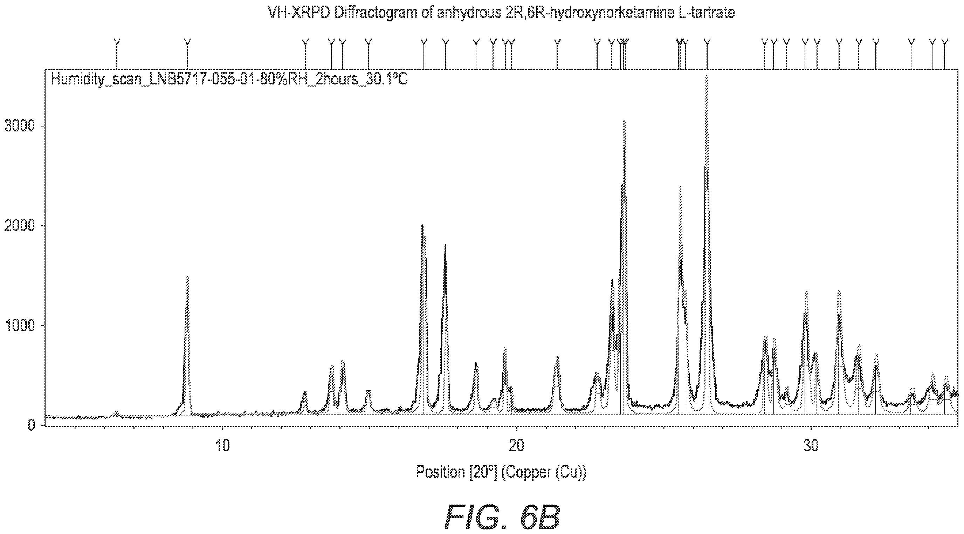

Subsequently, 2.5 mL of acetonitrile was added to 500 mg of 2R,6R-hydroxynorketamine free base. 2190 µL of 1M L-tartaric acid stock solution (1.05 equivalents) prepared in water was added and the mixture was thermally cycled for 72 hours according to the following program whilst being stirred: 25° C to 5 ° C at 0.1° C/min; hold at 5° C for 1 hour; 5° C to 25 ° C at 0.1° C/min; hold at 5° C for 1 hour. A small portion of solid was removed post thermal cycling for wet XRPD analysis to ensure that the correct material had been prepared. The remaining solids were isolated by Buchner filtration and dried under vacuum at ambient temperature for 3 hours. Further analysis identified the existence of a hydrated form and an anhydrous form of 2R,6R-hydroxynorketamine L-tartrate. The diffractograms of each form are presented in Figures 6A and 6B.

### 2.6 Crystallisation of 2R,6R-hydroxynorketamine difumarate

20 mg of 2R,6R-hydroxynorketamine was suspended in 187.6 µL of organic solvent. 0.2 mg of fumaric acid (1.05 equivalents) was added neat and the mixtures were then thermally cycled whilst being stirred for 72 hours according to the following program: 25° C to 5 ° C at 0.1° C/min; hold at 5° C for 1 hour; 5° C to 25 ° C at 0.1° C/min; hold at 5° C for 1 hour.

White solids were recovered from all solvent systems investigated. Post-thermal cycling, white solids were identified in acetone, acetonitrile, ethanol and THF.

The ¹H-NMR spectrum of the fumaric acid solid recovered from acetonitrile is presented in Figure 7. The singlet at 6.6 ppm with an integral of 4.2 protons gives 2 equivalents of fumaric acid per API. The presence of 2 equivalents of fumaric acid suggests the presence of a salt co-crystal.

Subsequently, 2.5 mL of acetonitrile was added to 500 mg of 2R,6R-hydroxynorketamine. 496.3 mg of fumaric acid (2.05 equivalents) was added and the mixture was thermally cycled for 72 hours according to the following program whilst being stirred: 25° C to 5 ° C at 0.1° C/min; hold at 5° C for 1 hour; 5° C to 25 ° C at 0.1° C/min; hold at 5° C for 1 hour.

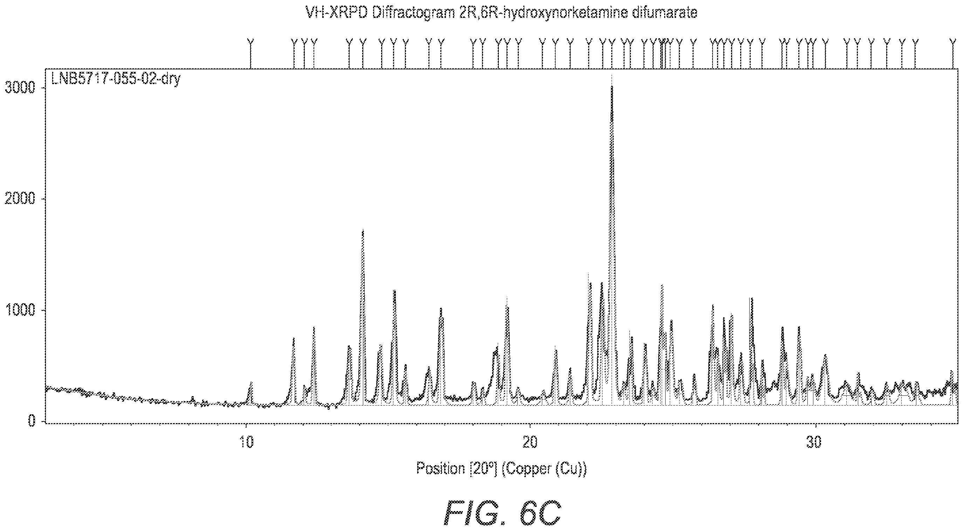

A small portion of solid was removed post thermal cycling for wet XRPD analysis to ensure that the correct material had been prepared. The remaining solids were isolated by Buchner filtration and dried under vacuum at ambient temperature for 3 hours. The diffractogram is presented in Figure 6C.

### 2.7 Crystallisation of 2R,6R-hydroxynorketamine-L-malate

20 mg of 2R,6R-hydroxynorketamine was suspended in 100 µL of organic solvent. 87.6 µL of 1M L-Malic acid stock solution prepared in water (1.05 equivalents) was added and the mixtures were then thermally cycled whilst being stirred for 72 hours according to the following program: 25° C to 5 ° C at 0.1° C/min; hold at 5° C for 1 hour; 5° C to 25 ° C at 0.1° C/min; hold at 5° C for 1 hour. No solids were recovered post-thermal cycling so the solutions were uncapped and allowed to evaporate at ambient temperature and pressure.

No solids were recovered post-evaporation so anti-solvent addition was carried out using tBME and the mixtures were matured for 16 hours. Further anti-solvent addition was carried out and the mixtures were matured for 72 hours. Clear solids were recovered from acetonitrile and ethanol following anti-solvent addition.

### 2.8 Crystallisation of 2R,6R-hydroxynorketamine D-malate

20 mg of 2R,6R-hydroxynorketamine was suspended in 100 µL of organic solvent. 87.6 µL of 1M D-Malic acid stock solution prepared in water (1.05 equivalents) was added and the mixtures were then thermally cycled whilst being stirred for 72 hours according to the following program: 25° C to 5 ° C at 0.1° C/min; hold at 5° C for 1 hour; 5° C to 25 ° C at 0.1° C/min; hold at 5° C for 1 hour. No solids were recovered post-thermal cycling so the solutions were uncapped and allowed to evaporate at ambient temperature and pressure.

No solids were recovered post-evaporation so anti-solvent addition was carried out using tBME and the mixtures were matured for 16 hours. Further anti-solvent addition was carried out and the mixtures were matured for 72 hours. Clear solids were recovered from acetone, acetonitrile, ethanol and THF following anti-solvent addition.

### 2.9 Crystallisation of 2R,6R-hydroxynorketamine citrate

20 mg of 2R,6R-hydroxynorketamine was suspended in 100 µL of organic solvent. 87.6 µL of 1M citric acid stock solution prepared in water (1.05 equivalents) was added and the mixtures were then thermally cycled whilst being stirred for 72 hours according to the following program: 25° C to 5° C at 0.1° C/min; hold at 5° C for 1 hour; 5° C to 25 ° C at 0.1° C/min; hold at 5° C for 1 hour. Solids were recovered from ethanol and THF post anti-solvent addition.

### 2.10 Crystallisation of 2R,6R-hydroxynorketamine L-pyroglutamate

20 mg of 2R,6R-hydroxynorketamine was suspended in 100 µL of organic solvent. 87.6 µL of 1M citric acid stock solution prepared in water (1.05 equivalents) was added and the mixtures were then thermally cycled whilst being stirred for 72 hours according to the following program: 25° C to 5° C at 0.1° C/min; hold at 5° C for 1 hour; 5° C to 25 ° C at 0.1° C/min; hold at 5° C for 1 hour.

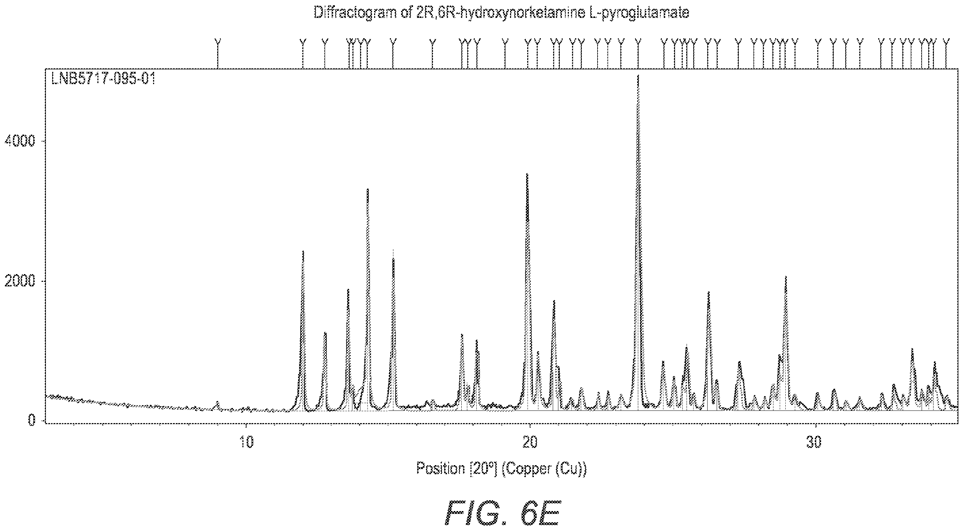

Crystalline material was recovered from acetone and acetonitrile post thermal cycling. The diffractogram is presented in Figure 6E. The same XRPD pattern was found from THF post re-dissolving and maturation.

### 2.11 Crystallisation of 2R,6R-hydroxynorketamine acetate

20 mg of 2R,6R-hydroxynorketamine was suspended in 100 µL of organic solvent. 87.6 µL of 1M acetic acid stock solution prepared in water (1.05 equivalents) was added and the mixtures were then thermally cycled whilst being stirred for 72 hours according to the following program: 25° C to 5 ° C at 0.1° C/min; hold at 5° C for 1 hour; 5° C to 25 ° C at 0.1° C/min; hold at 5° C for 1 hour.

No solids were recovered post-thermal cycling so the solutions were uncapped and allowed to evaporate at ambient temperature and pressure. No solids were recovered post-evaporation anti-solvent addition was carried out using tBME and the mixtures were matured for 16 hours. Further anti-solvent addition was carried out and the mixtures were matured for 72 hours. Clear solids were recovered from the acetic acid salt screen in ethanol post anti-solvent addition.

### 2.12 Crystallisation of 2R,6R-hydroxynorketamine tosylate

20 mg of 2R,6R-hydroxynorketamine was suspended in 100 µL of organic solvent. 87.6 µL of 1M acetic acid stock solution prepared in water (1.05 equivalents) was added and the mixtures were then thermally cycled whilst being stirred for 72 hours according to the following program: 25° C to 5 ° C at 0.1° C/min; hold at 5° C for 1 hour; 5° C to 25 ° C at 0.1° C/min; hold at 5° C for 1 hour.

Crystalline solids were recovered from acetone and THF post thermal-cycling. Acetonitrile and ethanol solutions were uncapped and allowed to evaporate at ambient temperature and pressure. Crystalline solids were recovered from acetonitrile post-evaporation.

### 2.13 Failure to obtain crystalline salt forms from phosphoric acid, sulfuric acid, methane sulfonic acid, benzene sulfonic acid, benzoic acid, D,L-lactic acid, and D,L-mandelic acid

20 mg of 2R,6R-hydroxynorketamine was suspended in 100 µL of organic solvent (each of acetone, acetonitrile, ethanol and THF). Crystallization was attempted in each solvent system with each of phosphoric acid, sulfuric acid, methane sulfuric acid, benzene sulfonic acid, benzoic acid, D,L-lactic acid, and D,L-mandelic acid. 87.6 µL of 1M acid stock solution prepared in water (1.05 equivalents) was added and the mixtures were then thermally cycled whilst being stirred for 48 hours according to the following program: 25° C to 5 ° C at 0.1° C/min; hold at 5° C for 1 hour; 5° C to 25 ° C at 0.1° C/min; hold at 5° C for 1 hour. For each acid no solids were recovered post-thermal cycling so the solutions were uncapped and allowed to evaporate at ambient temperature and pressure. For each acid, no solids were recovered post-evaporation so anti-solvent addition was carried out using tBME and the mixtures were matured for 16 hours. Further anti-solvent addition was carried out and the mixtures were matured for 72 hours. For each acid, no solids were recovered following anti-solvent addition.

### METHODS OF ANALYSIS

### X-ray Powder Diffraction (XRPD) - Transmission

XRPD analysis was carried out on a PANalytical X'pert pro, scanning the samples between 3 and 35° 2θ. The material was gently ground to release any agglomerates and loaded onto a multi-well plate with Kapton or Mylar polymer film to support the sample. The multi-well plate was then placed into the diffractometer and analysed using Cu K radiation (α1 λ = 1.54060 Å; α2 = 1.54443 Å; β = 1.39225 Å; α1 : α2 ratio = 0.5) running in transmission mode (step size 0.0130° 2θ) using 40 kV / 40 mA generator settings.

### X-ray Powder Diffraction (XRPD) - Reflectance

XRPD analysis was carried out on a Philips X'pert Pro Multipurpose Diffractometer using a spinning stage with autosampler, scanning the samples between 3 and 35° 2θ. The material was loaded onto a circular sample holder and flattened using a glass slide. The sample holder was then loaded into position on the autosampler cassette and analysed using Cu K radiation (α1 λ = 1.54060 Å; α2 = 1.54443 Å; β = 1.39225 Å; α1 : α2 ratio = 0.5) running in reflectance mode (step size 0.013° 2θ, time per step 59.67 s) using 40 kV / 40 mA generator settings and fitted with a Ni Cu Kβ filter).

### Polarised Light Microscopy (PLM)

The presence of crystallinity (birefringence) was determined using an Olympus BX50 polarising microscope, equipped with a Motic camera and image capture software (Motic Images Plus 2.0). All images were recorded using the 20x objective, unless otherwise stated.

### Thermogravimetric Analysis (TGA)

Approximately 5 mg of material was weighed into an open aluminium pan and loaded into a simultaneous thermogravimetric/differential thermal analyser (TG/DTA) and held at room temperature. The sample was then heated at a rate of 10°C/min from 20°C to 300°C during which time the change in sample weight was recorded along with any differential thermal events (DTA). Nitrogen was used as the purge gas, at a flow rate of 300 cm³/min.

### Differential Scanning Calorimetry (DSC)

Approximately, 5 mg of material was weighed into an aluminium DSC pan and sealed non-hermetically with a pierced aluminium lid. The sample pan was then loaded into a Seiko DSC6200 (equipped with a cooler) cooled and held at 20°C. Once a stable heat-flow response was obtained, the sample and reference were heated to 180°C at scan rate of 10°C/min and the resulting heat flow response monitored. Nitrogen was used as the purge gas, at a flow rate of 50 cm3/min.

### Infrared Spectroscopy (IR)

Infrared spectroscopy was carried out on a Bruker ALPHA P spectrometer. Sufficient material was placed onto the centre of the plate of the spectrometer and the spectra were obtained using the following parameters:
Resolution: 4 cm⁻¹; Background Scan Time: 16 scans; Sample Scan Time: 16 scans; Data Collection: 4000 to 400 cm-1; Result Spectrum: Transmittance; Software: OPUS version 6

### Nuclear Magnetic Resonance (NMR)

NMR experiments were performed on a Bruker AVIIIHD spectrometer equipped with a DCH cryoprobe operating at 500.12MHz for protons. Experiments were performed in deuterated DMSO-d6 and each sample was prepared to *ca.* 10 mM concentration.

### Dynamic Vapour Sorption (DVS)

Approximately, 10 mg of sample was placed into a mesh vapour sorption balance pan and loaded into a DVS-1 dynamic vapour sorption balance by Surface Measurement Systems. The sample was subjected to a ramping profile from 40 - 90% relative humidity (RH) at 10% increments, maintaining the sample at each step until a stable weight had been achieved (dm/dt 0.004%, minimum step length 30 minutes, maximum step length 500 minutes) at 25°C. After completion of the sorption cycle, the sample was dried using the same procedure to 0% RH and then a second sorption cycle back to 40% RH. Two cycles were performed. The weight change during the sorption/desorption cycles were plotted, allowing for the hygroscopic nature of the sample to be determined. XRPD analysis was then carried out on any solid retained.

Approximately, 10-20 mg of sample was placed into a mesh vapour sorption balance pan and loaded into a DVS Intrinsic dynamic vapour sorption balance by Surface Measurement Systems. The sample was subjected to a ramping profile from 40 - 90% relative humidity (RH) at 10% increments, maintaining the sample at each step until a stable weight had been achieved (dm/dt 0.004%, minimum step length 30 minutes, maximum step length 500 minutes) at 25°C. After completion of the sorption cycle, the sample was dried using the same procedure to 0% RH and then a second sorption cycle back to 40% RH. Two cycles were performed. The weight change during the sorption/desorption cycles were plotted, allowing for the hygroscopic nature of the sample to be determined. XRPD analysis was then carried out on any solid retained.

### Gravimetric Vapour Sorption (GVS)

Approximately 10-20 mg of sample was placed into a mesh vapour sorption balance pan and loaded into an IGASorp Moisture Sorption Analyser balance by Hiden Analytical. The sample was subjected to a ramping profile from 40 - 90% relative humidity (RH) at 10% increments, maintaining the sample at each step until a stable weight had been achieved (98% step completion, minimum step length 30 minutes, maximum step length 60 minutes) at 25°C. After completion of the sorption cycle, the sample was dried using the same procedure to 0 % RH, and finally taken back to the starting point of 40% RH. Two cycles were performed. The weight change during the sorption/desorption cycles were plotted, allowing for the hygroscopic nature of the sample to be determined.

### Variable Humidity X-ray powder diffraction (VH-XRPD)

VH-XRPD analysis was carried out on a Philips X'Pert Pro Multipurpose diffractometer equipped with a humidity chamber. The samples were scanned between 4 and 35.99 °2θ using Cu K radiation (α1 λ = 1.54060 Å; α2 = 1.54443 Å; β = 1.39225 Å; α1 : α2 ratio = 0.5) running in Bragg-Brentano geometry (step size 0.008 °2θ) using 40 kV / 40 mA generator settings. Measurements were performed at 40 %RH, 80 %RH, 10 %RH, 0 %RH. The temperature was raised to 60° C, 100° C and 120° C all at 40 %RH.

### High Performance Liquid Chromatography-Ultraviolet Detection (HPLC-UV)

Instrument: Agilent 1100/Dionex Ultimate 3000; Column: Ace Excel-3 C18-AR, 75 mm x 4.6 mm 3 µm; Column Temperature: 40° C; Autosampler Temperature: Ambient; UV wavelength: 210 nm; Injection Volume: 10; Flow Rate: 1 mL/min; Mobile Phase A: 10mM Ammonium Formate pH8; Mobile Phase B: 10mM Ammonium Formate pH8:Acetonitrile 20:80

**Table 5**

| Gradient program: **Time (minutes)** | **Solvent B [%]** |
|---|---|
| 0 | 12 |
| 1 | 12 |
| 11 | 100 |
| 11.1 | 12 |
| 15 | 12 |

### Mass Spectrometry

Instrument: LCQ Advantage Ion Trap MS; Sample concentration: 1 mg/ml, +ve ion mode by infusion; Source voltage (kV): 4.50; Source current (µA): 80.00; Sheath gas flow rate: 20; Aux/Sweep gas flow rate: 0; Capillary voltage (V): 8.0; Capillary temp (oC): 200; Tube lens (V, Sp): 40; HPLC conditions as above.

### EXAMPLE 3: Thermometric analysis of crystal forms of 2R,6R-hydroxynorketamine

**TG/DVA Analysis of 2R,6R-hydroxynorketamine hydrochloride** TG/DTA shows that there is a sharp mass loss of 17.3 wt.% with an associated thermal event at 159° C. The sharp mass loss is attributed to loss of bound HCl which would be lost as a gas at that temperature, hence the sharp loss. The 17.3 wt.% loss calculates to 1 equivalent of HCl.

**TG/DVA Analysis of 2R,6R-hydroxynorketamine difumarate** Figure 5A presents the TG/DTA thermogram of the solid recovered from acetonitrile. The material degrades above 159° C. There were no thermal events in the DTA.

The 1H-NMR spectrum of the fumaric acid solid recovered from acetonitrile shows a singlet at 6.6 ppm with an integral of 4.2 protons gives 2 equivalents of fumaric acid per API. The presence of 2 equivalents of fumaric acid suggests the presence of a salt co-crystal.

**TG/DVA Analysis of 2R,6R-hydroxynorketamine L-tartrate** Figure 5B presents the TG/DTA thermogram of the solid recovered from acetonitrile. A 5.8 wt.% loss is observed from the onset of heating with a related endotherm from the onset of heating with a peak at 74° C. The material degrades above 157° C.

1H-NMR analysis was carried out on the solids recovered from acetonitrile. The singlet at 4.15 with an integral of 2.2 protons equals one equivalent of L-tartaric acid. This confirms that a L-tartrate salt has been made.

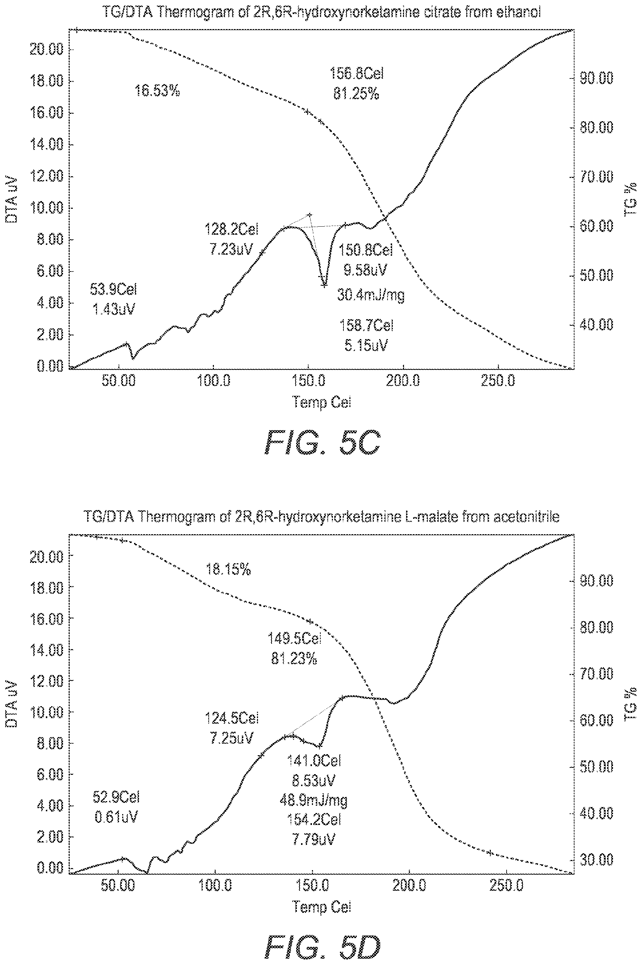

**TG/DVA Analysis of 2R,6R-hydroxynorketamine citrate** TG/DTA analysis was carried out on the solid recovered from ethanol. The thermogram is presented in Figure 5C. There is a loss of 16.5 wt.% from the onset of heating with an associated endothermic event. There is an endotherm with onset 151° C with a peak at 159° C related to degradation of the material. The material degrades above 157° C.

**TG/DVA Analysis of 2R,6R-hydroxynorketamine L-malate** TG/DTA analysis was carried out on solids from acetonitrile, shown in Figure 5D. There is a loss of 18 wt.% from the onset of heating with a related endotherm. The material degrades above 150°C.

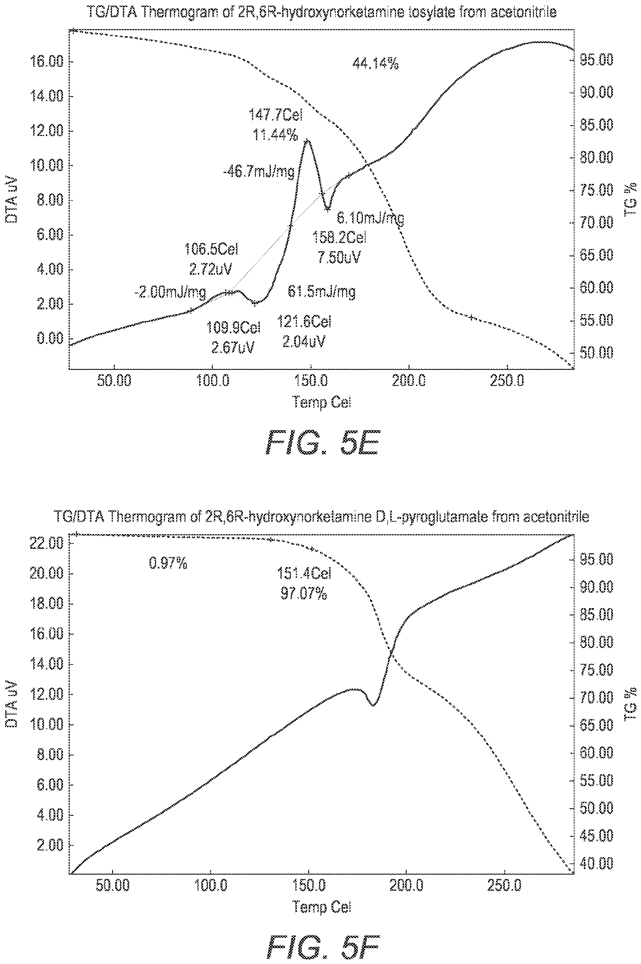

**TG/DVA Analysis of 2R,6R-hydroxynorketamine toluene sulfonate** TG/DTA analysis was carried out on solids from acetonitrile, shown in Figure 5E. Complex thermal events are observable.

**TG/DVA Analysis of 2R,6R-hydroxynorketamine D,L-pyroglutamate** TG/DTA analysis was carried out on solids from acetonitrile, shown in Figure 5F. There is a 1% mass loss from onset of heating. The material degrades above 159° C. There were no thermal events in the DVA.

### Example 4: Solubility analysis of 2R,6R-hvdroxynorketamine L-pyroglutamate

A solubility assessment was carried out on 2R,6R-hydroxynorketamine L-pyroglutamate in various vehicles.

A solution of the received material in water for injection was submitted for analysis after being shaken at 400 rpm for 24 hours at 25°C. The solution was filtered through a pre-heated (at 25°C) 0.22µm PTFE filter into a HPLC vial. Samples were analysed after being diluted in deionised water to achieve a concentration of approximately 1000µg/mL. HPLC method parameters used are provided in Table 6.

**Table 6**

| **HPLC-UV Parameters** | |
|---|---|
| System | Thermo Ultimate 3000 uHPLC with DAD |
| Analytical column | Ace Excel 3 C18 Ar 10mm × 3mm, particle size: 1.7µm |
| Column temperature | 40°C |
| Flow rate | 0.75 ml/min |
| Injection volume | 2.8 µl |
| Autosamper temperature | Ambient |

The method used is provided in Table 7. Analysis using this column showed that all peaks were sharp and no tailing was observed. Duplicate injections of standards gave consistent peak areas and no retention time drift or interfering peaks were observed.

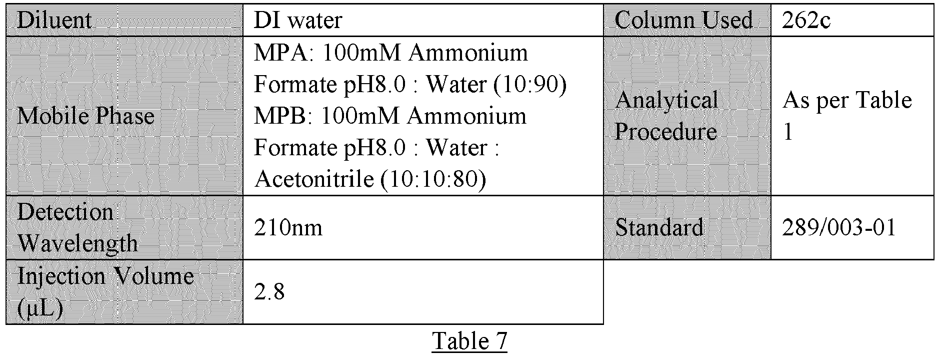

Following the successful uHPLC solubility method assessment, solubility assessment of 2R,6R-hydroxynorketamine L-pyroglutamate in water for injection was conducted. A single replicant of the sample was prepared for the solubility assessment. Approximately 150mg of 2R,6R-hydroxynorketamine L-pyroglutamate was weighed into a 2mL HPLC vial prior to the addition of 1.0mL of water for injection, forming a saturated solution. The sample was shaken at approximately 400 rpm for 24 hours at 25°C. After this the sample was hot filtered using pre-heated (at 25°C) 0.22µm PTFE syringe filters into a pre-heated HPLC vial. The samples were immediately analysed using the uHPLC method outlined above with analysis being performed on the samples diluted and undiluted for each of the major peaks observed. Analysis confirmed that 2R,6R-hydroxynorketamine L-pyroglutamate has a saturated solubility of 64 mg/mL in water for injection at 25°C, demonstrating significantly superior aqueous solubility over known crystal forms of 2R,6R-hdyroxynorketamine hydrochloride.

This method was repeated to determine solubility of 2R,6R-hydroxynorketamine L-pyroglutamate in 0.9% saline. HPLC-UV parameters and methods were the same as described above. Analysis confirmed that 2R,6R-hydroxynorketamine L-pyroglutamate has a saturated solubility of 87 mg/mL in 0.9% saline at 25°C.

### Example 5: High concentration solid oral dosage formulations of 2R,6R-hydroxynorketamine

**Formulation (a)**

| **2R,6R-HNK Capsule** | | | |
|---|---|---|---|
| **Ingredient/Component** | **~%w/w** | **Unit Quantity** | **Reference** |
| 2R,6R-hydroxynorketamine as difumarate salt: (free base equivalent): | 45 (23) | 98 (50 mg) | In-house |
| Dicalcium phosphate: | 50 | 108 mg | Ph.Eur |
| Sodium lauryl sulphate: | 5 | 10 mg | Ph.Eur |
| Coni-Snap^{®} Size 3 hard gelatin capsule or Vcap^{®} Size 3 HPMC capsule: | - | One | In-house |
| Total: | - | 216 mg | |

**Formulation (b)**

| **2R,6R-HNK Capsule** | | | |
|---|---|---|---|
| **Ingredient/Component** | **~%w/w** | **Unit Quantity** | **Reference** |
| 2R,6R-hydroxynorketamine as hydrochloride salt: (free base equivalent): | 73 (63) | 138 mg (120 mg) | In-house |
| Starch | 25 | 47 mg | Ph.Eur |
| Colloidal silica | 2 | 4 mg | Ph.Eur |
| Coni-Snap^{®} Size 3 hard gelatin capsule or Vcap^{®} Size 3 HPMC capsule | - | One | In-house |
| Total | - | 189 mg | |

**Formulation (c)**

| **2R,6R-HNK Capsule** | | | |
|---|---|---|---|
| **Ingredient/Component** | **~%w/ w** | **Unit Quantity** | **Reference** |
| 2R,6R-hydroxynorketamine as free base | 93 | 200 mg | In-house |
| Microcrystalline cellulose | 6 | 13 mg | Ph.Eur |
| Colloidal silica | 1 | 3 mg | |
| Coni-Snap^{®} Size 3 hard gelatin capsule or Vcap^{®} Size 3 HPMC capsule | - | One | In-house |
| Total | - | 216 mg | |

**Formulation (d)**

| **2R,6R-HNK Capsule** | | | |
|---|---|---|---|
| **Ingredient/Component** | **~%w/w** | **Unit Quantity** | **Reference** |
| 2R,6R-hydroxynorketamine as pyroglutamate salt: (free base equivalent): | 36 (23) | 77 (50 mg) | In-house |
| Dicalcium phosphate: | 60 | 130 mg | Ph.Eur |
| Sodium lauryl sulphate: | 4 | 9 mg | Ph.Eur |
| Coni-Snap^{®} Size 3 hard gelatin capsule or Vcap^{®} Size 3 HPMC capsule: | - | One | In-house |
| Total: | - | 216 mg | |

**Formulation (e)**

| **2R,6R-HNK Capsule** | | | |
|---|---|---|---|
| **Ingredient/Component** | **~%w/w** | **Unit Quantity** | **Reference** |
| 2R,6R-hydroxynorketamine as pyroglutamate salt: (free base equivalent): | 86 (56) | 185 mg (120 mg) | In-house |
| Starch | 13 | 28 mg | Ph.Eur |
| Colloidal silica | 1 | 3 mg | Ph.Eur |
| Coni-Snap^{®} Size 3 hard gelatin capsule or Vcap^{®} Size 3 HPMC capsule | - | One | In-house |
| Total | - | 216 mg | |

**Formulation (f)**

| **2R,6R-HNK Tablet** | | | |
|---|---|---|---|
| **Ingredient/Component** | **~%w/w** | **Unit Quantity** | **Reference** |
| 2R,6R-hydroxynorketamine as difumarate salt: (free base equivalent): | 49 (25) | 98 (50 mg) | In-house |
| Pregelatinised starch: | 35 | 70 mg | Ph.Eur |
| Calcium carbonate: | 10 | 20 mg | Ph.Eur |
| Crospovidone: | 4 | 8 mg | Ph.Eur |
| Stearic acid | 2 | 4 mg | Ph.Eur |
| Total: | - | 200 mg | |

**Formulation (g)**

| **2R,6R-HNK Tablet** | | | |
|---|---|---|---|
| **Ingredient/Component** | **~%w/w** | **Unit Quantity** | **Reference** |
| 2R,6R-hydroxynorketamine as hydrochloride salt: (free base equivalent): | 69 (60) | 138 (120 mg) | In-house |
| Microcrystalline cellulose: | 28 | 56 mg | Ph.Eur |
| Colloidal Silica: | 2 | 4 mg | Ph.Eur |
| Magnesium stearate: | 1 | 2 mg | Ph.Eur |
| Total: | - | 200 mg | |

**Formulation (h)**

| **2R,6R-HNK Tablet** | | | |
|---|---|---|---|
| **Ingredient/Component** | **~%w/w** | **Unit Quantity** | **Reference** |
| 2R,6R-hydroxynorketamine as pyroglutamate salt: (free base equivalent): | 38 (25) | 76 (50 mg) | In-house |
| Pregelatinised starch: | 46 | 92 mg | Ph.Eur |
| Calcium carbonate: | 10 | 20 mg | Ph.Eur |
| Crospovidone: | 4 | 8 mg | Ph.Eur |
| Stearic acid | 2 | 4 mg | Ph.Eur |
| Total: | - | 200 mg | |

**Formulation (i)**

| **2R,6R-HNK Tablet** | | | |
|---|---|---|---|
| **Ingredient/Component** | **~%w/w** | **Unit Quantity** | **Reference** |
| 2R,6R-hydroxynorketamine as pyroglutamate salt: (free base equivalent): | 92 (60) | 184 (120 mg) | In-house |
| Microcrystalline cellulose: | 5 | 10 mg | Ph.Eur |
| Colloidal Silica: | 2 | 4 mg | Ph.Eur |
| Magnesium stearate: | 1 | 2 mg | Ph.Eur |
| Total: | - | 200 mg | |

Formulations (a), (b), and (d)-(i) are formulations according to the present invention.

### Example 6: Analysis of composition of solid oral dosage forms

Differential Scanning Calorimetry (DSC) analysis was performed to determine the preferred diluent blend for formulating solid oral dosage forms of the present invention. The results indicate that, despite its widespread use in preparation of solid oral dosage forms, lactose monohydrate is incompatible with 2R,6R-hydroxynorketamine (see Figure 1). These data demonstrated compatibility of 2R,6R-hydroxynorketamine with dicalcium phosphate and microcrystalline cellulose.

Analysis also demonstrated compatibility of 2R,6R-hydroxynorketamine with gelatin and hydroxypropyl methylcellulose capsule shells.

### Example 7: Lack of drug-drug interactions (DDIs) between ketamine metabolites and serotonin modulators such as SSRIs

### Example 7a.

Potential interactions between ketamine metabolites of the present invention with the serotonergic system were investigated to determine whether co-administration with serotonin modulators, especially selective serotonin-reuptake inhibitors (SSRIs) affects the risk of serotonin syndrome.

Serotonin syndrome describes a specific set of symptoms resulting from an excess of serotonin within the central and peripheral nervous systems. Symptoms can vary largely, and range in severity, including possible increases in temperature, agitation and tremor, through to arrhythmias and seizure.

SSRIs work by inhibiting the reuptake of neuronal serotonin, thus increasing the synaptic concentration of the neurotransmitter and therefore activation of 5HT receptors. No one 5HT receptor is thought to be responsible for the development of serotonin syndrome, although much focus has highlighted the importance of 5HT2A receptors in the development of this serotonin related toxicity. The risk of serotonin syndrome is increased if SSRIs are taken in combination with another drug that increases extracellular serotonin, or that additively potentiates the 5HT receptors involved in its development.

2R,6R-hydroxynorketamine was investigated for binding to monoamine oxidases A and B, monoamine transporters, as well as a variety of 5HT receptors. It was found that 2R,6R-hydroxynorketamine does not interact with transporters or enzymes that directly affect synaptic monoamine concentration. In addition, studies revealed that 2R,6R-hydroxynorketamine does not bind 5HT1A, 5HT1B, 5HT2A, 5HT2B or 5HT2C receptor subtypes, indicating no risk of additive, or indeed competitive, effects if given in combination with SSRIs.

**Table 8**

| **Assay** | % **Inhibition of Control Specific Binding** | ***% of Control Specific Binding*** |
|---|---|---|
| 5-HT1A | 5 | 97.9 |
| 5-HT1B | -2 | *102.7* |
| 5-HT2A | 0 | *103.8* |
| 5-HT2B | 5 | *100.9* |
| 5-HT2C | 10 | 91.1 |
| norepinephrine transporter | -16 | *117.6* |
| dopamine transporter | 11 | *87.2* |
| 5-HT transporter | 2 | *102.0* |
| 5-HT1, Non-Selective | -9 | *105.4* |
| MAO-A | -5 | *97.7* |
| MAO-B | -1 | *100.4* |

An absence of interaction between 2R,6R-hydroxynorketamine and 5HT1 receptors is also of note. Rare postmarketing reports have described patients with weakness, hyperreflexia, and incoordination following the use of an SSRI and the 5HT1 agonist, sumatriptan. The possibility of such interactions should also be considered if other 5HT1 agonists are to be used in combination with SSRIs. As 2R,6R-hydroxynorketamine does not bind 5HT1 receptors, this potential drug-drug interaction (DDI) is avoided.

### Example 7b.

The p-gycloprotein-1 (p-gp) transporter mediates the efflux of drugs from cells. It is widely expressed throughout the body, including the luminal membrane of the small intestine, apical membranes of hepatocytes and kidney proximal tube epithelia, as well as the blood brain barrier (BBB). It is through its role at the BBB that p-gp plays a key role in limiting drug entry to the central nervous system. Many of the most commonly used antidepressants have been shown to be substrates of p-gp; including: amitriptyline, citalopram, desipramine, doxepine, fluoxetine, fluvoxamine, imipramine, nortriptyline, paroxetine, trimipramine, venlafaxine.

2R,6R-hydroxynorketamine was tested using a bidirectional transcellular transport assay (Caco-2). No active efflux of 2R,6R-hydroxynorketamine was observed (efflux ratio <2) indicating that 2R,6R-hydroxynorketamine is not a substrate of either p-gp or Breast Cancer Resistance Protein (BCRP) efflux transporters. In contrast to the effect on p-gp substrate, talinolol, the transporter inhibitor, elacridar, did not affect the efflux ratio of 2R,6R-hydroxynorketamine (2R,6R-hydroxynorketamine efflux ratio = 1.11, + elacridar = 1.15; talinolol efflux ratio = 37, + elacridar = 0.882).

This finding eliminates the possibility that combination therapy of 2R,6R-hydroxynorketamine and an SSRI described above, would induce competition for the p-gp transporter. Such a finding is relevant for the prevention of DDIs involving increased concentrations of either compound within the brain.

Many SSRIs also act as inhibitors of p-gp transporters, with sertraline and paroxetine displaying an IC₅₀ similar to that of established p-gp inhibitor quinidine. As 2R,6R-hydroxynorketamine is not a substrate of p-gp, combination therapy with these SSRIs will not induce DDIs based on a reduction in 2R,6R-hydroxynorketamine efflux from the brain.

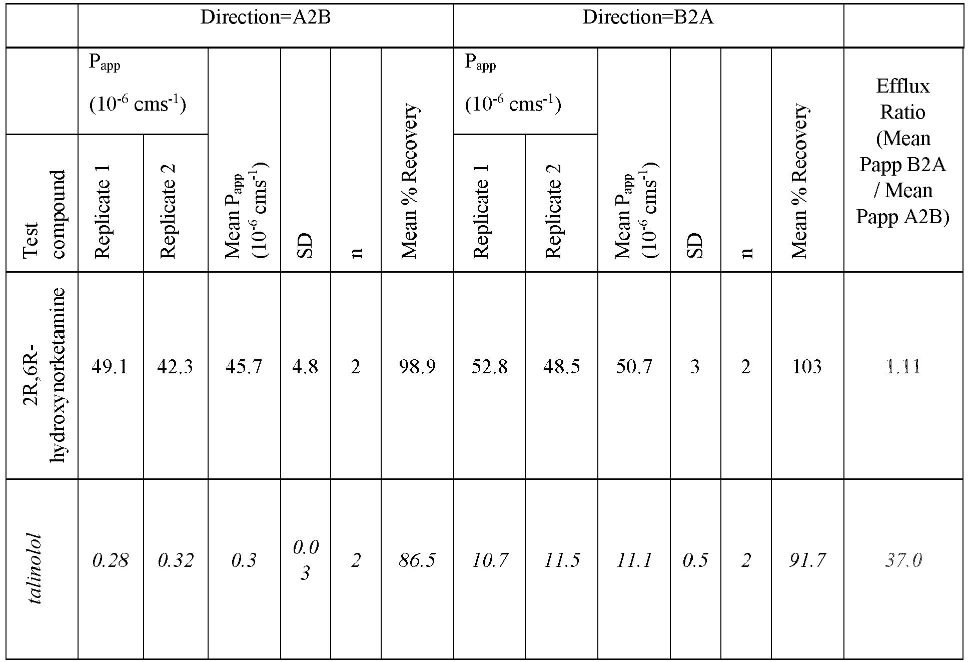

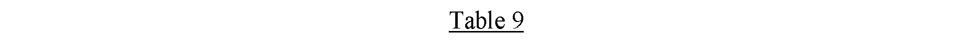

### Example 7c.

While the risk of DDIs due to displacement from plasma binding proteins (PPBs) is relatively low, it should be considered for drugs which display a high proportion PPB (fraction unbound (fu)<1%).

Many commonly used SSRIs are highly protein bound (fluoxetine: 94%; paroxetine: 95%; sertraline: 98%), with advice that co-administration with another drug that also displays high protein binding may result in adverse effects due to an increase in plasma levels of either unbound drug.

The fu was investigated for 2R,6R-hydroxynorketamine using a 100% plasma from 4 test species. 2R,6R-hydroxynorketamine was found to display low PPB in all species tested (mouse, rat, dog and human; Figure. 2).

As a small molecule with low levels of PPB, 2R,6R-hydroxynorketamine does not risk displacement of SSRIs.

### Example 7d.

Cytochrome P450s (CYPs) are a family of enzymes that play a primary role in the metabolism of a wide variety of drugs. 2R,6R-hydroxynorketamine was investigated for its potential to inhibit the action of 7 key CYP enzymes using an inhibition assay measuring reduction in the formation of metabolites compared to control. These data were then used to calculate an IC₅₀ value.

2R,6R-hydroxynorketamine did not inhibit cytochrome P450 isoforms: CYP2B6, CYP2C8, CYP2C9, CYP2C19 and CYP2D6. 2R,6R-hydroxynorketamine displayed inhibition of CYP1A2, with an IC₅₀ of ~100 µM. 2R,6R-hydroxynorketamine was found to inhibit the action of CYP3A4 on both substrate groups, midazolam and testosterone, with an IC₅₀ of 691µM and 81µM respectively.

**Table 10**

| **Cytochrome P450** | **% Inhibition** | | | | | | |
|---|---|---|---|---|---|---|---|
| | **0 µM** | **0.4 µM** | **1 µM** | **4 µM** | **10 µM** | **40 µM** | **100 µM** |
| CYP2D6 | 0 | 3.16 | 6.65 | 11.5 | 9.41 | 11.2 | 5.73 |
| CYP2C19 | 0 | -2.01 | -1.66 | 1.85 | 6.69 | 8.25 | 16.1 |
| CYP2C8 | 0 | 5.63 | 4.91 | 12.5 | 8.33 | 8.35 | 2.08 |
| CYP2C9 | 0 | 4.59 | 5.92 | 5.80 | 11.9 | -0.113 | -1.33 |
| CYP2B6 | 0 | 6.03 | 10.1 | 10.6 | 14.4 | 14.8 | 16.7 |
| CYP1A2 | 0 | 7.55 | 8.80 | 11.7 | 19.3 | 24.8 | 48.6 |
| CYP3A4, Substrate = midazolam | 0 | 11.5 | 8.44 | 15.7 | 25.7 | 43.7 | 57.5 |
| CYP3A4, Substrate = testosterone | 0 | 1.62 | -0.509 | 7.04 | 12.4 | 35.2 | 53.4 |

CYP2D6 is the major enzyme involved in the metabolism of the majority of commonly used antidepressants, including fluoxetine, paroxetine and venlafaxine. 2R,6R-hydroxynorketamine does not cause inhibition of this CYP enzyme, evading potential DDIs due to decreased metabolism of such SSRIs.

The free (unbound) concentration of 2R,6R-hydroxynorketamine producing pharmacodynamics activity in the brain is estimated to be approximately 10µM. This concentration has been demonstrated to be sufficient in causing an increase in field excitatory postsynaptic potentials (fEPSPs) in *in vitro* electrophysiological experiments, a finding is dependent on the potentiation of α-amino-3-hydroxy-5-methyl-4-isoxazolepropionic acid receptor (AMPAR)-mediated currents. A free concentration 10µM 2R,6R-hydroxynorketamine is sufficiently remote from the IC₅₀ values for CYP1A2 and CYP3A4 to avoid DDI risk.

### Example 7e.

Following phase I metabolism by CYP enzymes, many drugs undergo phase II metabolism, including glucuronidation, a process completed by the uridine glucuronyl transferase (UGT) family.

The SSRI sertraline is glucuronidated to sertraline *N*-carbamoyl glucuronide by a variety of UGT enzymes, with the greatest activity observed with UGT2B7. 2R,6R-hydroxynorketamine was therefore investigated for its potential to inhibit this isozyme, along with that of the major UGT isozyme, UGT1A1, using a UGT inhibition assay.

2R,6R-hydroxynorketamine does not inhibit UGT2B7 or UGT1A1, supporting the possibility of its use in combination with sertraline.

**Table 11**

| **UGT Inhibition: Summary of IC₅₀ Values (µM)** | | | |
|---|---|---|---|
| **Inhibitor** | **Test Concentration** | **UGT1A1** | **UGT2B7** |
| **2R-6R-Hydroxynorketamine Hydrochloride** | 0.1µM - 100µM | > 100 | > 100 |
| *Atazanavir* | *0.006µM* - *6µM* | *0.153* | |
| *Diclofenac* | *2.7µM* - *2000µM* | | *16.4* |

### Example 8: Supporting data for orally bioavailability studies

The solubility and permeability of 2R,6R-hydroxynorketamine was investigated using turbidimetric aqueous solubility and Caco-2 permeability assays respectively. 2R,6R-hydroxynorketamine was found to display high solubility and permeability.

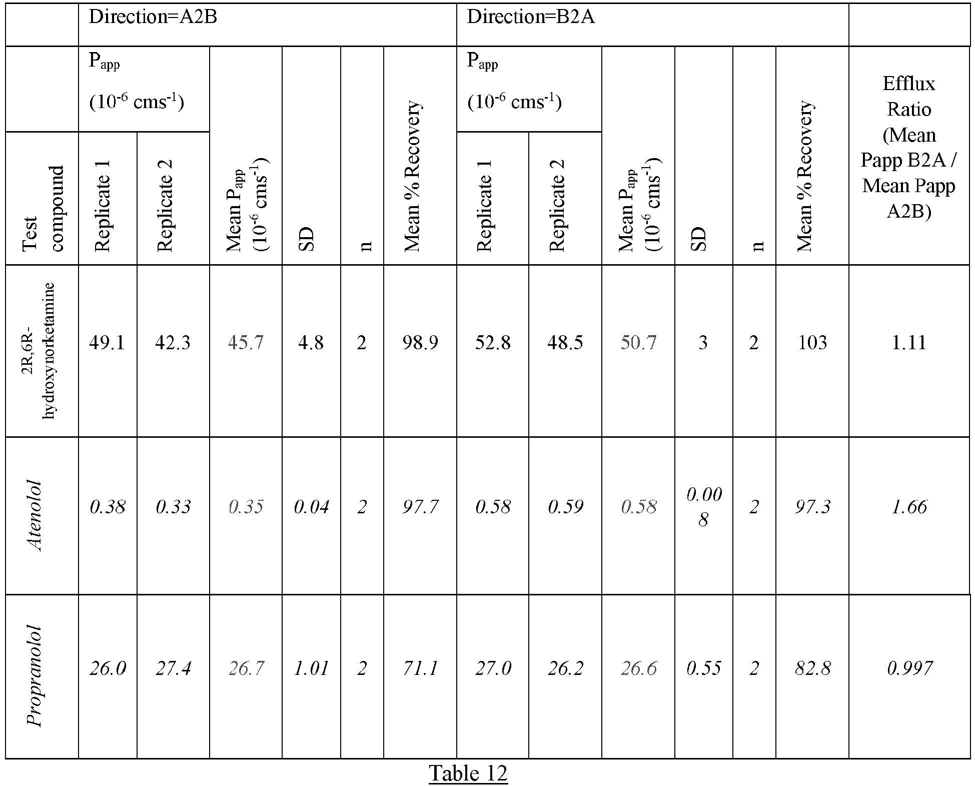

Propranolol has a known human absorption of 90%. Mean apparent permeability coefficient (Papp) values for 2R,6R-hydroxynorketamine are greater than those seen for propranolol controls, suggestive of high human absorption.

Both the solubility and permeability of a compound play a major role in its suitability for oral administration. The data reported presently support 2R,6R-hydroxynorketamine as a suitable candidate for oral administration.

Following absorption, orally administered drugs undergo presystemic metabolism. This firstpass effect contributes one of largest factors affecting the oral bioavailability of a drug.

2R,6R-hydroxynorketamine was investigated using an *in vitro* hepatocyte stability assay, during which 2R,6R-hydroxynorketamine was incubated with cryopreserved hepatocytes from 3 test species. 2R,6R-hydroxynorketamine displayed a negative intrinsic clearance value (Clᵢₙₜ) in a human hepatocyte stability assay, indicating it does not undergo hepatic clearance in humans (Figure 3). These data, along with metabolite profiling revealed that 2R,6R-hydroxynorketamine does not undergo phase I metabolism by CYP enzymes, but is glucuronidated and excreted via the renal system.

**Table 13**

| Species | CLᵢₙₜ (µL/min/10⁶ cells) | SE CLᵢₙₜ | t_{1/2} (min) | n |
|---|---|---|---|---|
| Rat | 55.2 | 4.24 | 25.1 | 5 |
| Dog | 4.72 | 1.15 | 294 | 6 |
| Human | -1.07 | 0.293 | -1300 | 6 |

Oral bioavailability was also investigated *in vivo* in male C57BL/6J mice after administration of 10, 30, 100 or 300mg/kg of 2R,6R-hydroxynorketamine by oral gavage. Samples were collected at 5 time points (10 minutes, 30 minutes, 1 hour, 6 hours and 24 hours) and analysed for the presence of 2R,6R-hydroxynorketamine (see Figures 4A and 4B). Data demonstrates that a low oral dose of 2R,6R-hydroxynorketamine has poor oral bioavailability due to glucuronidation in the gut. Conversely, a high oral dose of 2R,6R-hydroxynorketamine has good oral bioavailability due to saturation of UGT enzymes in the gut.

### Example 9: Evaluation of the effect of 2R,6R-hydroxynorketamine on cognitive function using the Progressive Ratio Test in the mouse

In order to confirm that the effects observed in Example 1 were a consequence of drug effects on cognitive function, 2R,6R-hydroxynorketamine L-pyroglutamate (2R,6R-HNK) was investigated using a Progressive ratio (PR) schedule.

PR was first described by Hodos in 1961. The schedule requires subjects to perform an incrementally increasing number of responses for the delivery of a single reward (reinforcer). This task is thought to most closely assay motivation and apathy in experimental animals. By challenging the mice to a task in which the difficulty is gradually increased, one can quantitatively measure the point at which individual mice stop responding (the 'breakpoint'). PR represents an important tool for assessing dysfunctional motivation behaviour in preclinical studies, including characterisation of rodent models and assessment of candidate therapeutics. Previous studies have demonstrated that PR performance can be affected by pharmacological modulation of a number of neurotransmitter systems, including targets relevant to antidepressant treatment including multiple 5-hydroxytryptamine and dopamine receptor subtypes.

Mice were tested during the dark part of their light/dark cycle. In order to promote reward consumption and therefore facilitate training, mild food restriction was implemented. Following stabilisation of restricted body weight, animals were familiarised with the reinforcer (strawberry milkshake) and subsequently the apparatus itself. Once habituated to the behavioural equipment, mice were initially trained to emit a set number of responses at the touchscreen for delivery of the reinforcer (fixed ratio schedule). When this behaviour was fully established, mice were tested on sessions which required an increasing number of responses for the delivery of each subsequent reinforcer (progressive ratio schedule; required responses increased by 4 for each trial, from 1 to 5 to 9 etc.). These sessions terminated following either completion of the 60 minute test period or 5 minutes of inactivity. The final number of responses that an animal made for a single reinforcer in a session was termed the 'breakpoint' and was taken as an index of motivation and apathy. In addition, the rate of responding (running rate) was analysed to provide further insight into the effect of drug administration on task performance and as an additional indicator of apathy.

A total of 3 dose groups were studied, with a sample number of 16 in each group. 2R,6R-HNK was evaluated at 3 doses administered 20 minutes before the test. The highest dose tested was 300mg/kg. This maximum dose was selected as it corresponded to the highest dose administered in previous studies, as well as falling below the highest dose of 2R,6R-HNK used in the literature (Zanos et al 2016). Ketamine (10mg/kg) was used as a reference substance. Saline for injection was used as a control, administered under the same experimental conditions as test compounds. All drugs were dosed via intraperitoneal (IP) injection, chosen based on the experience of the lab and historical data.

Results were analysed by comparing all groups using repeated measures two-way ANOVA or one-way ANOVA, where necessary.

### RESULTS

### Progressive Ratio Test in the mouse

Mice performed baseline testing on Day 1 to provide a drug-free reference point, with data used to calculate the fold change in breakpoint after drug treatment. Testing was completed 20 minutes after administration of test, reference or control compound for 4 consecutive days (Tuesday - Friday). This short latency was selected to capture a higher circulating concentration of 2R,6R-HNK as determined in pharmacokinetic studies of 2R,6R-HNK in the mouse.

Under these conditions, 2R,6R-HNK (300mg/kg) produced a significant increase in breakpoint compared to vehicle and ketamine (Figure 8A). This increase in breakpoint remained following normalisation to baseline (Figure 8B).

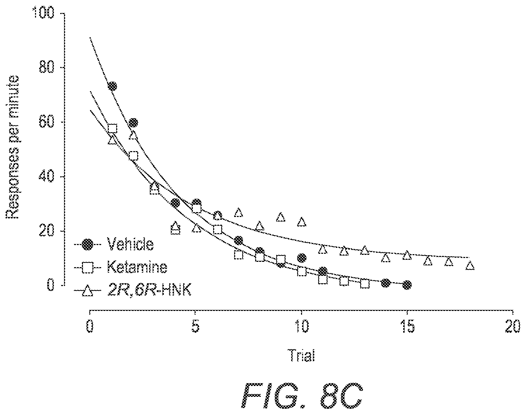

To investigate possible explanations for the increase in breakpoint, the running rate for each mouse was also determined. This rate was calculated by dividing the number of touches (responses) by the run time (time taken to complete the ratio, excluding the postreinforcement pause). These values were extracted per animal and fitted with the negative exponential y=a^(-b^{∗}x)). Coefficients for predicted peak response rate (a) and decay of response rate (-b) were extracted and evaluated for between-group significance using a one-way ANOVA or linear mixed-effect models. Running rate measures revealed that following administration of 2R,6R-HNK (300mg/kg), mice maintained a higher rate of responding throughout the 60 minute test session compared to ketamine and vehicle groups (Figure 8C), indicative of increased effort and thus reduced apathy. Average responses per minute performed by 2R,6R-HNK treated mice were 29% higher compared with mice treated only with vehicle, and 59% higher compared with mice treated with ketamine.

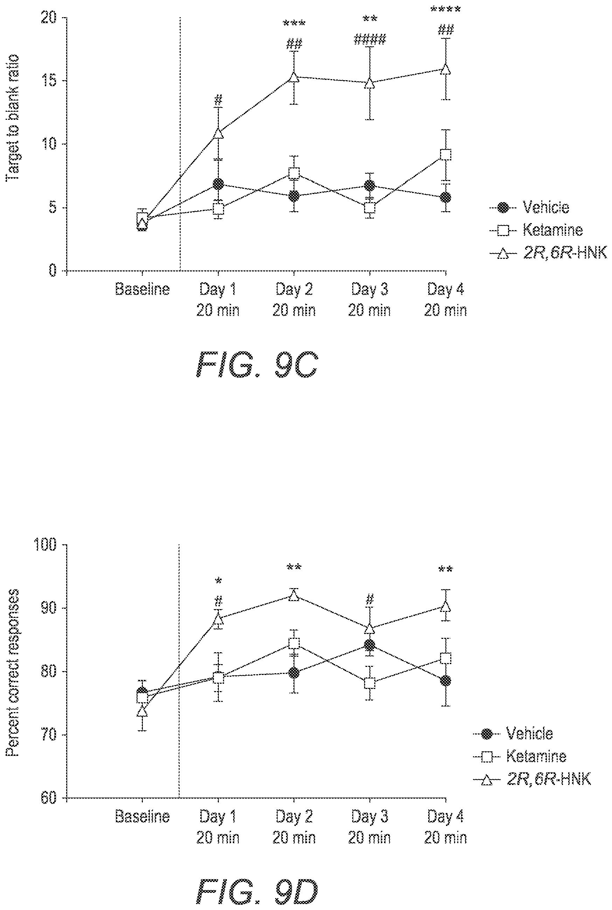

In addition to breakpoint (an indicator of motivation) and running rate (a measure of effort, indicative of apathy), the ratio of target to blank touches and percentage of correct touches were calculated, providing a measure of accuracy of task completion indicative of executive function and cognitive control. 2R,6R-HNK (300mg/kg) significantly increased the number of target touches compared to blank touches (Figure 9C) and percentage of correct touches over the 4-day testing period (Figure 9D).

This pattern of improved performance was replicated following a 3-week wash-out period. As demonstrated previously, 300mg/kg 2R,6R-HNK resulted in a significant increase in breakpoint compared to vehicle and ketamine (Figure 10). The number of target touches (Figure 11A), the number of target touches to blank touches (Figure 11B) and percent of correct touches (Figure 11D) were also increased, indicating the same improvement in accuracy as seen previously.

2R,6R-HNK was formulated for these assays as a pyroglutamate salt due to its high solubility and easy handling properties. To determine whether pyroglutamate was responsible for the effects observed, sodium L-pyroglutamate (NaPg) was administered as a control (dose matched to 300mg/kg 2R,6R-HNK). NaPg did not significantly affect any measure examined (Figure 12A and 12B).

### CONCLUSION

The results of the Progressive Ratio Task suggest that 2R,6R-HNK produces an acute increase in cognitive measures, in particular increased motivation, as evidenced by the increase in breakpoint. The maintenance of an increased running rate over the test hour provides a partial explanation of the increase in breakpoint, and is indicative of increased effort and decreased apathy. Moreover, the rapid and pronounced increase in accuracy in this task among mice exposed to 2R,6R-HNK demonstrates robust cognitive enhancement.

No increase in cognitive measures were observed at doses below 30 mg/kg. The human dose calculated from Zanos (2016), which reports effects at 10 mg/kg in mice, would be incapable of achieving the cognitive enhancement properties of 2R,6R-HNK, whereas the solid oral dosage forms of the present invention would be suitable to deliver the unexpectedly high dose of ketamine metabolites required to achieve cognitive enhancement in a patient suffering from a neurocognitive disorder, a neurodevelopmental disorder, and a psychocognitive disorder.

## Claims

1. A solid oral dosage form comprising a ketamine metabolite selected from 2R,6R-hydroxynorketamine and 2S,6S-hydroxynorketamine as a solid low molecular weight salt thereof, wherein the dosage form comprises at least 20% by weight of the ketamine metabolite, wherein the solid oral dosage form delivers the active ingredient with a human oral bioavailability of 60% or more; and
wherein the low molecular weight salt of the ketamine metabolite is obtainable by reaction of the ketamine metabolite with an organic acid having Formula I or II 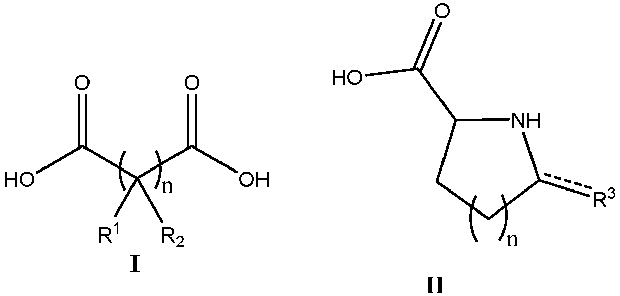 wherein n = 0-3,
R¹ and R² are each independently selected from -H, -OH, and -COOH, and wherein when n = 2, both R² may together represent a C=C bond, and wherein
R³ is -H, -OH, =O, or -COOH.

2. The solid oral dosage form of claim 1 having a length no greater than 16mm, preferably having a length between 6mm and 12mm.

3. The solid oral dosage form of claim 1 or claim 2 comprising up to 500mg of the ketamine metabolite, at least 40% by weight of the ketamine metabolite, and a length no greater than 12 mm.

4. The solid oral dosage form of any of claims 1 to 3 wherein the low molecular weight salt of the ketamine metabolite comprises up to two stoichiometric equivalents of an anionic counterion having a molecular weight of 160 daltons or less.

5. The solid oral dosage form of any one of claims 1 to 4 comprising at least 40% by weight of the ketamine metabolite.

6. The solid oral dosage form of any of claims 1 to 5 wherein the dosage form is a capsule or a tablet.

7. The solid oral dosage form of claim 6 comprising a blend of one or more diluent, wherein said blend comprises microcrystalline cellulose.

8. The solid oral dosage form of any one of claims 1 to 7 comprising a crystalline form of a ketamine metabolite selected from 2R,6R-hydroxynorketamine L-pyroglutamate and 2S,6S-hydroxynorketamine D-pyroglutamate having an X-ray powder diffraction pattern comprising characteristic peaks expressed in degrees 2-theta at position 14.3.

9. The solid oral dosage form of any one of claims 1 to 8 for use in a method of treating a disorder in a patient wherein said disorder is selected from a neurocognitive disorder, a neurodevelopmental disorder, and a psychocognitive disorder.

10. The solid oral dosage form for the use of claim 9 comprising an effective dose of the ketamine metabolite to enhance one or more cognitive domains of the patient, wherein the cognitive domain is selected from executive function, perceptual function, learning ability, memory, and attention.

11. The solid oral dosage form for the use of claim 9 or 10 wherein the neurocognitive disorder is selected from (i) delirium, (ii) Alzheimer's disease, (iii) pseudodementia, (iv) frontotemporal neurocognitive disorder, (v) dementia with Lewy Bodies (vi) vascular neurocognitive disorder, (vii) multi-infarct dementia, (viii) a tauopathy, (ix) Parkinson's Disease, (x) Huntingdon's disease, (xi) transmissible spongiform encephalopathy, (xii) amyotrophic lateral sclerosis, (xiii) traumatic brain injury, (xiv) post-concussion syndrome, (xv) amnesia, (xvi) substance-induced neurocognitive disorder, (xvii) alcohol-induced neurocognitive disorder, (xviii) stroke disorder, (xix) hypersomnia, and (xx) clonic perseveration, or wherein the neurodevelopmental disorder is selected from (i) intellectual disability, (ii) learning disability, (iii) dyslexia, (iv) dyscalculia, (v) dyspraxia, (vi) dysgraphia, (vii) autism-spectrum disorder, (viii) stereotypic movement disorder, (ix) tic disorder, (x) cerebral palsy (xi) fragile-X syndrome, (xii) Down syndrome, (xiii) attention-deficit disorder, (xiv) hypogonadotropic hypogonadal syndrome (xv) neurotoxicant poisoning, (xvi) foetal alcohol spectrum disorder, (xvii) Minamata disease, and (xviii) Rett syndrome, or wherein the psychocognitive disorder is selected from (i) an obsessive compulsive disorder, (ii) a depressive disorder, (iii) a schizophrenia disorder, (iv) a schizotypal disorder, (v) an anxiety disorder, (vi) substance abuse, and (vii) an avolition disorder.

12. A solid oral dosage form for the use of any one of claims 9 to 11 wherein said method comprises the steps of:
a. identifying a deficit in a cognitive domain of said patient, wherein the cognitive domain is selected from executive function, perceptual function, learning ability, memory, and attention, and
b. administering to said patient the solid oral dosage form as defined in any one of claims 1 to 8.

13. The solid oral dosage form for the use of claim 12 wherein the deficit in said cognitive domain is identified using a computer-based cognitive assessment.

## Patentansprüche

1. Feste orale Dosierungsform, umfassend einen Ketaminmetaboliten, ausgewählt aus 2R,6R-Hydroxynorketamin und 2S,6S-Hydroxynorketamin, als ein festes Salz mit niedrigem Molekulargewicht davon, wobei die Dosierungsform zumindest 20 Gew.-% des Ketaminmetaboliten umfasst, wobei die feste orale Dosierungsform den Wirkstoff mit einer oralen menschlichen Bioverfügbarkeit von 60 % oder mehr liefert; und
wobei das Salz mit niedrigem Molekulargewicht des Ketaminmetaboliten durch Umsetzung des Ketaminmetaboliten mit einer organischen Säure erhältlich ist mit der Formel I oder II 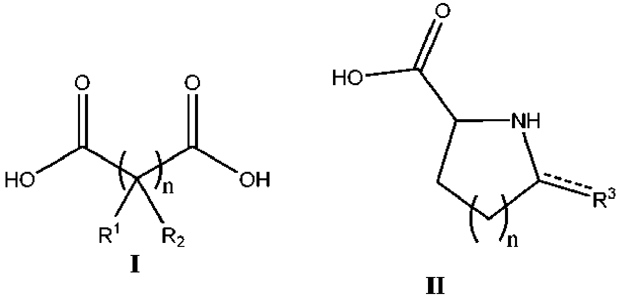 wobei n = 0-3,
R¹ und R² jeweils unabhängig ausgewählt sind aus -H, -OH und -COOH, und wobei, wenn n = 2, beide R² zusammen eine C=C-Bindung darstellen können, und wobei R³ -H, -OH, =O oder -COOH ist.

2. Feste orale Dosierungsform nach Anspruch 1 mit einer Länge von nicht mehr als 16 mm, vorzugsweise mit einer Länge zwischen 6 mm und 12 mm.

3. Feste orale Dosierungsform nach Anspruch 1 oder Anspruch 2, umfassend bis zu 500 mg des Ketaminmetaboliten, zumindest 40 Gew.-% des Ketaminmetaboliten und eine Länge von nicht mehr als 12 mm.

4. Feste orale Dosierungsform nach einem der Ansprüche 1 bis 3, wobei das Salz mit niedrigem Molekulargewicht des Ketaminmetaboliten bis zu zwei stöchiometrische Äquivalente eines anionischen Gegenions mit einem Molekulargewicht von 160 Dalton oder weniger umfasst.

5. Feste orale Dosierungsform nach einem der Ansprüche 1 bis 4, umfassend zumindest 40 Gew.-% des Ketaminmetaboliten.

6. Feste orale Dosierungsform nach einem der Ansprüche 1 bis 5, wobei die Dosierungsform eine Kapsel oder eine Tablette ist.

7. Feste orale Dosierungsform nach Anspruch 6, umfassend eine Mischung aus einem oder mehreren Verdünnungsmittel, wobei die Mischung mikrokristalline Cellulose umfasst.

8. Feste orale Dosierungsform nach einem der Ansprüche 1 bis 7, umfassend eine kristalline Form eines Ketaminmetaboliten, ausgewählt aus 2R,6R-Hydroxynorketamin-L-pyroglutamat und 2S,6S-Hydroxynorketamin-D-pyroglutamat mit einem Röntgenpulverbeugungsdiagramm, umfassend charakteristische Spitzen, ausgedrückt in Grad 2-Theta an Position 14,3.

9. Feste orale Dosierungsform nach einem der Ansprüche 1 bis 8 zur Verwendung in einem Verfahren zum Behandeln einer Störung bei einem Patienten, wobei die Störung ausgewählt ist aus einer neurokognitiven Störung, einer neurologischen Entwicklungsstörung und einer psychokognitiven Störung.

10. Feste orale Dosierungsform zur Verwendung nach Anspruch 9, umfassend eine wirksame Dosis des Ketaminmetaboliten zum Verbessern eines oder mehrerer kognitiver Bereiche des Patienten, wobei der kognitive Bereich ausgewählt ist aus Exekutivfunktion, Wahrnehmungsfunktion, Lernfähigkeit, Gedächtnis und Aufmerksamkeit.

11. Feste orale Dosierungsform zur Verwendung nach Anspruch 9 oder 10, wobei die neurokognitive Störung ausgewählt ist aus (i) Delirium, (ii) Alzheimer-Erkrankung, (iii) Pseudodemenz, (iv) frontotemporaler neurokognitiver Störung, (v) Demenz mit Lewy-Körperchen (vi) vaskulärer neurokognitiver Störung, (vii) Multi-Infarkt-Demenz, (viii) einer Tauopathie, (ix) Parkinson-Erkrankung, (x) Huntingdon-Erkrankung, (xi) übertragbarer spongiformer Enzephalopathie, (xii) amyotropher Lateralsklerose, (xiii) traumatischer Hirnverletzung, (xiv) Post-Gehirnerschütterungs-Syndrom, (xv) Amnesie, (xvi) substanzinduzierter neurokognitiver Störung, (xvii) alkoholinduzierter neurokognitiver Störung, (xviii) Schlaganfallstörung, (xix) Hypersomnie und (xx) klonischer Perseveration, oder wobei die neurologische Entwicklungsstörung ausgewählt ist aus (i) geistiger Behinderung, (ii) Lernbehinderung, (iii) Dyslexie, (iv) Dyskalkulie, (v) Dyspraxie, (vi) Dysgraphie, (vii) Autismus-Spektrum-Störung, (viii) stereotyper Bewegungsstörung, (ix) Tic-Störung, (x) Zerebralparese (xi) Fragiles-X-Syndrom, (xii) Down-Syndrom, (xiii) Aufmerksamkeitsdefizitstörung, (xiv) hypogonadotropem hypogonadalem Syndrom (xv) neurotoxischer Vergiftung, (xvi) fetaler Alkoholspektrumstörung, (xvii) Minamata-Erkrankung und (xviii) Rett-Syndrom, oder wobei die psychokognitive Störung ausgewählt ist aus (i) einer Zwangsstörung, (ii) einer depressiven Störung, (iii) einer Schizophreniestörung, (iv) einer schizotypischen Störung, (v) einer Angststörung, (vi) Drogenmissbrauch und (vii) einer Antriebsstörung.

12. Feste orale Dosierungsform zur Verwendung nach einem der Ansprüche 9 bis 11, wobei das Verfahren die Schritte umfasst von:
a. Identifizieren eines Defizits in einem kognitiven Bereich des Patienten, wobei der kognitive Bereich ausgewählt ist aus Exekutivfunktion, Wahrnehmungsfunktion, Lernfähigkeit, Gedächtnis und Aufmerksamkeit, und
b. Verabreichen der festen oralen Dosierungsform an den Patienten, wie in einem der Ansprüche 1 bis 8 definiert.

13. Feste orale Dosierungsform zur Verwendung nach Anspruch 12, wobei das Defizit in dem kognitiven Bereich unter Verwendung einer computergestützten kognitiven Bewertung identifiziert wird.

## Revendications

1. Forme galénique orale solide comprenant un métabolite de kétamine choisi parmi la 2R,6R-hydroxynorkétamine et la 2S,6S-hydroxynorkétamine sous la forme d'un sel solide de faible poids moléculaire de celle-ci, ladite forme galénique comprenant au moins 20 % en poids du métabolite de kétamine, ladite forme galénique orale solide délivrant l'ingrédient actif avec une biodisponibilité orale humaine de 60 % ou plus ; et
ledit sel de faible poids moléculaire du métabolite de kétamine pouvant être obtenu par réaction du métabolite de kétamine avec un acide organique de formule I ou II 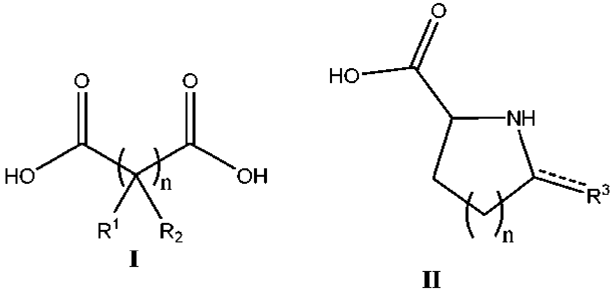 dans laquelle n = 0 à 3,
R¹ et R² sont chacun indépendamment choisis parmi les groupes -H, -OH et -COOH, et dans laquelle lorsque n = 2, les deux R² peuvent représenter ensemble une liaison C=C, et dans laquelle R³ représente un groupe -H, -OH, =O ou -COOH.

2. Forme galénique orale solide selon la revendication 1, présentant une longueur non supérieure à 16 mm, de préférence présentant une longueur comprise entre 6 mm et 12 mm.

3. Forme galénique orale solide selon la revendication 1 ou la revendication 2, comprenant jusqu'à 500 mg du métabolite de kétamine, au moins 40 % en poids du métabolite de kétamine et une longueur non supérieure à 12 mm.

4. Forme galénique orale solide selon l'une quelconque des revendications 1 à 3, ledit sel de faible poids moléculaire du métabolite de kétamine comprenant jusqu'à deux équivalents stœchiométriques d'un contre-ion anionique présentant un poids moléculaire de 160 daltons ou moins.

5. Forme galénique orale solide selon l'une quelconque des revendications 1 à 4, comprenant au moins 40 % en poids du métabolite de kétamine.

6. Forme galénique orale solide selon l'une quelconque des revendications 1 à 5, ladite forme galénique étant une capsule ou un comprimé.

7. Forme galénique orale solide selon la revendication 6, comprenant un mélange d'un ou plusieurs diluant, ledit mélange comprenant de la cellulose microcristalline.

8. Forme galénique orale solide selon l'une quelconque des revendications 1 à 7, comprenant une forme cristalline d'un métabolite de kétamine choisi parmi le L-pyroglutamate de 2R,6R-hydroxynorkétamine et le D-pyroglutamate de 2S,6S-hydroxynorkétamine présentant un diagramme de diffraction des rayons X sur poudre qui comprend les pics caractéristiques exprimés en degrés 2-thêta au niveau de la position à 14,3.

9. Forme galénique orale solide selon l'une quelconque des revendications 1 à 8 pour une utilisation dans une méthode de traitement d'un trouble chez un patient, ledit trouble étant choisi parmi un trouble neurocognitif, un trouble neurologique du développement et un trouble psychocognitif.

10. Forme galénique orale solide pour l'utilisation selon la revendication 9, comprenant une dose efficace du métabolite de kétamine pour améliorer un ou plusieurs domaines cognitifs du patient, ledit domaine cognitif étant choisi parmi la fonction exécutive, la fonction perceptuelle, la capacité d'apprentissage, la mémoire et l'attention.

11. Forme galénique orale solide pour l'utilisation selon la revendication 9 ou 10, ledit trouble neurocognitif étant choisi parmi (i) le délire, (ii) la maladie d'Alzheimer, (iii) la pseudo-démence, (iv) un trouble neurocognitif frontotemporal, (v) la démence à corps de Lewy (vi) un trouble neurocognitif vasculaire, (vii) la démence de multi-infarctus, (viii) une tauopathie, (ix) la maladie de Parkinson, (x) la maladie de Huntingdon, (xi) l'encéphalopathie spongiforme transmissible, (xii) la sclérose latérale amyotrophique, (xiii) une lésion cérébrale traumatique, (xiv) un syndrome post-commotionnel, (xv) l'amnésie, (xvi) un trouble neurocognitif induit par une substance, (xvii) un trouble neurocognitif induit par l'alcool, (xviii) un trouble associé à un AVC, (xix) l'hypersomnie, et (xx) la persévération clonique, ou ledit trouble neurologique du développement étant choisi parmi (i) une déficience intellectuelle, (ii) une incapacité d'apprentissage, (iii) une dyslexie, (iv) une dyscalculie, (v) une dyspraxie, (vi) une dysgraphie, (vii) un trouble du spectre autistique, (viii) un trouble du mouvement stéréotypé, (ix) un trouble de type tic, (x) une paralysie cérébrale, (xi) le syndrome de l'X fragile, (xii) le syndrome de Down, (xiii) un trouble de déficit de l'attention, (xiv) le syndrome d'hypogonadisme hypogonadotrophique, (xv) un empoisonnement par neurotoxique, (xvi) un trouble du spectre de l'alcoolisation fœtale, (xvii) la maladie de Minamata, et (xviii) le syndrome de Rett, ou ledit trouble psychocognitif étant choisi parmi (i) un trouble obsessionnel compulsif, (ii) un trouble dépressif, (iii) un trouble schizophrénique, (iv) un trouble schizotypique, (v) un trouble anxieux, (vi) la toxicomanie, et (vii) un trouble d'avolition.

12. Forme galénique orale solide pour l'utilisation selon l'une quelconque des revendications 9 à 11, ladite méthode comprenant les étapes de :
a. identification d'un déficit dans un domaine cognitif dudit patient, ledit domaine cognitif étant choisi parmi la fonction exécutive, la fonction perceptuelle, la capacité d'apprentissage, la mémoire et l'attention, et
b. l'administration audit patient de la forme galénique orale solide telle que définie dans l'une quelconque des revendications 1 à 8.

13. Forme galénique orale solide pour l'utilisation selon la revendication 12, ledit déficit dans ledit domaine cognitif étant identifié à l'aide d'une évaluation cognitive informatisée.
